(19) **Europäisches Patentamt / European Patent Office / Office européen des brevets**

(11) **EP 4 324 853 A1**

(12) **EUROPEAN PATENT APPLICATION**
published in accordance with Art. 153(4) EPC

(43) Date of publication:
**21.02.2024 Bulletin 2024/08**

(21) Application number: **22787602.6**

(22) Date of filing: **14.04.2022**

(51) International Patent Classification (IPC):
**C07K 16/46** [(2006.01)]    **C07K 16/28** [(2006.01)]
**A61K 39/395** [(2006.01)]    **A61P 35/00** [(2006.01)]

(52) Cooperative Patent Classification (CPC):
**A61K 39/395; A61P 35/00; C07K 16/28; C07K 16/46**

(86) International application number:
**PCT/CN2022/086842**

(87) International publication number:
**WO 2022/218380 (20.10.2022 Gazette 2022/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.04.2021  CN 202110405638**

(71) Applicant: **CHIA TAI TIANQING PHARMACEUTICAL GROUP CO., LTD.**
**Lianyungang,**
**Jiangsu 222062 (CN)**

(72) Inventors:
• **ZHANG, Bing**
**Nanjing, Jiangsu 211100 (CN)**
• **ZHAO, Wei**
**Nanjing, Jiangsu 211100 (CN)**
• **LU, Yamin**
**Nanjing, Jiangsu 211100 (CN)**
• **DU, Min**
**Nanjing, Jiangsu 211100 (CN)**

• **ZHEN, Zipeng**
**Nanjing, Jiangsu 211100 (CN)**
• **XIE, Meijuan**
**Nanjing, Jiangsu 211100 (CN)**
• **WANG, Xin**
**Nanjing, Jiangsu 211100 (CN)**
• **XI, Junxiao**
**Nanjing, Jiangsu 211100 (CN)**
• **SONG, Xin**
**Nanjing, Jiangsu 211100 (CN)**
• **DU, Xiuzhen**
**Nanjing, Jiangsu 211100 (CN)**

(74) Representative: **Grünecker Patent- und Rechtsanwälte**
**PartG mbB**
**Leopoldstraße 4**
**80802 München (DE)**

Remarks:
The complete document including Reference Table(s) and the Sequence Listing(s) can be downloaded from the EPO website

(54) **MULTI-SPECIFIC ANTIBODY TARGETING BCMA**

(57)    Provided is a multi-specific antibody targeting BCMA. Specifically provided are a multi-specific antibody, a nucleic acid encoding same, a vector comprising the nucleic acid, a cell comprising the vector, and a pharmaceutical composition comprising same. Further provided is the use thereof for treating a subject having a disease related to BCMA expression.

EP 4 324 853 A1

FIG. 5

**Description**

**TECHNICAL FIELD**

The present disclosure relates to a multispecific antibody, and in particular to a BCMA-targeting multispecific antibody.

**BACKGROUND**

B-cell maturation antigen (BCMA), also known as tumor necrosis factor receptor superfamily member 17 (TNFRS17), is a protein that in humans is encoded by the TNFRSF17 gene.

BAFF and APRIL are ligands for BCMA. BAFF (also known as BLyS, TALL-1, THANK, zTNF4, TNFSF20 or D8Ertd387e) is a high-affinity ligand for BCMA. APRIL (a proliferation-inducing ligand, also known as TNFSF13, TALL-2 or TRDL-1) is a low-affinity ligand for BCMA. In addition, BAFF and APRIL are also ligands for tumor necrosis factor receptor (TNFR) superfamily member B-cell activation factor receptor (BAFF-R), and for transmembrane activator and calcium modulator and cyclophilin ligand interactor (TACI). BCMA together with BAFF-R and TACI regulates the proliferation, survival, maturation and differentiation of B cells.

Multiple myeloma is a malignant disease of plasma cells. In cells of multiple myeloma, the BCMA expression level is significantly increased. BCMA can be a suitable tumor antigen target for immunotherapeutic agents for multiple myeloma. Immunotherapeutic agents such as antibodies that bind to BCMA are able to block the binding of BCMA to its natural ligand BAFF or/and APRIL.

In addition to conventional IgG1 antibodies of a four-chain structure comprising light chains and heavy chains, IgG antibodies in animals of the family Camelidae also include naturally occurring IgG2 and IgG3 heavy-chain antibodies (HcAbs) free of light chains. The single variable region domains (V$_H$Hs or single variable domains) of heavy-chain antibodies have the characteristics of specifically binding to antigens and have relatively high affinity for antigens. Based on their uniqueness, V$_H$H domains used alone or as part of a larger antigen-binding molecule have more significant advantages over conventional scFvs and antibody fragments such as Fabs: for example, only a single domain is required to specifically bind to an antigen with high affinity; they can be transformed into polyvalent and multispecific formats; V$_H$H domains are highly soluble and do not tend to aggregate; the molecules are small and thus demonstrate relatively high tissue permeability; single-domain antibodies do not need to be paired with light chains, and thus there is no light and heavy chain mismatch problem about forming multispecific antibodies, and the like. Multispecific antibodies targeting B-cell maturation antigen (BCMA) and T cell CD3 receptor can redirect CD3 T cells to BCMA-expressing myeloma cells to induce cytotoxicity against the target cells.

As a potential therapeutic target, some BCMA-targeting antibodies have been developed, but still are limited. There is a need for more available options.

**SUMMARY**

The present disclosure provides a BCMA-targeting antibody, and relates to a multispecific antibody targeting BCMA and an activating T cell antigen such as CD3. The present disclosure also provides related nucleic acids that can encode the provided antibody, a vector, a cell, a composition, a preparation method, and use.

In one aspect, the present disclosure provides a multispecific antibody, comprising

(i) a first antigen-binding moiety that binds to a first antigen;
(ii) a second antigen-binding moiety that binds to a second antigen; and
(iii) a third antigen-binding moiety that binds to the first antigen;

wherein the first antigen is BCMA and the second antigen is an activating T cell antigen, and the first antigen-binding moiety and the third antigen-binding moiety are both single variable domains and each independently comprise any one of:

(a) CDR1 comprising an amino acid sequence set forth in SEQ ID NO: 7, CDR2 comprising an amino acid sequence set forth in SEQ ID NO: 8, and CDR3 comprising an amino acid sequence set forth in SEQ ID NO: 9;
(b) CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12;
(c) CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15; or
(d) CDR1 comprising an amino acid sequence set forth in SEQ ID NO: 16, CDR2 comprising an amino acid sequence set forth in SEQ ID NO: 17, and CDR3 comprising an amino acid sequence set forth in SEQ ID NO: 18.

In some embodiments, the first antigen-binding moiety and the third antigen-binding moiety each independently comprise any one of:

(b) CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12; or
(c) CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15.

In some embodiments, the first antigen-binding moiety comprises CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12; and the third antigen-binding moiety comprises CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15. Further, in some embodiments, the first antigen-binding moiety comprises an amino acid sequence set forth in SEQ ID NO: 41, and the third antigen-binding moiety comprises an amino acid sequence set forth in SEQ ID NO: 42. In some specific embodiments, the first antigen-binding moiety comprises an amino acid sequence set forth in SEQ ID NO: 37, and the third antigen-binding moiety comprises an amino acid sequence set forth in SEQ ID NO: 39.

In other embodiments, the third antigen-binding moiety comprises CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12; and the first antigen-binding moiety comprises CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15. Further, in some embodiments, the first antigen-binding moiety comprises an amino acid sequence set forth in SEQ ID NO: 42, and the third antigen-binding moiety comprises an amino acid sequence set forth in SEQ ID NO: 41. In some specific embodiments, the first antigen-binding moiety comprises an amino acid sequence set forth in SEQ ID NO: 39, and the third antigen-binding moiety comprises an amino acid sequence set forth in SEQ ID NO: 37.

In some embodiments, the first antigen-binding moiety and the third antigen-binding moiety bind to different epitopes of BCMA.

The second antigen-binding moiety provides the ability to bind to an activating T cell antigen. In some embodiments, the second antigen-binding moiety comprises a heavy chain variable region and a light chain variable region, wherein the heavy chain variable region comprises the following complementarity determining regions: HCDR1 comprising an amino acid sequence set forth in SEQ ID NO: 43, HCDR2 comprising an amino acid sequence set forth in SEQ ID NO: 44, and HCDR3 comprising an amino acid sequence set forth in SEQ ID NO: 45; the light chain variable region comprises LCDR1 comprising an amino acid sequence set forth in SEQ ID NO: 46, LCDR2 comprising an amino acid sequence set forth in SEQ ID NO: 47, and LCDR3 comprising an amino acid sequence set forth in SEQ ID NO: 48. In further specific embodiments, the heavy chain variable region of the second antigen-binding moiety comprises an amino acid sequence set forth in SEQ ID NO: 49, and the light chain variable region thereof comprises an amino acid sequence set forth in SEQ ID NO: 50.

In some embodiments, the multispecific antibody further comprises an Fc domain composed of two Fc polypeptides.

In some specific embodiments, in the multispecific antibody, the first antigen-binding moiety comprises CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12; the third antigen-binding moiety comprises CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15; and the second antigen-binding moiety comprises a heavy chain variable region and a light chain variable region, wherein the heavy chain variable region comprises the following complementarity determining regions: HCDR1 comprising an amino acid sequence set forth in SEQ ID NO: 43, HCDR2 comprising an amino acid sequence set forth in SEQ ID NO: 44, and HCDR3 comprising an amino acid sequence set forth in SEQ ID NO: 45; the light chain variable region comprises LCDR1 comprising an amino acid sequence set forth in SEQ ID NO: 46, LCDR2 comprising an amino acid sequence set forth in SEQ ID NO: 47, and LCDR3 comprising an amino acid sequence set forth in SEQ ID NO: 48. In further specific embodiments, the first antigen-binding moiety comprises an amino acid sequence set forth in SEQ ID NO: 41, the third antigen-binding moiety comprises an amino acid sequence set forth in SEQ ID NO: 42, and the heavy chain variable region of the second antigen-binding moiety comprises an amino acid sequence set forth in SEQ ID NO: 49 and the light chain variable region thereof comprises an amino acid sequence set forth in SEQ ID NO: 50. In further specific embodiments, the first antigen-binding moiety comprises an amino acid sequence set forth in SEQ ID NO: 37, the third antigen-binding moiety comprises an amino acid sequence set forth in SEQ ID NO: 39, and the heavy chain variable region of the second antigen-binding moiety comprises an amino acid sequence set forth in SEQ ID NO: 49

and the light chain variable region thereof comprises an amino acid sequence set forth in SEQ ID NO: 50. In the specific embodiments, in a selectable configuration, the first antigen-binding moiety and the third antigen-binding moiety are both single variable domains, the second antigen-binding moiety is an ScFv, the first antigen-binding moiety, at its C-terminus, is fused to the N-terminus of one Fc polypeptide of the Fc domain, the second antigen-binding moiety, at its C-terminus, is fused to the N-terminus of the other Fc polypeptide of the Fc domain, and the third antigen-binding moiety, at its C-terminus, is fused to the N-terminus of the second antigen-binding moiety. In a further more specific embodiment, the multispecific antibody consists of two polypeptide chains, wherein the first polypeptide chain comprises the first antigen-binding moiety and one Fc polypeptide of the Fc domain described above, and the second polypeptide chain comprises the second antigen-binding moiety, the third antigen-binding moiety, and the other Fc polypeptide of the Fc domain described above.

In other specific embodiments, in the multispecific antibody, the third antigen-binding moiety comprises CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12; the first antigen-binding moiety comprises CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15; and the second antigen-binding moiety comprises a heavy chain variable region and a light chain variable region, wherein the heavy chain variable region comprises the following complementarity determining regions: HCDR1 comprising an amino acid sequence set forth in SEQ ID NO: 43, HCDR2 comprising an amino acid sequence set forth in SEQ ID NO: 44, and HCDR3 comprising an amino acid sequence set forth in SEQ ID NO: 45; the light chain variable region comprises LCDR1 comprising an amino acid sequence set forth in SEQ ID NO: 46, LCDR2 comprising an amino acid sequence set forth in SEQ ID NO: 47, and LCDR3 comprising an amino acid sequence set forth in SEQ ID NO: 48. In further specific embodiments, the first antigen-binding moiety comprises an amino acid sequence set forth in SEQ ID NO: 42, the third antigen-binding moiety comprises an amino acid sequence set forth in SEQ ID NO: 41, and the heavy chain variable region of the second antigen-binding moiety comprises an amino acid sequence set forth in SEQ ID NO: 49 and the light chain variable region thereof comprises an amino acid sequence set forth in SEQ ID NO: 50. In further specific embodiments, the first antigen-binding moiety comprises an amino acid sequence set forth in SEQ ID NO: 39, the third antigen-binding moiety comprises an amino acid sequence set forth in SEQ ID NO: 37, and the heavy chain variable region of the second antigen-binding moiety comprises an amino acid sequence set forth in SEQ ID NO: 49 and the light chain variable region thereof comprises an amino acid sequence set forth in SEQ ID NO: 50. In the embodiments, in a selectable configuration, the first antigen-binding moiety and the third antigen-binding moiety are both single variable domains, the second antigen-binding moiety is an Fab, the first antigen-binding moiety, at its C-terminus, is fused to the N-terminus of one Fc polypeptide of the Fc domain, the second antigen-binding moiety, at the C-terminus of its Fab heavy chain, is fused to the N-terminus of the other Fc polypeptide of the Fc domain, and the third antigen-binding moiety, at its C-terminus, is fused to the N-terminus of the first antigen-binding moiety. For the embodiment, in further more specific embodiments, the multispecific antibody consists of three polypeptide chains, wherein the first polypeptide chain comprises the first antigen-binding moiety, the third antigen-binding moiety, and one Fc polypeptide of the Fc domain described above, the second polypeptide chain comprises the Fab heavy chain of the second antigen-binding moiety and the other Fc polypeptide of the Fc domain described above, and the third polypeptide chain is the Fab light chain of the second antigen-binding moiety described above. In another selectable configuration, the first antigen-binding moiety and the third antigen-binding moiety are both single variable domains, the second antigen-binding moiety is an ScFv, the first antigen-binding moiety, at its C-terminus, is fused to the N-terminus of one Fc polypeptide of the Fc domain, the second antigen-binding moiety, at its C-terminus, is fused to the N-terminus of the other Fc polypeptide of the Fc domain, and the third antigen-binding moiety, at its C-terminus, is fused to the N-terminus of the first antigen-binding moiety. For the embodiment, in further more specific embodiments, the multispecific antibody consists of two polypeptide chains, wherein the first polypeptide chain comprises the first antigen-binding moiety, the third antigen-binding moiety, and one Fc polypeptide of the Fc domain described above, and the second polypeptide chain comprises the second antigen-binding moiety and the other Fc polypeptide of the Fc domain described above. In another selectable configuration, the first antigen-binding moiety and the third antigen-binding moiety are both single variable domains, the second antigen-binding moiety is an Fab, the first antigen-binding moiety, at its C-terminus, is fused to the N-terminus of one Fc polypeptide of the Fc domain, the second antigen-binding moiety, at the C-terminus of its Fab heavy chain, is fused to the N-terminus of the other Fc polypeptide of the Fc domain, and the third antigen-binding moiety, at its C-terminus, is fused to the N-terminus of the Fab light chain of the second antigen-binding moiety. For the embodiment, in further more specific embodiments, the multispecific antibody consists of three polypeptide chains, wherein the first polypeptide chain comprises the first antigen-binding moiety and one Fc polypeptide of the Fc domain described above, the second polypeptide chain comprises the Fab heavy chain of the second antigen-binding moiety and the other Fc polypeptide of the Fc domain described above, and the third polypeptide chain comprises the third antigen-binding moiety and the Fab light chain of the second antigen-binding moiety described above.

In one aspect, the present disclosure provides an isolated nucleic acid comprising a polynucleotide encoding the multispecific antibody.

In one aspect, the present disclosure provides a vector comprising the nucleic acid described herein.

In one aspect, the present disclosure provides a host cell comprising the nucleic acid or the vector described herein.

In another aspect, the present disclosure provides a method for preparing the multispecific antibody described herein, comprising culturing the host cell to express the multispecific antibody, and isolating and purifying the multispecific antibody in the system.

In another aspect, the present disclosure provides a pharmaceutical composition comprising the multispecific antibody and a pharmaceutically acceptable carrier.

In another aspect, the present disclosure provides use of the multispecific antibody or the pharmaceutical composition in preparing a medicament for treating a disease related to BCMA expression.

In another aspect, the present disclosure provides a method for treating a disease related to BCMA expression in a subject, comprising administering to the subject a therapeutically effective amount of the multispecific antibody or the pharmaceutical composition.

In yet another aspect, the present disclosure provides a BCMA-binding antibody comprising a single variable domain, wherein the single variable domain comprises:

(1) CDR1 comprising an amino acid sequence set forth in SEQ ID NO: 7, CDR2 comprising an amino acid sequence set forth in SEQ ID NO: 8, and CDR3 comprising an amino acid sequence set forth in SEQ ID NO: 9;

(2) CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12;

(3) CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15; or

(4) CDR1 comprising an amino acid sequence set forth in SEQ ID NO: 16, CDR2 comprising an amino acid sequence set forth in SEQ ID NO: 17, and CDR3 comprising an amino acid sequence set forth in SEQ ID NO: 18.

In some embodiments, the single variable domain comprises an amino acid sequence set forth in SEQ ID NO: 41 or 42, while the single variable domain does not comprise an amino acid sequence set forth in SEQ ID NO: 21 or 23.

The present disclosure provides a multispecific antibody targeting BCMA and an activating T cell antigen such as CD3. In some embodiments, a variety of forms of the multispecific antibody have been constructed using certain single variable domains, all of which exhibit a good anti-tumor effect such as tumor cell lysis performance and safety.

**BRIEF DESCRIPTION OF THE DRAWINGS**

FIG. 1A shows curves of flow cytometry assays for binding of anti-human BCMA $V_HH$-Fc chimeric antibodies to CHO-hBCMA cells.

FIG. 1B shows curves of flow cytometry assays for binding of anti-human BCMA $V_HH$-Fc chimeric antibodies to U266 cells.

FIG. 1C shows curves of flow cytometry assays for binding of anti-human BCMA $V_HH$-Fc chimeric antibodies to RPMI8226 cells.

FIG. 1D shows curves of flow cytometry assays for binding of anti-human BCMA $V_HH$-Fc chimeric antibodies to HUVEC cells.

FIG. 2 shows curves of flow cytometry assays for binding of 1A10-Fc and 1A11-Fc chimeric antibodies to HEK293T-CynoBCMA or HEK293T.

FIG. 3 shows the results of competitive ELISA assays for anti-human BCMA $V_HH$-Fc chimeric antibody and ligand APRIL.

FIG. 4A shows a comparison of the amino acid sequences of the extracellular regions of human BCMA and cynomolgus monkey BCMA.

FIG. 4B shows the crystal structure of the extracellular region of human BCMA.

FIG. 5 is a structural schematic view of exemplary multispecific antibodies of the present disclosure.

FIG. 6 is a structural schematic view of exemplary antibodies BC24, PC1, PC2, and PC3.

FIG. 7 shows curves for binding of anti-BCMA/anti-CD3 multispecific antibodies to NCI-H929 cells.

FIG. 8 shows curves for binding of anti-BCMA/anti-CD3 multispecific antibodies to MM1S cells.

FIG. 9 shows curves for binding of anti-BCMA/anti-CD3 multispecific antibodies to RPMI-8226 cells.

FIG. 10 shows curves for binding of anti-BCMA/anti-CD3 multispecific antibodies to CD3$^+$ T cells.

FIG. 11 shows the lysis rate of NCI-H929 tumor cells by effector cells induced by anti-BCMA/anti-CD3 multispecific antibodies at different concentrations.

FIG. 12 shows the lysis rate of MM1S tumor cells by effector cells induced by anti-BCMA/anti-CD3 multispecific antibodies at different concentrations.

FIG. 13 shows the lysis rate of RPMI-8226 tumor cells by effector cells induced by anti-BCMA/anti-CD3 multispecific antibodies at different concentrations.

FIG. 14 shows the effect of anti-BCMA/anti-CD3 multispecific antibodies on T cell activation in the presence of target cells NCI-H929.

FIG. 15 shows the effect of anti-BCMA/anti-CD3 multispecific antibodies on T cell activation in the presence of target cells MM1S.

FIG. 16 shows the release amount of cytokines IL-2, IL-6, TNF-$\alpha$, and IFN-$\gamma$ after incubation of different concentrations of anti-BCMA/anti-CD3 antibodies with NCI-H929 cells and effector cells for 24 h.

FIG. 17 shows the effect of anti-BCMA/anti-CD3 antibodies on the growth of NCI-H929 subcutaneous xenograft tumors.

FIG. 18 shows the LC-MS analysis results of the anti-BCMA/anti-CD3 antibody PC1.

## DETAILED DESCRIPTION OF DISCLOSURE

### Terminology

The term "antibody" refers to a protein comprising an antigen-binding site and encompasses natural and artificial antibodies of various structures, including but not limited to, monoclonal antibodies, monospecific antibodies, multispecific antibodies (e.g., bispecific antibodies, trispecific antibodies, and the like), single-chain antibodies, and the like.

The term "multispecific" means that an antibody is capable of specifically binding to a plurality of different antigenic determinants, e.g., capable of specifically binding to two or more different antigenic determinants. Generally, a bispecific antibody comprises two antigen-binding sites, each of which is specific for a different antigenic determinant. Different antigenic determinants may be expressed on the same or different cells. Different antigenic determinants may be different depending on different types of antigens (e.g., binding to antigens CD3 and BCMA) or may be present on the same antigen. An antigenic determinant is a special chemical group with a certain composition and structure on the surface or other parts of the antigenic substance molecule, which can specifically bind to its corresponding antibody or sensitized lymphocyte. An example of the antigenic determinant is an antigen B-cell maturation antigen (BCMA), which is an antigenic substance having various structurally defined or structurally undefined antigenic determinants. Antibodies which bind to two or more different antigenic determinants on an antigen are referred to herein as multispecific antibodies.

The term "N valent antibody" is intended to indicate the presence of N antigen-binding sites in the antibody. For example, a "bivalent antibody" refers to the presence of two antigen-binding sites in the antibody, and "trivalent antibody" refers to the presence of three antigen-binding sites in the antibody. A natural human immunoglobulin molecule typically has two antigen-binding sites, and an Fab typically has a single antigen-binding site. A single variable domain and an ScFv typically have a single antigen-binding site.

The term "antigen-binding moiety" refers to a polypeptide molecule specifically binding to an antigenic determinant. A specific antigen-binding moiety may be an Fab, an ScFv, or a single variable domain. The antigenic determinant is synonymous with an antigen epitope herein.

The term "activating T cell antigen" refers to an antigen expressed on the surface of a T lymphocyte, such as a cytotoxic T lymphocyte, which upon interaction with an antibody is capable of inducing T cell activation. For example, the interaction of an antibody with an activating T cell antigen can induce T cell activation by triggering a signaling cascade of the T cell receptor complex. In a specific embodiment, the activating T cell antigen is CD3, for example, an $\varepsilon$ subunit of CD3.

The term "T cell activation" refers to one or more cell responses of a T lymphocyte, such as a cytotoxic T lymphocyte, selected from the group consisting of proliferation, differentiation, cytokine secretion, release of cytotoxic effector molecules, cytotoxic activity, expression of activation markers, and the like. The multispecific antibody disclosed herein is capable of inducing T cell activation. Suitable methods for measuring T cell activation are known in the art and are described herein.

The term "first", "second", or "third" used herein with respect to an antigen-binding moiety, an antigen, an Fc polypeptide, a peptide linker, a polypeptide chain, and the like are used for ease of distinction when one or more parts of each type are present. Unless specifically stated, the use of these terms is not intended to confer a particular order or orientation of the multispecific antibody. The term "fusion" means that the components (e.g., an Fab, an ScFv, an Fc polypeptide, etc.) are linked either directly or by peptide bonds via one or more peptide linkers.

The term "variable domain" or "variable region" refers to a domain of an antibody that is involved in the binding of the antibody to an antigen. For example, a natural four-chain antibody (e.g., derived from human, murine, and the like) has a heavy chain variable region (VH) and a light chain variable region (VL), and a heavy-chain antibody derived from an animal such as camelid or shark has a single variable domain. Each variable domain of a natural antibody consists essentially of four "framework regions" and three "complementarity determining regions". The four framework regions are referred to as framework region 1 (or FR1), framework region 2 (or FR2), framework region 3 (or FR3), and framework region 4 (or FR4), respectively. The framework regions are separated by three complementarity determining regions (or CDRs) referred to in the art and hereinafter as complementarity determining region 1 (or CDR1), complementarity determining region 2 (or CDR2), and complementarity determining region 3 (or CDR3), respectively. Thus, the general structure of a variable domain can be represented as follows: FR1-CDR1-FR2-CDR2-FR3-CDR3-FR4. Variable domains impart specificity for an antigen to an antibody by virtue of having an antigen-binding site.

The term "single variable domain" refers to a variable domain capable of specifically binding to an antigen epitope without being paired with other variable domains. A single variable domain typically has three CDRs (CDR1, CDR2, and CDR3) and is present on a single domain. In some cases, a single variable domain may be a heavy chain variable domain (e.g. VH), so long as it can form a single antigen-binding unit (i.e., a functional antigen-binding unit consisting essentially of a single variable domain; in this way, a single antigen-binding domain can form a functional antigen-binding unit without interacting with another variable domain). Another example of single variable domain is "VHH domain" (or simply "VHH" or "$V_H H$") of the family Camelidae.

"CDR" (complementarity determining region), also known as "hypervariable region (HVR)", typically refers to each region of antibody variable regions which are highly variable in sequence and/or form structurally defined loops. A natural four-chain antibody typically comprises six CDRs, three in the heavy chain variable region (HCDR1, HCDR2, and HCDR3) and three in the light chain variable region (LCDR1, LCDR2, and LCDR3). A heavy-chain antibody or a single variable domain typically has three CDRs (CDR1, CDR2, and CDR3).

There are currently many ways to define CDRs. The Kabat scheme defines CDRs based on sequence variability and is the most commonly used (Elvin A. Kabat, et al, *Sequences of Proteins of Immunological Interest,* 5th Edition, Public Health Service, National Institutes of Health, Bethesda, Md. (1991)), while the Chothia scheme defines CDRs based on the position of structural loops (Cyrus Chothia, et al, Canonical Structures for the Hypervariable Regions of Immunoglobulins, J. Mol. Biol. 196:901-917(1987)). The AbM scheme, a compromise between the Kabat scheme and the Chothia scheme, is used by the Oxford Molecular's AbM antibody modeling software. "Contact" defines CDRs based on the analysis of the crystal structure of available complexes. However, it should be noted that boundaries of the CDRs of variable regions of the same antibody based on different methods and definitions may differ, that is, CDR sequences of the variable regions of the same antibody defined by different methods may differ. Thus, where reference is made to an antibody defined with a particular CDR sequence defined herein, the scope of the antibody also encompasses antibodies defined by CDR sequences that are converted to other arbitrary definitions (e.g., Chothia, AbM definitions, and the like).

CDR Sequences given herein are generally defined according to the Kabat scheme (Elvin A. Kabat, et al, Sequences of Proteins of Immunological Interest, 5th Edition, Public Health Service, National Institutes of Health, Bethesda, Md. (1991)).

The term "framework region" or "FR" refers to amino acid residues of variable domains other than the CDR residues defined herein.

The term "Fab" refers to a protein consisting of VH and CH1 domains of the heavy chain and VL and CL domains of the light chain of an immunoglobulin. The Fab herein refers to an Fab molecule in its natural form, i.e., comprising an Fab heavy chain consisting of a heavy chain variable region VH and a constant region CH1 (VH-CH1, in a direction from N-terminus to C-terminus), and an Fab light chain consisting of a light chain variable region and a constant region CL (VL-CL, in a direction from N-terminus to C-terminus).

The term "scFv" includes the VH and VL domains of an immunoglobulin, wherein these domains are present in a single polypeptide chain. In some embodiments, scFv further comprises a peptide linker between the VH and VL domains that enables the scFv to form the required structure for antigen-binding.

The term "Fc domain" or "Fc" is used herein to define the C-terminal region of an immunoglobulin heavy chain, which comprises at least part of a constant region. The term includes natural sequence Fc and variant Fc. The C-terminal lysine (Lys447) of the Fc may or may not be present. Unless otherwise stated, amino acid residues in the Fc or constant region are numbered according to the EU numbering system, also referred to as the EU index, as described in Kabat, E.A. et al., Sequences of Proteins of Immunological Interest, 5th Ed., Public Health Service, National Institutes of Health, Bethesda, MD (1991), NIH Publication 91-3242. As used herein, an "Fc polypeptide" of an Fc domain refers to one of the two polypeptides that form a dimeric Fc domain. For example, the Fc polypeptide of an IgG Fc domain comprises IgG CH2 and IgG CH3 constant regions.

The term "effector function" refers to those biological activities attributable to the Fc domain of an antibody, which varies with antibody isotypes. Examples of antibody effector functions include: C1q binding and complement-dependent cytotoxicity (CDC), Fc receptor binding, antibody-dependent cellular cytotoxicity (ADCC), antibody-dependent cellular phagocytosis (ADCP), and the like.

The term "KD" as used herein refers to the dissociation constant, expressed in molar concentration (M). The KD value of an antibody can be determined using methods well known in the art. For example, a method for determining the KD of an antibody is to use surface plasmon resonance, e.g., to use a biosensor system, such as a Biacore system.

The term "treating" or "treatment" refers to an attempt to alter the natural course of a disease in a treated individual, and may be a clinical intervention performed for prophylaxis or during the course of clinical pathology. Desired therapeutic effects include, but are not limited to, preventing the occurrence or recurrence of diseases, relieving symptoms, reducing any direct or indirect pathological outcomes of diseases, preventing metastasis, slowing disease progression rates, improving or alleviating disease conditions, and regressing or improving prognosis.

The term "subject" includes any human or non-human animal. The term "non-human animal" includes all vertebrates, e.g., mammals and non-mammals, such as non-human primates, sheep, dogs, cats, horses, cows, chicken, amphibians, and reptiles. Preferably, the subject according to the present disclosure is a human. The terms "patient" and "subject" are used interchangeably unless otherwise indicated.

The term "isolated" means that a compound of interest (e.g., VHH, a multispecific antibody, an antibody, or a nucleic acid) has been isolated from its natural environment.

The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without any conservative replacements as part of the sequence identity. Alignment of amino acid sequences for identity can be performed in a variety of ways within the skill in the art, such as BLAST, BLAST-2, ALIGN, or Megalign (DNASTAR) software. Those skilled in the art can determine suitable parameters for aligning sequences, including any algorithms required to obtain maximum alignment for the full length of sequences being compared.

Various aspects of the present disclosure will be described in further detail in the following sections.

*I. Single variable domain*

The present disclosure provides a single variable domain that binds to BCMA (such as human BCMA). The single variable domain provides more available options for the development or drug construction of BCMA-targeting drugs. The single variable domain has good affinity for human BCMA, and can provide targeting property. In certain cases, the single variable domain is capable of providing the ability to block the binding of the proliferation-inducing ligand APRIL to BCMA. In particular, in some embodiments, the single variable domain does not bind to human TACI and BAFF-R proteins, exhibiting specificity; in some embodiments, there are cross-reactions with monkey BCMA, which are beneficial to drug toxicology experiments since monkeys are the ideal experimental animal for drug toxicology experiments.

The present disclosure provides a BCMA-binding single variable domain, comprising:

(1) CDR1 comprising an amino acid sequence set forth in SEQ ID NO: 7, CDR2 comprising an amino acid sequence set forth in SEQ ID NO: 8, and CDR3 comprising an amino acid sequence set forth in SEQ ID NO: 9;
(2) CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12;
(3) CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15; or
(4) CDR1 comprising an amino acid sequence set forth in SEQ ID NO: 16, CDR2 comprising an amino acid sequence set forth in SEQ ID NO: 17, and CDR3 comprising an amino acid sequence set forth in SEQ ID NO: 18.

In some embodiments, the single variable domain comprises CDR1 having an amino acid sequence set forth in SEQ ID NO: 7, CDR2 having an amino acid sequence set forth in SEQ ID NO: 8, and CDR3 having an amino acid sequence set forth in SEQ ID NO: 9. In some embodiments, the single variable domain comprises CDR1 having an amino acid sequence set forth in SEQ ID NO: 10, CDR2 having an amino acid sequence set forth in SEQ ID NO: 11, and CDR3 having an amino acid sequence set forth in SEQ ID NO: 12. In some embodiments, the single variable domain comprises CDR1 having an amino acid sequence set forth in SEQ ID NO: 13, CDR2 having an amino acid sequence set forth in SEQ ID NO: 14, and CDR3 having an amino acid sequence set forth in SEQ ID NO: 15. In some embodiments, the single variable domain comprises CDR1 having an amino acid sequence set forth in SEQ ID NO: 16, CDR2 having an amino acid sequence set forth in SEQ ID NO: 17, and CDR3 having an amino acid sequence set forth in SEQ ID NO: 18. In some embodiments, the single variable domain is derived from animals of the family Camelidae. In some embodiments, the single variable domain is humanized.

In some embodiments, the single variable domain comprises an amino acid sequence having at least 85%, 90%, 91%, 92%, 93%, 94%, 95%, 96%, 97%, 98%, 99%, or 100% identity to a sequence set forth in SEQ ID NO: 19, 21, 23, 25, 37, 38, 39, 40, 41, or 42. In some more specific embodiments, the single variable domain comprises an amino acid sequence having at least 85%, 90%, 91%, 92%, 93%, 94%, 95%, 96%, 97%, 98%, 99%, or 100% identity to a sequence set forth in SEQ ID NO: 19, and comprises: CDR1 comprising an amino acid sequence set forth in SEQ ID NO: 7, CDR2 comprising an amino acid sequence set forth in SEQ ID NO: 8, and CDR3 comprising an amino acid sequence set forth in SEQ ID NO: 9. In some more specific embodiments, the single variable domain comprises an amino acid sequence having at least 85%, 90%, 91%, 92%, 93%, 94%, 95%, 96%, 97%, 98%, 99%, or 100% identity to a sequence set forth in SEQ ID NO: 41, and comprises: CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12. In some more specific embodiments, the single variable domain comprises an amino acid sequence having at least 85%, 90%, 91%, 92%, 93%, 94%, 95%, 96%, 97%, 98%, 99%, or 100% identity to a sequence set forth in SEQ ID NO: 37 or 38, and comprises: CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12. In some more specific embodiments, the single variable domain comprises an amino acid sequence having at least 85%, 90%, 91%, 92%, 93%, 94%, 95%, 96%, 97%, 98%, 99%, or 100% identity to a sequence set forth in SEQ ID NO: 42, and comprises: CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15. In some more specific embodiments, the single variable domain comprises an amino acid sequence having at least 85%, 90%, 91%, 92%, 93%, 94%, 95%, 96%, 97%, 98%, 99%, or 100% identity to a sequence set forth in SEQ ID NO: 39 or 40, and comprises: CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15. In some more specific embodiments, the single variable domain comprises an amino acid sequence having at least 85%, 90%, 91%, 92%, 93%, 94%, 95%, 96%, 97%, 98%, 99%, or 100% identity to a sequence set forth in SEQ ID NO: 25, and comprises: CDR1 comprising an amino acid sequence set forth in SEQ ID NO: 16, CDR2 comprising an amino acid sequence set forth in SEQ ID NO: 17, and CDR3 comprising an amino acid sequence set forth in SEQ ID NO: 18. In some embodiments, an amino acid sequence having at least 85%, 90%, 91%, 92%, 93%, 94%, 95%, 96%, 97%, 98%, or 99% identity comprises substitutions (e.g., conservative substitutions), insertions or deletions compared to a reference sequence; however, single variable domains comprising the sequence retain the ability to bind to BCMA. In some embodiments, a total of 1-18, 1-16, 1-14, 1-12, 1-10, 1-9, 1-8, 1-7, 1-6, 1-5, 1-4, 1-3, or 1-2 amino acids are substituted, inserted and/or deleted in an amino acid sequence selected from SEQ ID NOs: 19, 41, 42, and 25. In some embodiments, the substitutions, insertions or deletions occur in regions outside of CDRs (i.e., in FRs). In some embodiments, the substitutions, insertions or deletions occur in CDR regions, for example, in one, two, or three of CDR1, CDR2, and CDR3. In some embodiments, the substitutions, insertions or deletions occur in CDR regions and non-CDR regions.

In some embodiments, the amino acid sequence of the single variable domain comprises an amino acid sequence set forth in SEQ ID NO: 19, 21, 23, 25, 37, 38, 39, 40, 41, or 42.

In some embodiments, the amino acid sequence of the single variable domain is set forth in SEQ ID NO: 19, 21, 23, 25, 37, 38, 39, 40, 41, or 42.

In some embodiments, the single variable domain is a $V_HH$. In some embodiments, the $V_HH$ is humanized. A single variable domain of non-human origin may be "humanized" by replacing one or more amino acid residues in the amino acid sequence of the original single variable domain sequence with one or more amino acid residues present at corresponding positions in the VH domain of a human antibody. Humanization may be desirable to reduce immunogenicity. Typically a single variable domain has the following structure from N-terminus to C-terminus: FR1-CDR1-FR2-CDR2-FR3 -CDR3 -FR4.

In some embodiments, the present disclosure provides a single variable domain that binds to the same epitope as any one of the single variable domains described herein. In some specific embodiments, provided is a single variable domain that binds to the same epitopes as a single variable domain comprising an amino acid sequence of SEQ ID NO: 19, 21, 23, 25, 37, 38, 39, or 40. In some embodiments, the single variable domain that binds to the same epitopes is derived from animals of the family Camelidae or is humanized.

Based on the competitive binding to the same epitopes, screening of single variable domains can be carried out using conventional techniques known to those skilled in the art. Thus, in some embodiments, the present disclosure provides a single variable domain that competes for binding to BCMA with any one of the single variable domains described herein. In some specific embodiments, provided is a single variable domain that competes for binding to BCMA with a single variable domain comprising an amino acid sequence of SEQ ID NO: 19, 21, 23, 25, 37, 38, 39, or 40. The binding to BCMA can be measured by ELISA, flow cytometry or surface plasmon resonance (SPR) assay, or any other method known in the art. In some embodiments, the single variable domain that competes for binding to BCMA is derived from animals of the family Camelidae or is humanized.

The present disclosure provides some exemplary BCMA-binding single variable domains. The amino acid sequences of the CDRs (CDR1, CDR2, and CDR3) of the exemplary single variable domains provided herein are provided in Table S1 below. The full-length amino acid sequences of the exemplary single variable domains are provided in Table S2 below.

Table S1. CDR sequences of single variable domains

| Name | CDR1 | CDR2 | CDR3 |
|---|---|---|---|
| 1A1 single variable domain | GWNKH (SEQ ID NO: 7) | SIFRDGKTAYTDSVKG (SEQ ID NO: 8) | DLPGSGLPAF (SEQ ID NO: 9) |
| 1A10 single variable domain | VACMA (SEQ ID NO: 10) | TIVADFGTTNYAASVKG (SEQ ID NO: 11) | TQRGGIDWCDEINY (SEQ ID NO: 12) |
| 1A10-V1 single variable domain | VACMA (SEQ ID NO: 10) | TIVADFGTTNYAASVKG (SEQ ID NO: 11) | TQRGGIDWCDEINY (SEQ ID NO: 12) |
| 1A10-V2 single variable domain | VACMA (SEQ ID NO: 10) | TIVADFGTTNYAASVKG (SEQ ID NO: 11) | TQRGGIDWCDEINY (SEQ ID NO: 12) |
| 1A11 single variable domain | SACMG (SEQ ID NO: 13) | RIETGYGGTVYADSVKG (SEQ ID NO: 14) | KRSWCTPTWWHELDYNY (SEQ ID NO: 15) |
| 1A11-V1 single variable domain | SACMG (SEQ ID NO: 13) | RIETGYGGTVYADSVKG (SEQ ID NO: 14) | KRSWCTPTWWHELDYNY (SEQ ID NO: 15) |
| 1A11-V2 single variable domain | SACMG (SEQ ID NO: 13) | RIETGYGGTVYADSVKG (SEQ ID NO: 14) | KRSWCTPTWWHELDYNY (SEQ ID NO: 15) |
| 1B10 single variable domain | GWNKH (SEQ ID NO: 16) | SIFRDGKTAYTDSVKG (SEQ ID NO: 17) | DLPGSGLPEF (SEQ ID NO: 18) |

Table S2. Full length sequences of single variable domains

| Name | Amino acid sequence | SEQ ID NO: |
|---|---|---|
| 1A1 single variable domain | QVQLVESGGGSVQAGGSLRLSCTASGYTFRGWNKHWYRQA PGKERELVSSIFRDGKTAYTDSVKGRFTISQDNADATVYLQM NSLKPEDTAMYYCKYDLPGSGLPAFWGQGTLVTVSS | 19 |
| 1A10 single variable domain | QVQLVESGGGSVQAGGSLRLSCAASGYSSNVACMAWYRQA PGKEREWVATIVADFGTTNYAASVKGRFTISQDNAKNTVYL QMNSLKPEDSAMYYCAATQRGGIDWCDEINYWGQGTLVTV SS | 21 |
| 1A11 single variable domain | QVQLVESGGGSVQAGGSLRLSCAASGVTFNSACMGWFRQA PGKEREGVARIETGYGGTVYADSVKGRFTISRDNAKNTVYL QMNSLKPEDTAMYYCAAKRSWCTPTWWHELDYNYWGQG TQVTVSS | 23 |
| 1B10 single variable domain | EVQLVESGGGSVQAGGSLRLSCTASGYTFRGWNKHWYRQA PGKERELVSSIFRDGKTAYTDSVKGRFTISQDSADATVYLQM NSLKPEDTAMYYCKYDLPGSGLPEFWGQGTLVTVSS | 25 |
| 1A10-V1 single variable domain | QVQLVESGGGLVQPGGSLRLSCAASGYSSNVACMAWYRQA PGKGLEWVATIVADFGTTNYAASVKGRFTISQDNSKNTVYL QMNSLRAEDTAVYYCAATQRGGIDWCDEINYWGQGTLVTV SS | 37 |
| 1A10-V2 single variable domain | QVQLVESGGGLVQPGGSLRLSCAASGYSSNVACMAWYRQA PGKEREWVSTIVADFGTTNYAASVKGRFTISQDNSKNTVYL QMNSLRAEDTAVYYCAATQRGGIDWCDEINYWGQGTLVTV SS | 38 |

(continued)

| Name | Amino acid sequence | SEQ ID NO: |
|---|---|---|
| 1A11-V1 single variable domain | QVQLVESGGGLVQPGGSLRLSCAASGFTFNSACMGWFRQAP GKGREGVSRIETGYGGTVYADSVKGRFTISRDNSKNTVYLQ MNSLRAEDTAVYYCAAKRSWCTPTWWHELDYNYWGQGT QVTVSS | 39 |
| 1A11-V2 single variable domain | QVQLVESGGGLVQPGGSLRLSCAASGFTFNSACMGWFRQAP GKEREGVSRIETGYGGTVYADSVKGRFTISRDNSKNTVYLQ MNSLKAEDTAVYYCAAKRSWCTPTWWHELDYNYWGQGT QVTVSS | 40 |

Sequence of SEQ ID NO: 41:

QVQLVESGGG$X_1$VQX$_2$GGSLRLSCAASGYSSNVACMAWYRQAPGKX$_3$X$_4$EWVX$_5$TIVADF GTTNYAASVKGRFTISQDNX$_6$KNTVYLQMNSLX$_7$X$_8$EDX$_9$AX$_{10}$YYCAATQRGGIDWCDEI NYWGQGTLVTVSS

wherein $X_1$ is S or L, $X_2$ is P or A, $X_3$ is G or E, $X_4$ is R or L, $X_5$ is A or S, $X_6$ is A or S, $X_7$ is R or K, $X_8$ is A or P, $X_9$ is T or S, and $X_{10}$ is V or M.
Sequence of SEQ ID NO: 42:

QVQLVESGGG$X_{11}$VQX$_{12}$GGSLRLSCAASGX$_{13}$TFNSACMGWFRQAPGKX$_{14}$REGVX$_{15}$RIET GYGGTVYADSVKGRFTISRDNX$_{16}$KNTVYLQMNSLX$_{17}$X$_{18}$EDTAX$_{19}$YYCAAKRSWCTPTW WHELDYNYWGQGTQVTVSS

wherein $X_{11}$ is S or L, $X_{12}$ is P or A, $X_{13}$ is F or V, $X_{14}$ is E or G, $X_{15}$ is A or S, $X_{16}$ is A or S, $X_{17}$ is R or K, $X_{18}$ is A or P, and $X_{19}$ is V or M.

The present disclosure provides a BCMA-binding antibody comprising the single variable domain. In some embodiments, the BCMA-binding antibody may be a monospecific antibody or a multispecific antibody.

In one embodiment, the monospecific antibody comprises an Fc domain; preferably, the Fc is that of human IgG1, IgG2, IgG3, or IgG4.

In some embodiments, the monospecific antibody comprises an amino acid sequence having at least 85%, 90%, 91%, 92%, 93%, 94%, 95%, 96%, 97%, 98%, 99%, or 100% identity to a sequence set forth in SEQ ID NO: 27, 29, 31, 33, 51, 53, 55, or 57.

The present disclosure provides exemplary monospecific antibodies (e.g., 1A10-V1, 1A10-V1, 1A11-V1, 1A11-V2, and the like). The single variable domain is fused with Fc of human IgG1, forming a homodimer through Fc.

The present disclosure provides an isolated nucleic acid comprising a polynucleotide encoding the BCMA-binding antibody described herein.

The present disclosure provides a vector comprising the nucleic acid described herein.

The present disclosure provides an isolated host cell comprising the nucleic acid described herein or the vector described herein.

The present disclosure provides a method for preparing the BCMA-binding antibody described herein, comprising culturing the host cell described herein to express the BCMA-binding antibody, and isolating and purifying the BCMA-binding antibody in the system.

The present disclosure provides a pharmaceutical composition comprising the BCMA-binding antibody described herein and a pharmaceutically acceptable carrier.

The present disclosure provides a method for treating a disease related to BCMA expression in a subject, comprising administering to the subject a therapeutically effective amount of the BCMA-binding antibody or the pharmaceutical composition described herein.

*II. Multispecific antibody*

The present disclosure provides a multispecific antibody comprising a single variable domain that can target BCMA expressed on the surface of a tumor cell and can also bind to a T cell to activate the tumor killing activity of the T cell.

With a single variable domain, mismatches between the light and heavy chains can be reduced or avoided compared to more (e.g., two) forms with an Fab.

The multispecific antibody disclosed herein also exhibits excellent anti-tumor properties such as lysing tumor cells. In some embodiments, the multispecific antibody disclosed herein is capable of inducing killing of a variety of tumor cells, and the multispecific antibody disclosed herein exhibits good tumor killing activity against tumor cells of different BCMA expression levels. In particular, the multispecific antibody disclosed herein shows more excellent killing performance to some tumor cells with low expression in BCMA.

The multispecific antibody disclosed herein has low cytokine release level while showing good antitumor performance, can reduce the risk of a cytokine storm caused by the antibody targeting CD3, and improves safety.

The multispecific antibody may be constructed using any one of the single variable domains described in section I. Thus, the present disclosure provides a multispecific antibody, comprising

(i) a first antigen-binding moiety that binds to a first antigen;
(ii) a second antigen-binding moiety that binds to a second antigen; and
(iii) a third antigen-binding moiety that binds to the first antigen;

wherein the first antigen is BCMA and the second antigen is an activating T cell antigen, and the first antigen-binding moiety and the third antigen-binding moiety are both single variable domains and each independently comprise any one of:

(a) CDR1 comprising an amino acid sequence set forth in SEQ ID NO: 7, CDR2 comprising an amino acid sequence set forth in SEQ ID NO: 8, and CDR3 comprising an amino acid sequence set forth in SEQ ID NO: 9;
(b) CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12;
(c) CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15; or
(d) CDR1 comprising an amino acid sequence set forth in SEQ ID NO: 16, CDR2 comprising an amino acid sequence set forth in SEQ ID NO: 17, and CDR3 comprising an amino acid sequence set forth in SEQ ID NO: 18.

In the present disclosure, the first antigen-binding moiety and the third antigen-binding moiety are both BCMA-binding single variable domains. In some embodiments, the first antigen-binding moiety comprises a complementary determining region described in (a), (b), (c), or (d) above. In some embodiments, the third antigen-binding moiety comprises a complementarity determining region described in (a), (b), (c), or (d) above. In the multispecific antibody, the first antigen-binding moiety and the third antigen-binding moiety may be identical or different, and any single variable domain described herein may be independently selected for combination; in a specific embodiment, the first antigen-binding moiety and the third antigen-binding moiety are identical; in another specific embodiment, the first antigen-binding moiety is different from the third antigen-binding moiety.

Multispecific antibodies constructed with different combinations of a first antigen-binding moiety and a third antigen-binding moiety with a defined CDR feature are illustratively listed in Table S3. In a specific embodiment, the first antigen-binding moiety comprises the complementarity determining region described in (b) above, and the third antigen-binding moiety comprises the complementarity determining region described in (c) above, i.e. the first antigen-binding moiety comprises CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12; and the third antigen-binding moiety comprises CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15. In another specific embodiment, the third antigen-binding moiety comprises the complementarity determining region described in (b) above, and the first antigen-binding moiety comprises the complementarity determining region described in (c) above, i.e. the third antigen-binding moiety comprises CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12; and the first antigen-binding moiety comprises CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15. In a more specific embodiment, the first antigen-binding moiety comprises CDR1 set forth in SEQ ID NO: 10, CDR2 set forth in SEQ ID NO: 11, and CDR3 set forth in SEQ ID NO: 12; and the third antigen-binding moiety comprises CDR1 set forth in SEQ ID NO: 13, CDR2 set forth in SEQ ID NO: 14, and CDR3 set forth in SEQ ID NO: 15. In another more specific embodiment, the third antigen-binding moiety comprises CDR1 set forth in SEQ ID NO: 10, CDR2 set forth in SEQ ID NO: 11, and CDR3 set forth in SEQ ID NO: 12; and the first antigen-binding moiety comprises CDR1 set forth in SEQ ID NO: 13, CDR2 set forth in SEQ ID NO: 14, and CDR3 set forth in SEQ ID NO: 15.

Table S3. Exemplary multispecific antibodies in which the CDR feature of a first antigen-binding moiety and a third antigen-binding moiety is defined

| First antigen-binding moiety / Third antigen-binding moiety | (a) | (b) | (c) | (d) |
|---|---|---|---|---|
| (a) | (a)+ (a) | (a)+ (b) | (a) +(c) | (a) +(d) |
| (b) | (b) +(a) | (b) +(b) | (b) +(c) | (b) +(d) |
| (c) | (c) +(a) | (c) +(b) | (c) +(c) | (c) +(d) |
| (d) | (d) +(a) | (d) +(b) | (d) +(c) | (d) +(d) |

Note: in the table, (X) + (Y) indicates the combination of the third antigen-binding moiety and the first antigen-binding moiety of the multispecific antibody. For example, (b) + (a) indicates that the third antigen-binding moiety of the multispecific antibody comprises a complementarity determining region described in (b), while the first antigen-binding moiety comprises a complementarity determining region described in (a).

Further, in some embodiments, the first antigen-binding moiety and the third antigen-binding moiety each independently comprise an amino acid sequence having at least 85%, 86%, 87%, 88%, 89%, 90%, 91%, 92%, 93%, 94%, 95%, 96%, 97%, 98%, 99%, or 100% identity to a sequence set forth in SEQ ID NO: 19, 41, 42, or 25.

In some embodiments, the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to a sequence set forth in SEQ ID NO: 19, 41, 42, or 25. In some embodiments, the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to a sequence set forth in SEQ ID NO: 19, 21, 23, 37, 38, 39, 40, or 25. In some specific embodiments, the first antigen-binding moiety comprises an amino acid sequence set forth in SEQ ID NO: 19, 41, 42, or 25. In some more specific embodiments, the first antigen-binding moiety comprises an amino acid sequence set forth in SEQ ID NO: 19, 21, 23, 37, 38, 39, 40, or 25.

In some embodiments, the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to a sequence set forth in SEQ ID NO: 19, 41, 42, or 25. In some embodiments, the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to a sequence set forth in SEQ ID NO: 19, 21, 23, 37, 38, 39, 40, or 25. In some specific embodiments, the third antigen-binding moiety comprises an amino acid sequence set forth in SEQ ID NO: 19, 41, 42, or 25. In some more specific embodiments, the third antigen-binding moiety comprises an amino acid sequence set forth in SEQ ID NO: 19, 21, 23, 37, 38, 39, 40, or 25.

In some specific embodiments, the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to a sequence set forth in SEQ ID NO: 41; and the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to a sequence set forth in SEQ ID NO: 42. In a specific embodiment, the first antigen-binding moiety comprises an amino acid sequence set forth in SEQ ID NO: 41, and the third antigen-binding moiety comprises an amino acid sequence set forth in SEQ ID NO: 42. In some more specific embodiments, the first antigen-binding moiety comprises an amino acid sequence set forth in SEQ ID NO: 21, 37, or 38, and the third antigen-binding moiety comprises an amino acid sequence set forth in SEQ ID NO: 23, 39, or 40.

In some embodiments, the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to a sequence set forth in SEQ ID NO: 42, and the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to a sequence set forth in SEQ ID NO: 41. In a specific embodiment, the first antigen-binding moiety comprises an amino acid sequence set forth in SEQ ID NO: 42, and the third antigen-binding moiety comprises an amino acid sequence set forth in SEQ ID NO: 41. In some more specific embodiments, the first antigen-binding moiety comprises an amino acid sequence set forth in SEQ ID NO: 23, 39, or 40, and the third antigen-binding moiety comprises an amino acid sequence set forth in SEQ ID NO: 21, 37, or 38.

In one example, the first antigen-binding moiety comprises an amino acid sequence set forth in SEQ ID NO: 21, and the third antigen-binding moiety comprises an amino acid sequence set forth in SEQ ID NO: 21, 23, 37, 38, 39, or 40.

In one example, the third antigen-binding moiety comprises an amino acid sequence set forth in SEQ ID NO: 21, and the first antigen-binding moiety comprises an amino acid sequence set forth in SEQ ID NO: 21, 23, 37, 38, 39, or 40.

In one example, the first antigen-binding moiety comprises an amino acid sequence set forth in SEQ ID NO: 37, and the third antigen-binding moiety comprises an amino acid sequence set forth in SEQ ID NO: 39.

In one example, the first antigen-binding moiety comprises an amino acid sequence set forth in SEQ ID NO: 37, and the third antigen-binding moiety comprises an amino acid sequence set forth in SEQ ID NO: 38.

In one example, the first antigen-binding moiety comprises an amino acid sequence set forth in SEQ ID NO: 37, and the third antigen-binding moiety comprises an amino acid sequence set forth in SEQ ID NO: 40.

In one example, the first antigen-binding moiety comprises an amino acid sequence set forth in SEQ ID NO: 38, and the third antigen-binding moiety comprises an amino acid sequence set forth in SEQ ID NO: 39.

In one example, the first antigen-binding moiety comprises an amino acid sequence set forth in SEQ ID NO: 38, and the third antigen-binding moiety comprises an amino acid sequence set forth in SEQ ID NO: 40.

In one example, the first antigen-binding moiety comprises an amino acid sequence set forth in SEQ ID NO: 39, and the third antigen-binding moiety comprises an amino acid sequence set forth in SEQ ID NO: 40.

In one example, the third antigen-binding moiety comprises an amino acid sequence set forth in SEQ ID NO: 37, and the first antigen-binding moiety comprises an amino acid sequence set forth in SEQ ID NO: 39.

In one example, the third antigen-binding moiety comprises an amino acid sequence set forth in SEQ ID NO: 37, and the first antigen-binding moiety comprises an amino acid sequence set forth in SEQ ID NO: 38.

In one example, the third antigen-binding moiety comprises an amino acid sequence set forth in SEQ ID NO: 37, and the first antigen-binding moiety comprises an amino acid sequence of SEQ ID NO: 40.

In one example, the third antigen-binding moiety comprises an amino acid sequence set forth in SEQ ID NO: 38, and the first antigen-binding moiety comprises an amino acid sequence of SEQ ID NO: 39.

In one example, the third antigen-binding moiety comprises an amino acid sequence set forth in SEQ ID NO: 38, and the first antigen-binding moiety comprises an amino acid sequence of SEQ ID NO: 40.

In one example, the third antigen-binding moiety comprises an amino acid sequence set forth in SEQ ID NO: 39, and the first antigen-binding moiety comprises an amino acid sequence of SEQ ID NO: 40.

In one example, the first antigen-binding moiety and the third antigen-binding moiety both comprise an amino acid sequence set forth in SEQ ID NO: 21, 23, 37, 38, 39, or 40.

Table S4 illustrates multispecific antibodies constructed with different combinations of a first antigen-binding moiety and a third antigen-binding moiety with the feature of a full-length amino acid sequence.

Table S4. Exemplary multispecific antibodies in which the amino acid sequences of a first antigen-binding moiety and a third antigen-binding moiety are defined

| First antigen-binding moiety / Third antigen-binding moiety | 1A1 single variable domain | 1A10 single variable domain | 1A11 single variable domain | 1B10 single variable domain | 1A10-V1 single variable domain | 1A10-V2 single variable domain | 1A11-V1 single variable domain | 1A11-V2 single variable domain |
|---|---|---|---|---|---|---|---|---|
| 1A1 single variable domain | 1A1 + 1A1 single variable domains | 1A1 + 1A10 single variable domains | 1A1 + 1A11 single variable domains | 1A1 + 1B10 single variable domains | 1A1 + 1A10-V1 single variable domains | 1A1 + 1A10-V2 single variable domains | 1A1 + 1A11-V1 single variable domains | 1A1 + 1A11-V2 single variable domains |
| 1A10 single variable domain | 1A10 + 1A1 single variable domains | 1A10 + 1A10 single variable domains | 1A10 + 1A11 single variable domains | 1A10 + 1B10 single variable domains | 1A10 + 1A10-V1 single variable domains | 1A10 + 1A10-V2 single variable domains | 1A10 + 1A11-V1 single variable domains | 1A10 + 1A11-V2 single variable domains |
| 1A11 single variable domain | 1A11 + 1A1 single variable domains | 1A11 + 1A10 single variable domains | 1A11 + 1A11 single variable domains | 1A11 + 1B10 single variable domains | 1A11 + 1A10-V1 single variable domains | 1A11 + 1A10-V2 single variable domains | 1A11 + 1A11-V1 single variable domains | 1A11 + 1A11-V2 single variable domains |
| 1B10 single variable domain | 1B10 + 1A1 single variable domains | 1B10 + 1A10 single variable domains | 1B10 + 1A11 single variable domains | 1B10 + 1B10 single variable domains | 1B10 + 1A10-V1 single variable domains | 1B10 + 1A10-V2 single variable domains | 1B10 + 1A11-V1 single variable domains | 1B10 + 1A11-V2 single variable domains |
| 1A10-V1 single variable domain | 1A10-V1 + 1A1 single variable domains | 1A10-V1 + 1A10 single variable domains | 1A10-V1 + 1A11 single variable domains | 1A10-V1 + 1B10 single variable domains | 1A10-V1 + 1A10-V1 single variable domains | 1A10-V1 + 1A10-V2 single variable domains | 1A10-V1 + 1A11-V1 single variable domains | 1A10-V1 + 1A11-V2 single variable domains |
| 1A10-V2 single variable domain | 1A10-V2 + 1A1 single variable domains | 1A10-V2 + 1A10 single variable domains | 1A10-V2 + 1A11 single variable domains | 1A10-V2 + 1B10 single variable domains | 1A10-V2 + 1A10-V1 single variable domains | 1A10-V2 + 1A10-V2 single variable domains | 1A10-V2 + 1A11-V1 single variable domains | 1A10-V2 + 1A11-V2 single variable domains |
| 1A11-V1 single variable domain | 1A11-V1 + 1A1 single variable domains | 1A11-V1 + 1A10 single variable domains | 1A11-V1 + 1A11 single variable domains | 1A11-V1 + 1B10 single variable domains | 1A11-V1 + 1A10-V1 single variable domains | 1A11-V1 + 1A10-V2 single variable domains | 1A11-V1 + 1A11-V1 single variable domains | 1A11-V1 + 1A11-V2 single variable domains |
| 1A11-V2 single variable domain | 1A11-V2 + 1A1 single variable domains | 1A11-V2 + 1A10 single variable domains | 1A11-V2 + 1A11 single variable domains | 1A11-V2 + 1B10 single variable domains | 1A11-V2 + 1A10-V1 single variable domains | 1A11-V2 + 1A10-V2 single variable domains | 1A11-V2 + 1A11-V1 single variable domains | 1A11-V2 + 1A11-V2 single variable domains |

Note: in the table, (X) + (Y) indicates the combination of the third antigen-binding moiety and the first antigen-binding moiety of the multispecific antibody. For example, "1A10 + 1A1 single variable domains" indicates that the third antigen-binding moiety of the multispecific antibody is the amino acid sequence of a 1A10 single variable domain, while the first antigen-binding moiety is the amino acid sequence of a 1A1 single variable domain; the amino acid sequences of the single variable domains referred to in the table are shown in Table S2.

In the present disclosure, the first antigen-binding moiety may be derived from animals of the family Camelidae or be humanized. The third antigen-binding moiety may be derived from animals of the family Camelidae or be humanized. Humanization may reduce immunogenicity, and in some embodiments, both the first antigen-binding moiety and the third antigen-binding moiety are humanized. In some embodiments, both the first antigen-binding moiety and the third antigen-binding moiety are derived from animals of the family Camelidae.

In the multispecific antibody disclosed herein, the second antigen-binding moiety provides the ability to target an activating T cell antigen. The second antigen-binding moiety may be an Fab, an ScFv, or an ScFab. In one embodiment, the activating T cell antigen is CD3. In a specific embodiment, the second antigen-binding moiety is an Fab that binds to CD3. In another specific embodiment, the second antigen-binding moiety is an ScFv that binds to CD3.

In some embodiments, the second antigen-binding moiety is murine, chimeric, or humanized.

In some embodiments, the second antigen-binding moiety comprises a heavy chain variable region and a light chain variable region, wherein the heavy chain variable region comprises the following complementarity determining regions: HCDR1 comprising an amino acid sequence set forth in SEQ ID NO: 43, HCDR2 comprising an amino acid sequence set forth in SEQ ID NO: 44, and HCDR3 comprising an amino acid sequence set forth in SEQ ID NO: 45; the light chain variable region comprises LCDR1 comprising an amino acid sequence set forth in SEQ ID NO: 46, LCDR2 comprising an amino acid sequence set forth in SEQ ID NO: 47, and LCDR3 comprising an amino acid sequence set forth in SEQ ID NO: 48.

In a specific embodiment, the heavy chain variable region comprises HCDR1, HCDR2, and HCDR3 of a variable region set forth in SEQ ID NO: 49; and the light chain variable region comprises LCDR1, HCDR2, and LCDR3 of a variable region set forth in SEQ ID NO: 50.

In some embodiments, the second antigen-binding moiety comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to a sequence set forth in SEQ ID NO: 49 and comprises a light chain variable region having an amino acid sequence having at least 85%, 86%, 87%, 88%, 89%, 90%, 91%, 92%, 93%, 94%, 95%, 96%, 97%, 98%, 99%, or 100% identity to a sequence set forth in SEQ ID NO: 50. In a specific embodiment, the heavy chain variable region of the second antigen-binding moiety comprises an amino acid sequence set forth in SEQ ID NO: 49, and the light chain variable region thereof comprises an amino acid sequence set forth in SEQ ID NO: 50. In a more specific embodiment, the heavy chain variable region and the light chain variable region of the second antigen-binding moiety are set forth in SEQ ID NOs: 49 and 50, respectively.

Table S5. CDR and variable region sequences of exemplary second antigen-binding moiety

| Region | Amino acid sequence | SEQ ID NO: |
|--------|---------------------|------------|
| CD3-HCDR1 | TYAMN | 43 |
| CD3-HCDR2 | RIRSKYNNYATYYADSVKD | 44 |
| CD3-HCDR3 | HGNFGNSYVSWFAY | 45 |
| CD3-LCDR1 | RSSTGAVTTSNYAN | 46 |
| CD3-LCDR2 | GTNKRAP | 47 |
| CD3-LCDR3 | ALWYSNLWV | 48 |
| CD3-VH | EVQLVESGGGLVQPGGSLRLSCAASGFTFNTYAMNW | 49 |
| | VRQAPGKGLEWVARIRSKYNNYATYYADSVKDRFTIS RDDSKNTAYLQMNNLKTEDTAMYYCVRHGNFGNSY VSWFAYWGQGTLVTVSS | |
| CD3-VL | ELVVTQEPSLTTSPGGTVTLTCRSSTGAVTTSNYANWV QQKPGQAPRGLIGGTNKRAPGTPARFSGSLLGGKAAL TITGVQPEDEAEYYCALWYSNLWVFGGGTKLTVL | 50 |

The multispecific antibody disclosed herein may have no Fc domain, and the first antigen-binding moiety, the second antigen-binding moiety, and the third antigen-binding moiety are connected by suitable linkers.

The multispecific antibody disclosed herein may have an Fc domain, and the Fc domain may prolong the half-life, provide the Fc domain-related effector, and the like.

Multispecific antibody configurations

The components of the multispecific antibody disclosed herein may be fused to one another in a variety of configurations. In some embodiments, the multispecific antibody further comprises (iv) an Fc domain composed of two Fc polypeptides.

The third antigen-binding moiety may be fused to the first antigen-binding moiety or the second antigen-binding moiety. In alternative embodiment (1), the third antigen-binding moiety and the first antigen-binding moiety are fused to each other, optionally via a peptide linker. In alternative embodiment (2), the third antigen-binding moiety and the second antigen-binding moiety are fused to each other, optionally via a peptide linker.

In alternative embodiment (1), the third antigen-binding moiety and the first antigen-binding moiety are fused to each other, optionally via a peptide linker. In a more specific embodiment, the third antigen-binding moiety, at its C-terminus, is fused to the N-terminus of the first antigen-binding moiety. Further, in some embodiments, the first antigen-binding moiety, at its C-terminus, is fused to the N-terminus of one Fc polypeptide of the Fc domain, the second antigen-binding moiety is an ScFv, and the second antigen-binding moiety, at its C-terminus, is fused to the N-terminus of the other Fc polypeptide of the Fc domain. In other embodiments, the first antigen-binding moiety, at its C-terminus, is fused to the N-terminus of one Fc polypeptide of the Fc domain, the second antigen-binding moiety is an Fab, and the second antigen-binding moiety, at the C-terminus of its Fab heavy chain, is fused to the N-terminus of the other Fc polypeptide of the Fc domain.

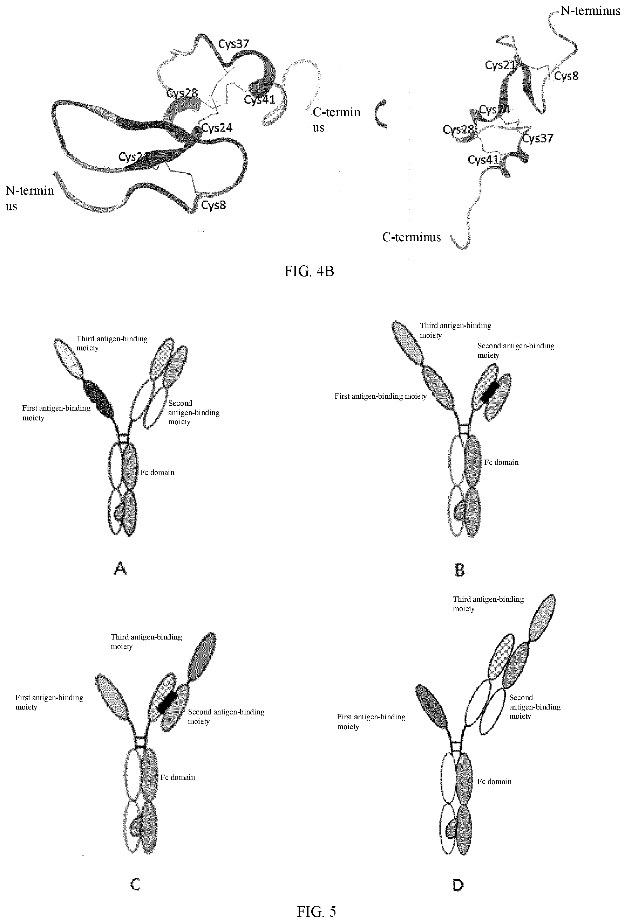

In a specific embodiment, the first antigen-binding moiety and the third antigen-binding moiety are both single variable domains, the second antigen-binding moiety is an Fab, the first antigen-binding moiety, at its C-terminus, is fused to the N-terminus of one Fc polypeptide of the Fc domain, the second antigen-binding moiety, at the C-terminus of its Fab heavy chain, is fused to the N-terminus of the other Fc polypeptide of the Fc domain, and the third antigen-binding moiety, at its C-terminus, is fused to the N-terminus of the first antigen-binding moiety. Such configurations are schematically depicted in FIG. 5A. For the embodiment, in further more specific embodiments, the multispecific antibody has three polypeptide chains, wherein the first polypeptide chain comprises the first antigen-binding moiety, the third antigen-binding moiety, and one Fc polypeptide of the Fc domain described above, the second polypeptide chain comprises the Fab heavy chain of the second antigen-binding moiety and the other Fc polypeptide of the Fc domain described above, and the third polypeptide chain is the Fab light chain of the second antigen-binding moiety described above.

In another specific embodiment, the first antigen-binding moiety and the third antigen-binding moieties are both single variable domains, the second antigen-binding moiety is an ScFv, the first antigen-binding moiety, at its C-terminus, is fused to the N-terminus of one Fc polypeptide of the Fc domain, the second antigen-binding moiety, at its C-terminus, is fused to the N-terminus of the other Fc polypeptide of the Fc domain, and the third antigen-binding moiety, at its C-terminus, is fused to the N-terminus of the first antigen-binding moiety. Such configurations are schematically depicted in FIG. 5B. For the embodiment, in further more specific embodiments, the multispecific antibody has two polypeptide chains, wherein the first polypeptide chain comprises the first antigen-binding moiety, the third antigen-binding moiety, and one Fc polypeptide of the Fc domain described above, and the second polypeptide chain comprises the second antigen-binding moiety and the other Fc polypeptide of the Fc domain described above.

For the connection mode of the third antigen-binding moiety, in alternative embodiment (2), the third antigen-binding moiety and the second antigen-binding moiety are fused to each other, optionally via a peptide linker. In more specific embodiments, the third antigen-binding moiety, at its C-terminus, is fused to the N-terminus of the second antigen-binding moiety. Further, in some embodiments, the first antigen-binding moiety, at its C-terminus, is fused to the N-terminus of one Fc polypeptide of the Fc domain, the second antigen-binding moiety is an ScFv, and the second antigen-binding moiety, at its C-terminus, is fused to the N-terminus of the other Fc polypeptide of the Fc domain. In other embodiments, the first antigen-binding moiety, at its C-terminus, is fused to the N-terminus of one Fc polypeptide of the Fc domain, the second antigen-binding moiety is an Fab, and the second antigen-binding moiety, at the C-terminus of its Fab heavy chain, is fused to the N-terminus of the other Fc polypeptide of the Fc domain. Wherein, The third antigen-binding moiety is fused to the N-terminus of the Fab light chain or the Fab heavy chain of the second antigen-binding moiety.

In a specific embodiment, the first antigen-binding moiety and the third antigen-binding moieties are both single variable domains, the second antigen-binding moiety is an ScFv, the first antigen-binding moiety, at its C-terminus, is fused to the N-terminus of one Fc polypeptide of the Fc domain, the second antigen-binding moiety, at its C-terminus, is fused to the N-terminus of the other Fc polypeptide of the Fc domain, and the third antigen-binding moiety, at its C-terminus, is fused to the N-terminus of the second antigen-binding moiety. Such configurations are schematically depicted in FIG. 5C. For the embodiment, further more specifically, the multispecific antibody has two polypeptide chains, wherein the first polypeptide chain comprises the first antigen-binding moiety and one Fc polypeptide of the Fc domain described above, and the second polypeptide chain comprises the second antigen-binding moiety, the third antigen-binding moiety, and the other Fc polypeptide of the Fc domain described above.

In another specific embodiment, the first antigen-binding moiety and the third antigen-binding moieties are both single variable domains, the second antigen-binding moiety is an Fab, the first antigen-binding moiety, at its C-terminus, is fused to the N-terminus of one Fc polypeptide of the Fc domain, the second antigen-binding moiety, at the C-terminus of its Fab heavy chain, is fused to the N-terminus of the other Fc polypeptide of the Fc domain, and the third antigen-binding moiety, at its C-terminus, is fused to the N-terminus of the Fab light chain of the second antigen-binding moiety (such configurations are schematically depicted in FIG. 5D.) or the N-terminus of the Fab heavy chain of the second antigen-binding moiety. For the embodiment, further more specifically, the multispecific antibody has three polypeptide chains, wherein the first polypeptide chain comprises the first antigen-binding moiety and one Fc polypeptide of the Fc domain described above, the second polypeptide chain comprises the Fab heavy chain of the second antigen-binding moiety and the other Fc polypeptide of the Fc domain described above, and the third polypeptide chain comprises the third antigen-binding moiety and the Fab light chain of the second antigen-binding moiety described above. Optionally, the multispecific antibody has three polypeptide chains, wherein the first polypeptide chain comprises the first antigen-binding moiety and one Fc polypeptide of the Fc domain described above, the second polypeptide chain comprises the Fab heavy chain of the second antigen-binding moiety, the third antigen-binding moiety, and the other Fc polypeptide of the Fc domain described above, and the third polypeptide chain comprises the Fab light chain of the second antigen-binding moiety described above.

When the second antigen-binding moiety described above is an ScFv, the heavy chain variable region (VH) and the light chain variable region (VL) included in the second antigen-binding moiety are arranged in any order. For example, in one embodiment, the VH and the VL of the second antigen-binding moiety are arranged in the order VL-VH from the N-terminus to the C-terminus; in another embodiment, the VH and the VL of the second antigen-binding moiety are arranged in the order VH-VL from the N-terminus to the C-terminus. In some embodiments, the VH and the VL of the second antigen-binding moiety are fused via a peptide linker to form an ScFv.

In any one of the embodiments described above, the components of the multispecific antibody are operably linked, e.g., fused directly or via various peptide linkers (e.g., peptide linkers comprising one or more amino acids, typically about 1-50 amino acids) as described herein or known in the art and hinges, which can be reasonably selected by those skilled in the art.

The third antigen-binding moiety may be fused to the first antigen-binding moiety or the second antigen-binding moiety either directly or via a peptide linker. In one embodiment, the third antigen-binding moiety is fused to the first antigen-binding moiety or the second antigen-binding moiety via a peptide linker. In some embodiments, when the second antigen-binding moiety is an ScFv structure, its light chain variable region and heavy chain variable region are connected via a peptide linker.

Each peptide linker may be independently any suitable, e.g., electrically charged and/or flexible, linker polypeptide. In a specific embodiment, the peptide linker consists of 1 to 50 amino acids linked by peptide bonds, wherein the amino acids may be selected from the group consisting of 20 naturally occurring amino acids; in a more preferred embodiment, the 1 to 50 amino acids are selected from the group consisting of glycine, alanine, proline, serine, asparagine, glutamine, and lysine. Thus, exemplary peptide linkers may be polyglycines (particularly (Gly)4 and (Gly)5), poly(Gly-Ser), (Gly)3AsnGlySer(Gly)2, (Gly)3Cys(Gly)4, GlyProAsnGlyGly, or those disclosed in Table 4 of patent application WO2019195535, etc.

In some embodiments, the peptide linker may be a peptide linker consisting of glycine and serine. In some embodiments, the peptide linker may comprise 0, 1, 2, 3, 4, 5, 6, 7, 8, 9, 10, 11, 12, 13, 14, 15, 16, 17, 18, 19, 20 or more amino acids. In some embodiments, the peptide linker is a peptide linker comprising GGGGS as a unit, a peptide linker comprising GGSGGSGGSGGSGG (SEQ ID NO: 2), or a peptide linker comprising GGGPGKR. Preferably, the peptide linker comprising GGGGS as a unit is (GGGGS)n, wherein n is any number between 1 and 10, i.e., 1, 2, 3, 4, 5, 6, 7, 8, 9, and 10, or any range defined by any two of the aforementioned numbers, e.g., 1-5, 2-5, 3-6, 2-4, and 1-4. In some specific embodiments, the peptide linker is a linker polypeptide comprising GGGGS, (GGGGS)2, (GGGGS)3, or (GGGGS)4. In some specific embodiments, the third antigen-binding moiety is fused to the first antigen-binding moiety or the second antigen-binding moiety via a peptide linker GGGGS (SEQ ID NO: 1) or (GGGGS)2 or (GGGGS)3. In some embodiments, when the second antigen-binding moiety is an ScFv structure, its light chain variable region and heavy chain variable region are connected via a peptide linker GGGGS or (GGGGS)2 or (GGGGS)3.

When the antigen-binding moiety is fused to an Fc, it is typically fused via a hinge region. In one embodiment, the first antigen-binding moiety is fused to one Fc polypeptide of the Fc domain via a first hinge, and the second antigen-binding moiety is fused to one Fc polypeptide of the Fc domain via a second hinge. In some embodiments, the first hinge and the second hinge can form a covalent bond, such as a disulfide bond. The first hinge or/and the second hinge may comprise amino acids of the hinge region of human IgG, and the hinge region of human IgG comprises a native hinge region or a variant thereof. In some embodiments, the first hinge or/and the second hinge comprises/comprise amino acids of the hinge region of human IgG1; in some embodiments, the first hinge or/and the second hinge comprises/comprise amino acids of the hinge region of human IgG4. In some specific embodiments, the first hinge comprises GEPKSSDKTHTCPPCP (SEQ ID NO: 3), and the second hinge comprises EPKSCDKTHTCPPCP (SEQ ID NO: 4); in other specific embodiments, the first hinge comprises EPKSCDKTHTCPPCP, and the second hinge comprises GEPKSSDKTHTCPPCP. In some specific embodiments, both the first hinge and the second hinge comprise GEPKSSDKTHTCPPCP or EPKSCDKTHTCPPCP.

Fc domain

An Fc domain of a multispecific antibody consists of a pair of polypeptide chains comprising a heavy chain domain of an immunoglobulin molecule. For example, the Fc domain of immunoglobulin G (IgG) molecule is a dimer, and each of Fc polypeptides comprises CH2 and CH3 of the IgG heavy chain constant regions. The two Fc polypeptides of the Fc domain are capable of stable association with each other. In one embodiment, the multispecific antibody disclosed herein comprises one Fc domain.

In one embodiment, the Fc domain of the multispecific antibody is an IgG Fc domain. In a specific embodiment, the Fc domain is an IgG1 Fc domain. In another embodiment, the Fc domain is an IgG4 Fc domain. In a more specific embodiment, the Fc domain is an IgG4 Fc domain comprising an amino acid substitution at position S228, particularly amino acid substitution S228P, which reduces the *in vivo* Fab arm exchange for an IgG4 antibody. In yet another specific embodiment, the Fc domain is a human Fc domain. In a more specific embodiment, the Fc domain is a human IgG1 Fc domain.

In some embodiments, the Fc domain comprises a modification, such as an amino acid substitution. The modification may be, for example, a modification that promotes heterodimerization, a modification that alters effector function, a modification that alters the binding ability to protein A, or the like.

In some embodiments, the Fc comprises a modification that promotes heterodimerization.

The multispecific antibody disclosed herein comprises different antigen-binding moieties fused to one or the other of the two Fc polypeptides in the Fc domain, thus, the two Fc polypeptides are typically comprised in two different polypeptide chains. Several possible combinations of the two polypeptides are generated by recombinant co-expression and subsequent dimerization of these polypeptides. In order to increase the yield and purity of the multispecific antibody in recombinant production, it would be advantageous to introduce a modification that promotes the binding of the desired polypeptides into the Fc domain of the multispecific antibody. Thus, in a specific embodiment, the Fc domain comprises an amino acid substitution that promotes association of the two Fc polypeptides of the Fc domain.

The site for the most extensive protein-protein interaction between the two Fc polypeptides of the human IgG Fc domain is in the CH3 domain of the Fc domain. Thus, in one embodiment, the modification is in the CH3 domain of the Fc domain.

In a specific embodiment, the modification is a so-called "knob-into-hole" modification, which comprises a "knob" modification in one of the two Fc polypeptides of the Fc domain and a "hole" modification in the other one of the two Fc polypeptides of the Fc domain. Generally, the method involves introducing a protuberance ("knob") at the interface of one Fc polypeptide and a corresponding cavity ("hole") at the interface of the other Fc polypeptide, such that the protuberance can be positioned in the cavity to promote heterodimer formation and to interfere with homodimer formation. The protuberance is constructed by substituting a small amino acid side chain from the interface of one Fc polypeptide with a larger side chain (e.g., tyrosine or tryptophan, etc.). The complementary cavity having the same or similar size as the protuberance is created in the interface of the other Fc polypeptide by substituting a large amino acid side chain with a smaller amino acid side chain (e.g., alanine or threonine, etc.).

Thus, in a specific embodiment, an amino acid residue is substituted with an amino acid residue having a larger side chain volume in the CH3 domain of one Fc polypeptide of the multispecific antibody, thereby creating a protuberance within the CH3 domain of the Fc polypeptide that can be positioned in a cavity within the CH3 domain of the other Fc polypeptide, and an amino acid residue is substituted with an amino acid residue having a smaller side chain volume in the CH3 domain of the other Fc polypeptide, thereby creating a cavity within the CH3 domain of the Fc polypeptide.

In some specific embodiments, one Fc polypeptide of the Fc domain comprises T366Y/W or/and S354C, and the other Fc polypeptide comprises Y407T/V, Y349C, T366S, or/and L368A. In a more specific embodiment, one Fc polypeptide of the Fc domain comprises amino acid substitutions T366Y/W and S354C, and the other Fc polypeptide comprises amino acid substitutions Y407T/V, Y349C, T366S, and L368A. In a more specific embodiment, the Fc may be an Fc of human IgG1.

In some embodiments, the Fc domain comprises a modification that alters effector function. In some specific embodiments, the Fc domain of the multispecific antibody is modified to reduce the binding affinity of the Fc domain for an Fc receptor and/or effector function of the Fc domain as compared to an Fc not subjected to the modification. Reducing the binding affinity of the Fc domain for an Fc receptor and/or effector function of the Fc domain is beneficial to improve cytokine release and reduce side effects.

In some embodiments, the modification that reduces the binding affinity of the Fc domain for an Fc receptor and/or reduces effector function of the Fc domain is an amino acid substitution. In some embodiments, the Fc domain comprises an amino acid substitution at one or more positions selected from E233, L234, L235, N297, P331, and P329. In some embodiments, the Fc domain comprises an amino acid substitution at one or more positions selected from L234, L235, and P329. In some embodiments, the Fc domain comprises amino acid substitutions L234A and L235A. In one such embodiment, the Fc is IgG1 Fc, particularly human IgG1 Fc. In some embodiments, the Fc domain comprises an amino acid substitution at position P329. In a more specific embodiment, the amino acid substitution is P329A, P329R, or P329G. In some embodiments, the Fc domain comprises an amino acid substitution at position P329 and another amino acid substitution at a position selected from E233, L234, L235, N297, and P331. In a more specific embodiment, the another amino acid substitution is E233P, L234A, L235A, L235E, N297A, N297D, or P331S. In some embodiments, the Fc domain comprises an amino acid substitution at positions P329, L234, and L235. In a more specific embodiment, the Fc domain comprises amino acid substitutions L234A, L235A, and P329G ("P329G LALA"). In another more specific embodiment, the Fc domain comprises amino acid substitutions L234A, L235A, and P329A ("P329A LALA"). In one such embodiment, the Fc is IgG1 Fc, particularly human IgG1 Fc.

In some specific embodiments, the Fc domain is a human IgG1 Fc domain comprising amino acid substitutions L234A, L235A, and P329A/G/R.

The amino acid substitution that reduces the binding affinity of the Fc domain for an Fc receptor and/or effector function of the Fc domain described above occurs on two polypeptide chains of the Fc domain.

In some embodiments, the Fc domain comprises a modification that reduces or eliminates the binding of the CH3 region of one Fc polypeptide in the Fc domain to Protein A (from *Staphylococcus aureus)*. In some embodiments, the modification is an amino acid substitution. In some embodiments, the Fc domain comprises an amino acid substitution H435R or/and Y436F, which occurs only in one Fc polypeptide rather than in the other Fc polypeptide. In a specific embodiment, the Fc domain comprises an amino acid substitution H435R or/and Y436F, which occurs only in one Fc polypeptide. In one such specific embodiment, the Fc is IgG1 Fc, particularly human IgG1 Fc.

In the multispecific antibody disclosed herein, the Fc domain may comprise one, two, or three of a modification that promotes heterodimerization, a modification that alters effector function, and a modification that reduces or eliminates the binding of the CH3 region of one Fc polypeptide in the Fc to Protein A. In some embodiments, the Fc comprises a modification that promotes the association of two Fc polypeptides, a modification that reduces the binding affinity of the Fc domain for an Fc receptor and/or reduced effector function of the Fc domain, and a modification that reduces or eliminates the binding of the CH3 region of one Fc polypeptide in the Fc to Protein A. The specific embodiments of the different modification types described above can be combined. For example, in a specific embodiment, the Fc domain comprises the following groups of amino acid substitutions:

i. Y407T/V, Y349C, T366S, L368A, T366Y/W, and S354C, wherein amino acid substitutions T366Y/W and S354C are on the same Fc polypeptide, and are not on the same Fc polypeptide with other amino acid substitutions in (i.);
ii. L234A, L235A, and P329A; and
iii. H435R and Y436F, which occur only in one Fc polypeptide.

In a more specific embodiment, according to the EU numbering, one Fc polypeptide of the Fc domain comprises amino acid substitutions L234A, L235A, P329A, Y349C, T366S, L368A, and Y407V, and the other Fc polypeptide comprises amino acid substitutions L234A, L235A, P329A, S354C, T366W, H435R, and Y436F. In one such specific embodiment, the Fc is IgG1 Fc, particularly human IgG1 Fc.

In the context of the present disclosure, amino acid substitutions are represented as: original amino acid-position-substituted amino acid, using three-letter (Xaa) or single-letter (X) codes to represent amino acid residues. Thus, for example, "H435R" means that amino acid H at position 435 is substituted with amino acid R, and more than 1 substituted amino acid may be contained, for example, T366Y/W means that amino acid T at position 366 is substituted with amino acid Y or W.

In some embodiments, the multispecific antibody described herein is trivalent, i.e., the first antigen-binding moiety, the second antigen-binding moiety, and the third antigen-binding moiety each provide monovalent binding to the corresponding antigen.

The present disclosure provides an exemplary trivalent multispecific antibody.

In one example, the multispecific antibody consists of two polypeptide chains, the amino acid sequences of which are set forth in SEQ ID NO: 65 and SEQ ID NO: 67, respectively. In some embodiments, the nucleotide sequences encoding the two polypeptide chains are set forth in SEQ ID NO: 66 and SEQ ID NO: 68, respectively.

In one example, the multispecific antibody consists of two polypeptide chains, the amino acid sequences of which are set forth in SEQ ID NO: 69 and SEQ ID NO: 71, respectively. In some embodiments, the nucleotide sequences encoding the two polypeptide chains are set forth in SEQ ID NO: 70 and SEQ ID NO: 72, respectively.

In one example, the multispecific antibody consists of three polypeptide chains, the amino acid sequences of which are set forth in SEQ ID NO: 59, SEQ ID NO: 61, and SEQ ID NO: 63, respectively. In some embodiments, the nucleotide sequences encoding the two polypeptide chains are set forth in SEQ ID NO: 60, SEQ ID NO: 62, and SEQ ID NO: 64, respectively.

In one example, the multispecific antibody consists of three polypeptide chains, the amino acid sequences of which are set forth in SEQ ID NO: 73, SEQ ID NO: 61, and SEQ ID NO: 75, respectively. In some embodiments, the nucleotide sequences encoding the two polypeptide chains are set forth in SEQ ID NO: 74, SEQ ID NO: 62, and SEQ ID NO: 76, respectively.

*Composition*

The present disclosure provides a pharmaceutical composition comprising the multispecific antibody and further comprising one or more pharmaceutically acceptable carriers. The pharmaceutically acceptable carriers include, for example, excipients, diluents, encapsulating materials, fillers, buffers, or other agents.

*Isolated Nucleic Acid*

The present disclosure provides an isolated nucleic acid comprising a polynucleotide encoding the multispecific antibody described herein. The nucleic acid sequences of some multispecific antibodies are illustratively listed in the sequence listing.

*Vector*

The present disclosure provides a vector comprising the nucleic acid. In some embodiments, the vector is a cloning vector; in other embodiments, the vector is an expression vector; in a specific embodiment, the expression vector is pcDNA3.1(+). The expression vector is optionally any expression vector capable of expressing the multispecific antibody described herein.

*Host cell*

In some embodiments, the present disclosure provides a host cell comprising the vector described herein, the host cell being a suitable host cell for use in cloning or expressing a multispecific antibody. In some embodiments, the host cell is a prokaryotic cell. In other embodiments, the host cell is a eukaryotic cell. In some embodiments, the host cell is selected from a yeast cell, a mammalian cell, or other cells suitable for preparing a multispecific antibody. Mammalian cells are, for example, Chinese hamster ovary (CHO) cells or CHO-S cells.

*Method for preparins, multispecific antibody*

In some embodiments, the present disclosure provides a method for preparing a multispecific antibody, comprising culturing the host cell to express the multispecific antibody, and isolating and purifying the multispecific antibody in the system. To produce the multispecific antibody, a nucleic acid encoding the multispecific antibody is isolated and inserted into one or more vectors for further use in cloning or/and expression in a host cell. The nucleic acid can be obtained using various methods known in the art, such as gene splicing and chemical synthesis.

*Use*

The present disclosure provides use of a multispecific antibody. In some specific embodiments, the multispecific antibody used may be BC24, PC1, PC2, and/or PC3.
The present disclosure provides use of the multispecific antibody or the pharmaceutical composition in preparing a medicament for treating a disease related to BCMA expression.
The present disclosure provides a method for treating a disease related to BCMA expression in a subject, comprising administering to the subject a therapeutically effective amount of the multispecific antibody or the pharmaceutical composition.
In some embodiments, the disease is a tumor or an autoimmune disease.
In some embodiments, the disease is lymphoma, multiple myeloma, leukemia, systemic lupus erythematosus, or rheumatoid arthritis.
The present disclosure further provides exemplary embodiments:

Embodiment 1. A multispecific antibody, comprising

(i) a first antigen-binding moiety that binds to a first antigen;
(ii) a second antigen-binding moiety that binds to a second antigen; and
(iii) a third antigen-binding moiety that binds to the first antigen;

wherein the first antigen is BCMA and the second antigen is an activating T cell antigen, and the first antigen-binding moiety and the third antigen-binding moiety are both single variable domains and each independently comprise any one of:

(a) CDR1 comprising an amino acid sequence set forth in SEQ ID NO: 7, CDR2 comprising an amino acid sequence set forth in SEQ ID NO: 8, and CDR3 comprising an amino acid sequence set forth in SEQ ID NO: 9;

(b) CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12;

(c) CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15; or

(d) CDR1 comprising an amino acid sequence set forth in SEQ ID NO: 16, CDR2 comprising an amino acid sequence set forth in SEQ ID NO: 17, and CDR3 comprising an amino acid sequence set forth in SEQ ID NO: 18.

Embodiment 2. The multispecific antibody according to Embodiment 1, wherein the first antigen-binding moiety and the third antigen-binding moiety each independently comprise any one of:

(b) CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12; or

(c) CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15.

Embodiment 3. The multispecific antibody according to Embodiment 2, wherein the first antigen-binding moiety comprises the following complementarity determining regions: CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12; and the third antigen-binding moiety comprises the following complementarity determining regions: CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15.

Embodiment 4. The multispecific antibody according to Embodiment 2, wherein the third antigen-binding moiety comprises the following complementarity determining regions: CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12; and the first antigen-binding moiety comprises the following complementarity determining regions: CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15.

Embodiment 5. The multispecific antibody according to Embodiment 1, wherein the first antigen-binding moiety and the third antigen-binding moiety each independently comprise CDR1, CDR2, and CDR3 of a single variable domain having an amino acid sequence set forth in SEQ ID NO: 19, 21, 23, 25, 37, 38, 39, or 40; preferably, the first antigen-binding moiety and the third antigen-binding moiety each independently comprise CDR1, CDR2, and CDR3 of a single variable domain having an amino acid sequence set forth in SEQ ID NO: 37 or 39.

Embodiment 6. The multispecific antibody according to any one of Embodiments 1-5, wherein the first antigen-binding moiety and the third antigen-binding moiety each independently comprise an amino acid sequence having at least 85%, 90%, 91%, 92%, 93%, 94%, 95%, 96%, 97%, 98%, 99%, or 100% identity to a sequence set forth in SEQ ID NO: 19, 41, 42, or 25.

Embodiment 7. The multispecific antibody according to any one of Embodiments 1-6, wherein the first antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 41.

Embodiment 8. The multispecific antibody according to Embodiment 7, wherein the first antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 21, 37, or 38; preferably, the first antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 37.

Embodiment 9. The multispecific antibody according to any one of Embodiments 1-8, wherein the third antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 42.

Embodiment 10. The multispecific antibody according to Embodiment 9, wherein the third antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 23, 39, or 40; preferably, the third antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 39.

Embodiment 11. The multispecific antibody according to any one of Embodiments 1-6, wherein the first antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 42.

Embodiment 12. The multispecific antibody according to Embodiment 11, wherein the first antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 23, 39, or 40; preferably, the first antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 39.

Embodiment 13. The multispecific antibody according to any one of Embodiments 1-6 and 11-12, wherein the third antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 41.

Embodiment 14. The multispecific antibody according to Embodiment 13, wherein the third antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 21, 37, or 38; preferably, the third antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 37.

Embodiment 15. The multispecific antibody according to any one of Embodiments 1-5, wherein the first antigen-binding moiety and the third antigen-binding moiety are selected from any one of:

(1) a first antigen-binding moiety comprising an amino acid sequence set forth in SEQ ID NO: 21 and a third antigen-binding moiety comprising an amino acid sequence set forth in SEQ ID NO: 23, 39, or 40;
(2) a first antigen-binding moiety comprising an amino acid sequence set forth in SEQ ID NO: 37 and a third antigen-binding moiety comprising an amino acid sequence set forth in SEQ ID NO: 23, 39, or 40;
(3) a first antigen-binding moiety comprising an amino acid sequence set forth in SEQ ID NO: 38 and a third antigen-binding moiety comprising an amino acid sequence set forth in SEQ ID NO: 23, 39, or 40;
(4) a third antigen-binding moiety comprising an amino acid sequence set forth in SEQ ID NO: 21 and a first antigen-binding moiety comprising an amino acid sequence set forth in SEQ ID NO: 23, 39, or 40;
(5) a third antigen-binding moiety comprising an amino acid sequence set forth in SEQ ID NO: 37 and a first antigen-binding moiety comprising an amino acid sequence set forth in SEQ ID NO: 23, 39, or 40;
(6) a third antigen-binding moiety comprising an amino acid sequence set forth in SEQ ID NO: 38 and a first antigen-binding moiety comprising an amino acid sequence set forth in SEQ ID NO: 23, 39, or 40;
(7) a third antigen-binding moiety comprising an amino acid sequence set forth in SEQ ID NO: 41 and a first antigen-binding moiety comprising an amino acid sequence set forth in SEQ ID NO: 42;
(8) a third antigen-binding moiety comprising an amino acid sequence set forth in SEQ ID NO: 42 and a first antigen-binding moiety comprising an amino acid sequence set forth in SEQ ID NO: 41;
(9) a third antigen-binding moiety comprising an amino acid sequence set forth in SEQ ID NO: 37 and a first antigen-binding moiety comprising an amino acid sequence set forth in SEQ ID NO: 39; or
(10) a third antigen-binding moiety comprising an amino acid sequence set forth in SEQ ID NO: 39 and a first antigen-binding moiety comprising an amino acid sequence set forth in SEQ ID NO: 37.

Embodiment 16. The multispecific antibody according to any one of Embodiments 1-15, wherein the single variable domain is derived from animals of the family Camelidae or is humanized.

Embodiment 17. The multispecific antibody according to any one of Embodiments 1-16, wherein the first antigen-binding moiety and the third antigen-binding moiety are identical.

Embodiment 18. The multispecific antibody according to any one of Embodiments 1-16, wherein the first antigen-binding moiety and the third antigen-binding moiety are different.

Embodiment 19. The multispecific antibody according to any one of Embodiments 1-18, wherein the second antigen-binding moiety is an Fab, an ScFv, or an ScFab.

Embodiment 20. The multispecific antibody according to any one of Embodiments 1-19, wherein the activating T cell antigen is CD3.

Embodiment 21. The multispecific antibody according to any one of Embodiments 1-20, wherein the second antigen-binding moiety comprises a heavy chain variable region and a light chain variable region, wherein the heavy chain variable region comprises the following complementarity determining regions: HCDR1 comprising an amino acid sequence set forth in SEQ ID NO: 43, HCDR2 comprising an amino acid sequence set forth in SEQ ID NO: 44, and HCDR3 comprising an amino acid sequence set forth in SEQ ID NO: 45; the light chain variable region comprises LCDR1 comprising an amino acid sequence set forth in SEQ ID NO: 46, LCDR2 comprising an amino acid sequence set forth in SEQ ID NO: 47, and LCDR3 comprising an amino acid sequence set forth in SEQ ID NO: 48.

Embodiment 22. The multispecific antibody according to any one of Embodiments 1-20, wherein the second antigen-binding moiety comprises a heavy chain variable region and a light chain variable region, wherein the heavy chain variable region comprises HCDR1, HCDR2, and HCDR3 of a variable region set forth in SEQ ID NO: 49; and the light chain variable region comprises LCDR1, HCDR2, and LCDR3 of a variable region set forth in SEQ ID NO: 50.

Embodiment 23. The multispecific antibody according to any one of Embodiments 1-22, wherein the second antigen-binding moiety comprises a heavy chain variable region having an amino acid sequence having at least 85%, 90%, 91%, 92%, 93%, 94%, 95%, 96%, 97%, 98%, 99%, or 100% identity to a sequence set forth in SEQ ID NO: 49 and a light chain variable region having an amino acid sequence having at least 85%, 90%, 91%, 92%, 93%, 94%, 95%, 96%, 97%, 98%, 99%, or 100% identity to a sequence set forth in SEQ ID NO: 50; preferably, the heavy chain variable region of the second antigen-binding moiety comprises an amino acid sequence set forth in SEQ ID NO: 49 and the light chain variable region thereof comprises an amino acid sequence set forth in SEQ ID NO: 50.

Embodiment 24. The multispecific antibody according to any one of Embodiments 1-23, wherein the second antigen-binding moiety is murine, chimeric, or humanized.

Embodiment 25. The multispecific antibody according to any one of Embodiments 1-24, wherein the third antigen-binding moiety and the first antigen-binding moiety are fused to each other, optionally via a peptide linker.

Embodiment 26. The multispecific antibody according to Embodiment 25, wherein the third antigen-binding moiety, at its C-terminus, is fused to the N-terminus of the first antigen-binding moiety.

Embodiment 27. The multispecific antibody according to any one of Embodiments 1-24, wherein the third antigen-binding moiety and the second antigen-binding moiety are fused to each other, optionally via a peptide linker.

Embodiment 28. The multispecific antibody according to Embodiment 27, wherein the third antigen-binding moiety, at its C-terminus, is fused to the N-terminus of the second antigen-binding moiety.

Embodiment 29. The multispecific antibody according to any one of Embodiments 1-28, further comprising (iv) an Fc domain composed of two Fc polypeptides.

Embodiment 30. The multispecific antibody according to Embodiment 29, wherein the first antigen-binding moiety, at its C-terminus, is fused to the N-terminus of one Fc polypeptide of the Fc domain, the second antigen-binding moiety is an ScFv, and the second antigen-binding moiety, at its C-terminus, is fused to the N-terminus of the other Fc polypeptide of the Fc domain.

Embodiment 31. The multispecific antibody according to Embodiment 30, wherein the structure from the N-terminus to the C-terminus of the second binding moiety is VL-VH or VH-VL.

Embodiment 32. The multispecific antibody according to Embodiment 29, wherein the first antigen-binding moiety, at its C-terminus, is fused to the N-terminus of one Fc polypeptide of the Fc domain, the second antigen-binding moiety is an Fab, and the second antigen-binding moiety, at the C-terminus of its Fab heavy chain, is fused to the N-terminus of the other Fc polypeptide of the Fc domain.

Embodiment 33. The multispecific antibody according to Embodiment 32, wherein the third antigen-binding moiety is fused to the N-terminus of the Fab light chain or the Fab heavy chain of the second antigen-binding moiety.

Embodiment 34. The multispecific antibody according to Embodiment 29, wherein the second antigen-binding moiety is an ScFv, the first antigen-binding moiety, at its C-terminus, is fused to the N-terminus of one Fc polypeptide of the Fc domain, the second antigen-binding moiety, at its C-terminus, is fused to the N-terminus of the other Fc polypeptide of the Fc domain, and the third antigen-binding moiety, at its C-terminus, is fused to the N-terminus of the first antigen-binding moiety.

Embodiment 35. The multispecific antibody according to Embodiment 29, wherein the second antigen-binding moiety is an ScFv, the first antigen-binding moiety, at its C-terminus, is fused to the N-terminus of one Fc polypeptide of the Fc domain, the second antigen-binding moiety, at its C-terminus, is fused to the N-terminus of the other Fc polypeptide of the Fc domain, and the third antigen-binding moiety, at its C-terminus, is fused to the N-terminus of the second antigen-binding moiety.

Embodiment 36. The multispecific antibody according to Embodiment 29, wherein the second antigen-binding moiety is an Fab, the first antigen-binding moiety, at its C-terminus, is fused to the N-terminus of one Fc polypeptide of the Fc domain, the second antigen-binding moiety, at the C-terminus of its Fab heavy chain, is fused to the N-terminus of the other Fc polypeptide of the Fc domain, and the third antigen-binding moiety, at its C-terminus, is fused to the N-terminus of the first antigen-binding moiety.

Embodiment 37. The multispecific antibody according to Embodiment 29, wherein the second antigen-binding moiety is an Fab, the first antigen-binding moiety, at its C-terminus, is fused to the N-terminus of one Fc polypeptide of the Fc domain, the second antigen-binding moiety, at the C-terminus of its Fab heavy chain, is fused to the N-terminus of the other Fc polypeptide of the Fc domain, and the third antigen-binding moiety, at its C-terminus, is fused to the N-terminus of the Fab light chain of the second antigen-binding moiety or the N-terminus of the Fab heavy chain of the second antigen-binding moiety.

Embodiment 38. The multispecific antibody according to any one of Embodiments 29-37, wherein the Fc domain is an IgG Fc domain.

Embodiment 39. The multispecific antibody according to Embodiment 38, wherein the IgG Fc domain is a human IgG Fc domain, preferably an Fc domain of human IgG1 or human IgG4.

Embodiment 40. The multispecific antibody according to any one of Embodiments 29-39, wherein the Fc domain comprises an amino acid substitution that promotes association of the two Fc polypeptides of the Fc domain.

Embodiment 41. The multispecific antibody according to any one of Embodiments 29-40, wherein according to the EU numbering, one Fc polypeptide of the Fc domain comprises amino acid substitutions T366Y/W and S354C, and the other Fc polypeptide comprises amino acid substitutions Y407T/V, Y349C, T366S, and L368A.

Embodiment 42. The multispecific antibody according to any one of Embodiments 29-41, wherein the Fc domain comprises an amino acid substitution that reduces the binding affinity of the Fc domain for an Fc receptor and/or effector function of the Fc domain.

Embodiment 43. The multispecific antibody according to Embodiment 42, wherein according to the EU numbering, the amino acid substitution that reduces the binding affinity of the Fc domain for an Fc receptor and/or effector function of the Fc domain is at one or more positions selected from the group consisting of L234, L235, and P329; preferably, both the two Fc polypeptides of the Fc domain comprise amino acid substitutions L234A, L235A, and P329G, or comprise amino acid substitutions L234A, L235A, and P329A.

Embodiment 44. The multispecific antibody according to any one of Embodiments 29-41, wherein the Fc domain comprises an amino acid substitution that reduces or eliminates the binding of a CH3 region of one Fc polypeptide in the Fc domain to Protein A.

Embodiment 45. The multispecific antibody according to Embodiment 44, wherein according to the EU numbering, the Fc domain comprises an amino acid substitution H435R or/and Y436F, which occurs only in one Fc polypeptide.

Embodiment 46. The multispecific antibody according to any one of Embodiments 29-39, wherein according to the EU numbering, one Fc polypeptide of the Fc domain comprises amino acid substitutions L234A, L235A, P329A, Y349C, T366S, L368A, and Y407V, and the other Fc polypeptide comprises amino acid substitutions L234A, L235A, P329A, S354C, T366W, H435R, and Y436F.

Embodiment 47. The multispecific antibody according to any one of Embodiments 1-46, wherein the multispecific antibody is trivalent.

Embodiment 48. The multispecific antibody according to any one of Embodiments 1-46, wherein the multispecific antibody consists of:

(1) two polypeptide chains, wherein one polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 65, and the other polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 67;

(2) two polypeptide chains, wherein one polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 69, and the other polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 71;

(3) three polypeptide chains, wherein one polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 59, the another polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 61, and the yet another polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 63; or

(4) three polypeptide chains, wherein one polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 73, the another polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 61, and the yet another polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 75.

Embodiment 49. The multispecific antibody according to Embodiment 1, wherein the multispecific antibody consists of:

(1) two polypeptide chains, wherein one polypeptide chain comprises an amino acid sequence set forth in SEQ ID NO: 65, and the other polypeptide chain comprises an amino acid sequence set forth in SEQ ID NO: 67;

(2) two polypeptide chains, wherein one polypeptide chain comprises an amino acid sequence set forth in SEQ ID NO: 69, and the other polypeptide chain comprises an amino acid sequence set forth in SEQ ID NO: 71;

(3) three polypeptide chains, wherein one polypeptide chain comprises an amino acid sequence set forth in SEQ ID NO: 59, the another polypeptide chain comprises an amino acid sequence set forth in SEQ ID NO: 61, and the yet another polypeptide chain comprises an amino acid sequence set forth in SEQ ID NO: 63; or
(4) three polypeptide chains, wherein one polypeptide chain comprises an amino acid sequence set forth in SEQ ID NO: 73, the another polypeptide chain comprises an amino acid sequence set forth in SEQ ID NO: 61, and the yet another polypeptide chain comprises an amino acid sequence set forth in SEQ ID NO: 75.

Embodiment 50. An isolated nucleic acid comprising a polynucleotide encoding the multispecific antibody according to any one of Embodiments 1-49.

Embodiment 51. A vector comprising the nucleic acid according to Embodiment 50.

Embodiment 52. A host cell comprising the nucleic acid according to Embodiment 50 or the vector according to Embodiment 51.

Embodiment 53. A method for preparing the multispecific antibody according to any one of Embodiments 1-49, comprising culturing the host cell according to Embodiment 52 to express the multispecific antibody, and isolating and purifying the multispecific antibody in the system.

Embodiment 54. A pharmaceutical composition comprising the multispecific antibody according to any one of Embodiments 1-49 and a pharmaceutically acceptable carrier.

Embodiment 55. A method for treating a disease related to BCMA expression in a subject, comprising administering to the subject a therapeutically effective amount of the multispecific antibody according to any one of Embodiments 1-49 or the pharmaceutical composition according to Embodiment 54.

Embodiment 56. The method according to Embodiment 55, wherein the disease is lymphoma, multiple myeloma, leukemia, systemic lupus erythematosus, or rheumatoid arthritis.

Embodiment 57. A BCMA-binding antibody comprising a single variable domain, wherein the single variable domain comprises:

(1) CDR1 comprising an amino acid sequence set forth in SEQ ID NO: 7, CDR2 comprising an amino acid sequence set forth in SEQ ID NO: 8, and CDR3 comprising an amino acid sequence set forth in SEQ ID NO: 9;
(2) CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12;
(3) CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15; or
(4) CDR1 comprising an amino acid sequence set forth in SEQ ID NO: 16, CDR2 comprising an amino acid sequence set forth in SEQ ID NO: 17, and CDR3 comprising an amino acid sequence set forth in SEQ ID NO: 18.

Embodiment 58. The BCMA-binding antibody according to Embodiment 57, wherein the single variable domain comprises an amino acid sequence having at least 85%, 90%, 91%, 92%, 93%, 94%, 95%, 96%, 97%, 98%, 99%, or 100% identity to a sequence set forth in SEQ ID NO: 19, 21, 23, 25, 37, 38, 39, 40, 41, or 42.

Embodiment 59. The BCMA-binding antibody according to Embodiment 58, wherein the single variable domain comprises an amino acid sequence set forth in SEQ ID NO: 37, 38, 39, or 40.

Embodiment 60. The BCMA-binding antibody according to any one of Embodiments 57-59, wherein the single variable domain is derived from animals of the family Camelidae or is humanized.

Embodiment 61. The BCMA-binding antibody according to any one of Embodiments 57-60, wherein the antibody comprises an amino acid sequence having at least 85%, 90%, 91%, 92%, 93%, 94%, 95%, 96%, 97%, 98%, 99%, or 100% identity to a sequence set forth in SEQ ID NO: 27, 29, 31, 33, 51, 53, 55, or 57.

DETAILED DESCRIPTION

**Example 1. Construction of Anti-BCMA Single-Domain Antibody Phage Display Library**

A recombinant human BCMA-Fc fusion protein (ACRO, product catalog No. BC7-H5254) was mixed and emulsified with complete Freund's adjuvant in a ratio by volume of 1:1. A bactrian camel was first immunized by subcutaneous multipoint injection, and then immunized with the recombinant human BCMA-Fc fusion protein mixed and emulsified with incomplete Freund's adjuvant in a ratio by volume of 1:1 as a booster at intervals of 2 weeks. The serum was collected after the fourth or fifth immunization and assayed for anti-human BCMA antibody titer. After multiple rounds of immunization, the peripheral blood of the bactrian camel was collected, and peripheral blood mononuclear cells (PBMCs) were isolated. After being extracted, the RNA of PBMCs was reverse transcribed into cDNA, and the $V_HH$ coding fragment of the camel antibody was amplified.

The $V_HH$ coding fragment obtained from the amplification was digested with PstII NotI endonuclease and inserted into a phagemid vector pMECS (NTCC Plasmid Vector, Strain, Cell and Gene Collection, product catalog No. pMECS) to construct a recombinant vector, which was then electrically transferred into *Escherichia coli* TG1 (Lucigen, product catalog No. 60502-1) to construct a library stock. The $V_HH$ library stock was amplified to logarithmic growth phase. Library amplification was performed by adding M13KO7 helper phages (New England Biolabs, product catalog No. N0315S), with shaking overnight at 200 rpm at 28 °C. The bacterial liquid was centrifuged, and the supernatant was collected. 1/4 supernatant volume of a PEG6000/NaCl solution (20% PEG6000 (w/v), 2.5 M NaCl) was added. The mixture was incubated on ice for 1-2 h to settle the phages and centrifuged. The settled phages were collected and resuspended in PBS, and then 20% glycerol was added. The suspension was preserved at -80 °C as a single-domain antibody phage display library.

**Example 2. Anti-Human BCMA Single-Domain Antibody Screening**

The single-domain antibody phage display library was subjected to solid-phase panning. Single clones obtained from panning were cultured. Expression was induced by isopropyl-β-D-thiogalactoside (IPTG), and the supernatant was collected.

The picked clones were subjected to positive identification by indirect ELISA for human BCMA-His (ACRO, product catalog No. BCA-H522y). Positive clones that bind only to human BCMA-His with relatively high signal values were selected for preservation and sequenced. Positive clones 1A1, 1A10, 1A11, and 1B10 were obtained from the screening. The sequence analysis shows that 1A1 has a $V_HH$ nucleotide sequence of SEQ ID NO: 20 and an amino acid sequence of SEQ ID NO: 19; 1A10 has a $V_HH$ nucleotide sequence of SEQ ID NO: 22 and an amino acid sequence of SEQ ID NO: 21; 1A11 has a $V_HH$ nucleotide sequence of SEQ ID NO: 24 and an amino acid sequence of SEQ ID NO: 23; 1B10 has a $V_HH$ nucleotide sequence of SEQ ID NO: 26 and an amino acid sequence of SEQ ID NO: 25.

**Example 3. Preparation of Anti-Human BCMA $V_HH$-Fc Chimeric Antibodies**

3.1 Preparation of $V_HH$-Fc chimeric antibodies

The $V_HH$ sequences of the positive clones obtained from the screening were linked to a human Fc region to construct $V_HH$-Fc chimeric antibodies. Specifically, the $V_HH$ sequences or the sequence of the anti-human BCMA $V_HH$ control antibody (BM) (the amino acid sequence was the same as SEQ ID NO: 125 in CN109153731A) was inserted into apCDNA3.1 eukaryotic expression vector containing a human IgG1 constant region (the amino acid sequence was SEQ ID NO: 5). These $V_HH$-Fc chimeric antibodies (BM-Fc as a control) were expressed using an Expifectamine™ CHO Transfection Kit transient transfection expression system (Thermo Fisher Scientific Inc., product catalog No. A29129). After the antibodies were purified through a protein A affinity column, the binding of the antibodies to human TACI and BAFF-R proteins was detected by surface plasmon resonance (see 3.2 for the method). The detection shows that 1A1-Fc, 1A10-Fc, 1A11-Fc, and 1B10-Fc are all able to specifically bind to BCMA-His protein but do not bind to human TACI and BAFF-R proteins. The sequence analysis shows that the amino acid sequence of full-length of 1A1-Fc is set forth in SEQ ID NO: 27, and its nucleotide sequence is set forth in SEQ ID NO: 28; the amino acid sequence of full-length of 1A10-Fc is set forth in SEQ ID NO: 29, and its nucleotide sequence is set forth in SEQ ID NO: 30; the amino acid sequence of full-length of 1A11-Fc is set forth in SEQ ID NO: 31, and its nucleotide sequence is set forth in SEQ ID NO: 32; the amino acid sequence of full-length of 1B10-Fc is set forth in SEQ ID NO: 33, and its nucleotide sequence is set forth in SEQ ID NO: 34.

3.2 Detection of binding of antibodies to human TACI and BAFF-R proteins by surface plasmon resonance

The anti-human BCMA VHH-Fc chimeric antibodies were tested for specificity using a biomolecular interaction analysis system (GE, Biacore T200). An anti-hIgG (Fc) antibody (GE, product catalog No. BR-1008-39) was amino-coupled to a CM5 sensor chip. The anti-human BCMA $V_HH$-Fc chimeric antibodies were diluted to 2 μg/mL with a running buffer (137 mM NaCl, 2.7 mM KCl, 10 mM $Na_2HPO_4 \cdot 12H_2O$, 1.8 mM $KH_2PO_4$, 0.05% surfactant P-20 (w/v), pH 7.4), and capture was allowed for 90 s by passing the dilutions through experimental channels at a flow rate of 30 μL/min. Human TACI or BAFF-R protein was diluted to 100 nM with the running buffer, and binding was allowed by passing the dilution at a flow rate of 50 μL/min. The binding signal curve was observed. No binding curve was observed for 1A1-Fc, 1A10-Fc, 1A11-Fc, and 1B10-Fc, and they do not bind to TACI and BAFF-R proteins.

**Example 4. Affinity of Anti-Human BCMA V$_H$H-Fc Chimeric Antibodies for Human BCMA and Cynomolgus Monkey BCMA**

4.1 Determination of affinity of antibodies for human BCMA and cynomolgus monkey BCMA by surface plasmon resonance

The anti-human BCMA V$_H$H-Fc chimeric antibodies were tested for affinity using a biomolecular interaction analysis system (GE, Biacore T200). The detection shows that 1A1-Fc, 1A10-Fc, 1A11-Fc, and 1B10-Fc all have relatively high affinity for human BCMA protein, wherein there are cross-reactions of 1A10-Fc and 1A11-Fc with cynomolgus monkey BCMA protein, and 1A1-Fc, 1B10-Fc, and BM-Fc do not bind to cynomolgus monkey BCMA protein.

4.2 Detection of binding of antibodies to cells by flow cytometry

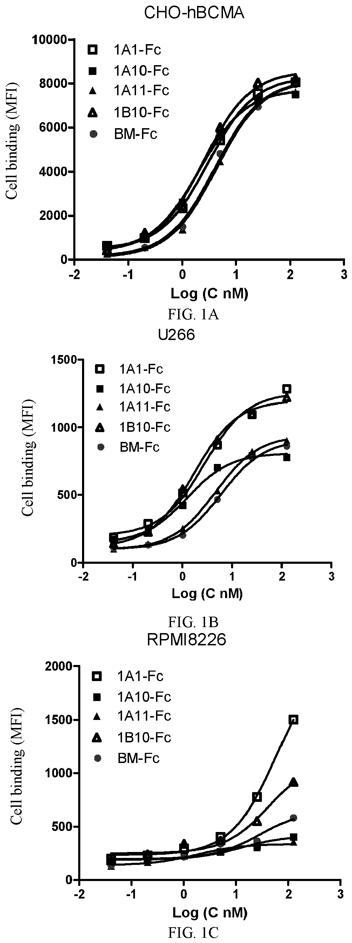

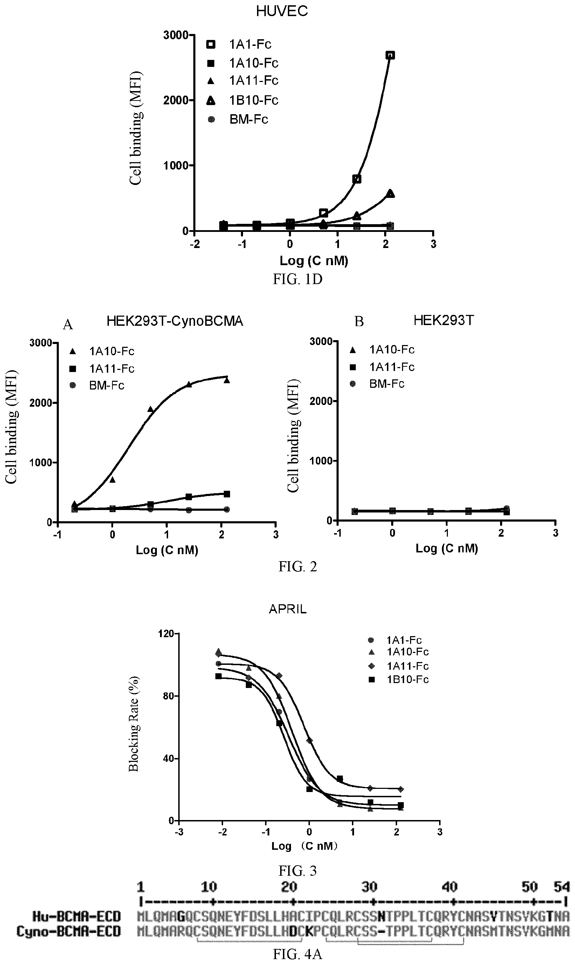

The binding of the anti-human BCMA V$_H$H-Fc chimeric antibodies to target cells with different BCMA expression levels was detected by flow cytometry. The CHO-hBCMA cells (iCarTab Biotechnology (Suzhou) Co. Ltd., product catalog No. AKD001A) were a stably transfected cell line highly expressing BCMA. The U266 cells (Cell Culture Center, Institute of Basic Medical Sciences, Chinese Academy of Medical Sciences, product catalog No. 3111C0001CCC000684) were a natural human myeloma cell line expressing BCMA at medium levels. The PRMI8226 cells (Cell Culture Center, Institute of Basic Medical Sciences, Chinese Academy of Medical Sciences, product catalog No. 3111C0001CCC000083) were a natural human myeloma cell line expressing BCMA at low levels. The HUVEC cells (ScienCell Research Laboratories, product catalog No. AKD001A 8000) were a human umbilical vein endothelial cell line not expressing BCMA. After $2 \times 10^5$ target cells were incubated with a serially diluted anti-human BCMA V$_H$H-Fc chimeric antibodies (serially diluted 5-fold from the initial concentration of 126 nM to 6 concentrations) on ice for 1 h, the cells were washed. A PE-labeled anti-human IgG Fc antibody (Jackson Immuno Research, product catalog No. 109-116-170) was added, and the mixture was incubated on ice for 0.5 h. The cells were washed and then tested using a flow cytometer (Thermo Fisher Scientific Inc., Attune NXT).
As shown in FIGs. 1A-1C and Table 1, 1A1-Fc, 1A10-Fc, 1A11-Fc, and 1B10-Fc could all effectively bind to CHO-hBCMA cells that express BCMA at relatively high levels and U266 cells that express BCMA at medium levels; they also bound significantly to RPMI8226 cells that express BCMA at relatively low levels; 1A10-Fc and 1A11-Fc bound to U266 cells better than BM-Fc did. As shown in FIG. 1D, 1A10-Fc and 1A11-Fc did not bind to HUVEC cells that did not express BCMA, but 1A1-Fc and 1B10-Fc did bind to HUVEC cells that did not express BCMA.

Table 1. Affinity of anti-human BCMA V$_H$H-Fc chimeric antibodies for target cells

| V$_H$H-Fc chimeric antibody | CHO-hBCMA cells EC50(nM) | U266 cells EC50(nM) |
|---|---|---|
| 1A1-Fc | 3.04 | 2.77 |
| 1A10-Fc | 4.28 | 4.64 |
| 1A11-Fc | 2.08 | 1.26 |
| 1B10-Fc | 2.51 | 1.72 |
| BM-Fc | 4.06 | 6.02 |

4.3 Detection of binding of antibodies to HEK293T cells transiently transfected with cynomolgus monkey BCMA by flow cytometry

The full-length (SEQ ID NO: 6) cynomolgus monkey BCMA gene was synthesized *in vitro* and inserted into the pCDNA3.1 eukaryotic expression vector. HEK293T cells were transfected with the expression vector using Lipofectamine™ 3000 (Thermo Fisher Scientific Inc., product catalog No. L3000015) according to the manufacturer's protocol. Briefly, the HEK293T cells were plated into a 6-well plate at $5 \times 10^5$/well. After about 24 h, the fetal bovine serum-containing medium was removed using a pipette, and the cells were washed with PBS. Then a transfection reagent was added to each well in a DNA-to-Lipo3000 ratio of 5 μg:7.5 μL. After about 6 h, the transfection reagent was replaced with 10% fetal bovine serum (v/v) medium. After being transfected for about 24 h, the cells were collected for flow cytometry detection.
The 1A10-Fc, 1A11-Fc and BM-Fc antibodies were serially diluted (serially diluted 5-fold from the initial concentration of 126 nM to 5 concentrations, and the sample dilutions were used as negative control groups) and each were used to incubate $2 \times 10^5$ HEK293T cells transiently transfected with cynomolgus monkey BCMA and ones not transfected. After being incubated on ice for 1 h, the cells were washed, and a PE-labeled anti-human IgG Fc antibody (Jackson Immuno Research, product catalog No. 109-116-170) was added. The mixtures were incubated on ice for 0.5 h. The cells were washed and then tested using a flow cytometer (Thermo Fisher Scientific Inc., Attune NXT). In FIG. 2A, HEK293T cells transiently transfected with cynomolgus monkey BCMA (HEK293T-CynoBCMA) were adopted. In FIG. 2B, HEK293T cells not transfected with cynomolgus monkey BCMA were adopted. The $EC_{50}$ values and the maximum binding MFI values (Bmax) of the binding of Fc chimeric antibodies to the HEK293T cells transiently transfected with cynomolgus monkey BCMA were shown in Table 2. The results show that 1A10-Fc and 1A11-Fc bound to cynomolgus monkey BCMA from the cells, and that the BM-Fc antibody did not bind to cynomolgus monkey BCMA.

Table 2. Binding of anti-human BCMA $V_H$H-Fc chimeric antibodies to HEK293T cells transiently transfected with cynomolgus monkey BCMA

| VHH-Fc chimeric antibody | EC50(nM) | Bmax(MFI) |
|---|---|---|
| 1A10-Fc | 2.02 | 2475.0 |
| 1A11-Fc | 10.36 | 501.8 |
| BM-Fc | / | 217.0 |
| Negative control | / | 157.0 |

## Example 5. Anti-Human BCMA $V_H$H-Fc Chimeric Antibodies Block Binding of APRIL to BCMA

After serially diluted anti-human BCMA $V_H$H-Fc chimeric antibodies 1A10-Fc, 1A11-Fc, and BM-Fc (serially diluted 5-fold from the initial concentration of 252 nM to 7 concentrations) were each mixed with 100 ng/mL biotin-conjugated recombinant BCMA-His protein (ACRO, product catalog No. BCA-H522y) in a ratio by volume of 1:1 and incubated at room temperature for 1 h, the mixtures were added to a microplate to which a recombinant APRIL protein (ACRO, product catalog No. APL-H5244) was fixed, and meanwhile a control group to which only 50 ng/mL biotin-conjugated recombinant BCMA-His protein was added was set. The plate was incubated at 37 °C for 1 h and then washed to remove unbound biotin-conjugated recombinant BCMA-His protein. HRP-conjugated streptavidin (eBioscience, product catalog No. 18-4100-51) was added, and the plate was washed 5 times and then subjected to color development. The light absorption signal values at the wavelength of 450 nm and the reference wavelength of 650 nm were measured. The blocking rate of each concentration of each antibody sample for the binding signal value of BCMA to APRIL was calculated according to the formula blocking rate = (signal value of control group - signal value of sample)/signal value of control group $\times$ 100%., and a curve was fit. The results are shown in FIG. 3 and Table 3. 1A1-Fc, 1B10-Fc, 1A10-Fc, and 1A11-Fc could all effectively block the binding of APRIL to BCMA.

As shown in FIG. 4A, the amino acid sequence of the human BCMA extracellular region (SEQ ID NO: 35) differs from that of the cynomolgus monkey BCMA extracellular region (SEQ ID NO: 36) by having Gly6, Ala20, Ile22, Asn31, Val45, and Thr52. There were cross-reactions of clones 1A10 and 1A11 with cynomolgus monkey BCMA protein, based on which the possible epitopes of the two clones are presumed to be located at Gln7-His19, and/or Pro23-Ser30, and/or Asn31-Ser44, and/or Thr46-Gly51. FIG. 4B shows the structure of the extracellular region of human BCMA in the PDB database (PDB No. 2kn1). As shown in FIGs. 4A and 4B, human BCMA contains 3 pairs of disulfide bonds in the extracellular region: Cys8-Cys21, Cys24-Cys37 and Cys28-Cys41; human BCMA and APRIL mostly bind to the β-hairpin structure (Bossen, C. et al. Semin. Immunol. 2006, 18(5):263-275), based on which the main binding sites of clones 1A10-Fc and 1A11-Fc are presumed to be located at Gln7-His19, and/or Pro23-Ser30, and/or Asn31-Ser44.

Table 3. Anti-human BCMA $V_H$H-Fc blocks binding of APRIL to BCMA

| $V_H$H-Fc chimeric antibody | IC50(nM) |
|---|---|
| 1A1-Fc | 0.34 |
| 1A10-Fc | 0.41 |
| 1A11-Fc | 0.76 |
| 1B10-Fc | 0.26 |

## Example 6. Epitope Difference Analysis Between Different Clones of Anti-Human BCMA $V_H$H

6.1 Investigation of competitive relationships between different clones of anti-human BCMA $V_H$H by ELISA (chessboard method)

The anti-human BCMA $V_H$H-Fc chimeric antibodies were diluted to 2 $\mu$g/mL and allowed to coat a high-adsorption microplate. The plate was washed and then blocked. 20 $\mu$g/mL anti-human BCMA $V_H$H-Fc antibodies were incubated with a biotin-conjugated BCMA-His protein (ACRO, product catalog No. BCA-H522y) at room temperature for 0.5 h to give mixtures of antigen and antibody. According to a chessboard arrangement, the incubated mixtures of antigen and antibody or the biotin-conjugated BCMA-His protein alone (control group) were added in sequence to the well plate at 100 $\mu$L/well. The plate was incubated at 37 °C for 1 h. The plate was washed to remove unbound biotin-conjugated recombinant BCMA-His protein. HRP-conjugated streptavidin (eBioscience, product catalog No. 18-4100-51) was added, and the plate was washed 5 times and then subjected to color development. The light absorption signal values at the wavelength of 450 nm and the reference wavelength of 650 nm were measured. The blocking rate of one antibody for the binding signal of another antibody to BCMA-Bio was calculated according to the formula blocking rate = (signal value of control group - signal value of sample group)/signal value of control group $\times$ 100%. The results are shown in Table 4. Positive control groups in which antibodies competed with themselves are shown on the diagonal line "\" of the chessboard (indicated in gray). The blocking rates were 99% or more. The blocking rate of 1A10-Fc for the binding of 1A11-Fc to human BCMA (32.6%) was less than 50%, and so was the blocking rate of 1A11-Fc for the binding of 1A10-Fc to human BCMA (21.6%), which indicates that there was no significant competitive relationship between 1A10-Fc and 1A11-Fc and that 1A10-Fc and 1A11-Fc could bind to different epitopes of BCMA protein simultaneously. Similarly, there were no significant competitive relationships between 1A10-Fc and 1A1-Fc, 1A10-Fc and 1B10-Fc, which indicates that 1A10-Fc and 1A1-Fc could bind to different epitopes of BCMA protein simultaneously, and that 1A10-Fc and 1B10 could bind to different epitopes of BCMA protein simultaneously. The mutual blocking rates of 1A1-Fc, 1A11-Fc and 1B10-Fc are all over 99%.

Table 4. ELISA (chessboard method) epitope competition

| Chessboard method | 1A1-Fc | 1A10-Fc | 1A11-Fc | 1B10-Fc |
|---|---|---|---|---|
| 1A1-Fc | 100.1% | 7.6% | 99.4% | 101.2% |
| 1A10-Fc | 36.9% | 100.4% | 32.6% | 46.4% |
| 1A11-Fc | 100.6% | 21.6% | 99.7% | 99.4% |
| 1B10-Fc | 100.3% | 26.7% | 99.5% | 100.6% |

6.2 Epitope difference analysis by surface plasmon resonance

Epitope competition relationship analysis was performed using a biomolecular interaction analysis system (GE, Biacore T200). An anti-His antibody (GE, product catalog No. 28995056) was amino-coupled to a CM5 sensor chip. A BCMA-His protein (ACRO, product catalog No. BCA-H522y) was diluted with a running buffer to about 1 $\mu$g/mL, and capture was allowed by passing the dilution through an experimental channel at a flow rate of 30 $\mu$L/min. The capture signal was controlled at 180-190 RU by adjusting the binding time. The anti-human BCMA $V_H$H-Fc chimeric antibody $V_H$H-Fc 1 was diluted to 10 $\mu$g/mL (saturation concentration; the binding signal value remained unchanged after the concentration was increased) with the running buffer and injected until signals reached a plateau. Upon completion of the injection, another anti-human BCMA $V_H$H-Fc chimeric antibody $V_H$H-Fc2 was injected. The antibody binding curves were observed and the binding signal values for both antibodies were recorded separately. Changes in the signal values were shown in Table 5. The signal value for the saturated binding of 1A10-Fc antibody to BCMA was 472.5 RU. At this time, 1A11-Fc was injected, and the saturated signal value was 579.0 RU, which is comparable with the saturated signal value of 653.1 RU when 1A11-Fc was injected alone. Vice versa, and the positive and negative injection orders resulted into comparable cumulative signal values, which indicates that 1A10-Fc and 1A11-Fc could bind to different epitopes of BCMA protein simultaneously.

Table 5. Changes in SPR signal values of $V_H$H-Fc antibodies and analysis

| | BCMA capture signal (RU) | 1 | VHH-FC2 2 | VHH-Fc1 binding signal (RU) | VHH-Fc2 binding signal (RU) | Cumulative signal value (RU) |
|---|---|---|---|---|---|---|
| RUN1 | 184.0 | 1A10-Fc | A11-Fc | 472.5 | 579.0 | 1051.5 |
| RLTN2 | 184.6 | 1A11-Fc | A10-Fc | 653.1 | 405.5 | 1058.6 |

6.3 Epitope difference analysis by flow cytometry

As the BCMA protein expressed on cell membranes may differ from free protein in effective exposure of epitopes, whether the two clones 1A10 and 1A11 can bind to the BCMA protein on cell membranes simultaneously was further determined by flow cytometry. The target cells were U266 cells. 20 $\mu$g/mL or 10 $\mu$g/mL 1A10-Fc and 1A11-Fc antibodies were each used to incubate $2 \times 10^5$ target cells; or 20 $\mu$g/mL 1A10-Fc antibody was mixed with 20 $\mu$g/mL 1A11-Fc antibody in a ratio by volume of 1:1, and the mixture was used to incubate $2 \times 10^5$ target cells. After being incubated on ice for 1 h, the cells were washed. A PE-labeled anti-human IgG Fc antibody (Jackson Immuno Research, product catalog No. 109-116-170) was added, and the mixture was incubated on ice for 0.5 h. The cells were washed and then tested using a flow cytometer (Thermo Fisher Scientific Inc., Attune NXT). The results are shown in Table 6. When 10 $\mu$g/mL identical antibody was added, the percent increase in the mean fluorescence value for the 1A10-Fc sample was 902.0/823.5 - 1 = 9.5%, and the percent increase in the mean fluorescence value for the 1A11-Fc sample was 702.0/697.5 - 1 = 0.6%, which indicates that the concentration at 10 $\mu$g/mL of both antibodies was close to saturation concentrations. On the above basis, 10 $\mu$g/mL different antibody was added: when 10 $\mu$g/mL 1A11-Fc was added to 10 $\mu$g/mL 1A10-Fc, the percent increase in the mean fluorescence value was 1443.5/823.5 - 1 = 75.3%; when 10 $\mu$g/mL 1A10-Fc was added to 10 $\mu$g/mL 1A11-Fc, the percent increase in the mean fluorescence value was 1443.5/697.5 - 1 = 107.0%, which indicates that the two antibodies 1A10-Fc and 1A11-Fc could bind to different epitopes of BCMA protein on cell membranes simultaneously.

Table 6. Binding of anti-human BCMA VHH chimeric antibodies to U266 cells

| No. | $V_HH$-Fc chimeric antibody | Mean fluorescence value (MFI) |
|---|---|---|
| a | 1A10-Fc, 10 $\mu$g/ml+ 1A11-Fc, 10 g/ml | 1443.5 |
| b | 1A10-Fc, 20 $\mu$g/ml | 902.0 |
| c | 1A11-Fc, 20 $\mu$g/ml | 702.0 |
| d | 1A10-Fc, 10 $\mu$g/ml | 823.5 |
| e | 1A11-Fc, 10 $\mu$g/ml | 697.5 |

## Example 7. Construction, Expression and Purification of Anti-Human BCMA $V_HH$-Fc Chimeric Monoclonal Antibodies and Humanized Monoclonal Antibodies Thereof

$V_HH$-Fc chimeric antibodies 1A10-Fc and 1A11-Fc were humanized separately, wherein two humanized antibodies, 1A10-V1 and 1A10-V2 respectively, were obtained after the chimeric antibody 1A10-Fc was humanized; two humanized antibodies, 1A11-V1 and 1A11-V2 respectively, were obtained after the chimeric antibody 1A11-Fc was humanized. Nucleotide and amino acid sequences of the antibodies described above are shown in Table 7.

DNA sequences encoding anti-human BCMA $V_HH$-Fc chimeric antibodies and humanized antibodies thereof (SEQ ID NOs: 30, 52, 54, 32, 56, 58, see Table 7 for corresponding names) were synthesized and cloned into pcDNA3.1(+) expression vectors, respectively. Each antibody was expressed using an ExpiCHO expression kit (Thermo Fisher, catalog No. A29133). The constructed expression vector containing the DNA sequence of the antibody was first transfected into ExpiCHO cells (CHO-S, Thermo), and the cells were cultured in ExpiCHO expression medium in a humidified atmosphere in an incubator at 37 °C with 8% $CO_2$ on an orbital shaker spinning at 130 rpm. The culture supernatant was collected, and protein purification was performed using protein A magnetic beads (Genscript, catalog No. L00273). The protein concentration was measured using a UV-Vis spectrophotometer (NanoDrop lite, Thermo Scientific).

Table 7. Sequence information of anti-human BCMA V$_H$H-Fc chimeric monoclonal antibody and humanized monoclonal antibody thereof

| Name | Amino acid sequence (SEQ ID NO:) | Encoding DNA sequence (SEQ ID NO:) | Note |
|---|---|---|---|
| 1A10-Fc | QVQLVESGGGSVQAGGSLRLSCAASGYSSNVAC MAWYRQAPGKEREWVATIVADFGTTNYAASVK GRFTISQDNAKNTVYLQMNSLKPEDSAMYYCA ATQRGGIDWCDEINYWGQGTLVTVSSEPKSCDK THTCPPCPAPELLGGPSVFLFPPKPKDTLMISRTP EVTCVVVDVSHEDPEVKFNWYVDGVEVHNAK TKPREEQYNSTYRVVSVLTVLHQDWLNGKEYK CKVSNKALPAPIEKTISKAKGQPREPQVYTLPPS RDELTKNQVSLTCLVKGFYPSDIAVEWESNGQPE NNYKTTPPVLDSDGSFFLYSKLTVDKSRWQQGN VFSCSVMHEALHNHYTQKSLSLSPGK SEQ ID NO: 29 | SEQ ID NO: 30 | Human camel chimeric antibody |
| 1A10-V 1 | QVQLVESGGGLVQPGGSLRLSCAASGYSSNVAC MAWYRQAPGKGLEWVATIVADFGTTNYAASVK GRFTISQDNSKNTVYLQMNSLRAEDTAVYYCAA TQRGGIDWCDEINYWGQGTLVTVSSGEPKSSDK THTCPPCPAPELLGGPSVFLFPPKPKDTLMISRTP EVTCVVVDVSHEDPEVKFNWYVDGVEVHNAK TKPREEQYNSTYRVVSVLTVLHQDWLNGKEYK CKVSNKALPAPIEKTISKAKGQPREPQVYTLPPS RDELTKNQVSLTCLVKGFYPSDIAVEWESNGQPE NNYKTTPPVLDSDGSFFLYSKLTVDKSRWQQGN VFSCSVMHEALHNHYTQKSLSLSPGK SEQ ID NO: 51 | SEQ ID NO: 52 | Humanized antibody |
| 1A10-V 2 | QVQLVESGGGLVQPGGSLRLSCAASGYSSNVAC MAWYRQAPGKEREWVSTIVADFGTTNYAASVK GRFTISQDNSKNTVYLQMNSLRAEDTAVYYCAA TQRGGIDWCDEINYWGQGTLVTVSSGEPKSSDK THTCPPCPAPELLGGPSVFLFPPKPKDTLMISRTP EVTCVVVDVSHEDPEVKFNWYVDGVEVHNAK TKPREEQYNSTYRVVSVLTVLHQDWLNGKEYK CKVSNKALPAPIEKTISKAKGQPREPQVYTLPPS RDELTKNQVSLTCLVKGFYPSDIAVEWESNGQPE NNYKTTPPVLDSDGSFFLYSKLTVDKSRWQQGN VFSCSVMHEALHNHYTQKSLSLSPGK SEQ ID NO: 53 | SEQ ID NO: 54 | Humanized antibody |
| 1A11-Fc | QVQLVESGGGSVQAGGSLRLSCAASGVTFNSAC MGWFRQAPGKEREGVARIETGYGGTVYADSVK GRFTISRDNAKNTVYLQMNSLKPEDTAMYYCA AKRSWCTPTWWHELDYNYWGQGTQVTVSSEP KSCDKTHTCPPCPAPELLGGPSVFLFPPKPKDTL MISRTPEVTCVVVDVSHEDPEVKFNWYVDGVE VHNAKTKPREEQYNSTYRVVSVLTVLHQDWLN GKEYKCKVSNKALPAPIEKTISKAKGQPREPQV YTLPPSRDELTKNQVSLTCLVKGFYPSDIAVEWE SNGQPENNYKTTPPVLDSDGSFFLYSKLTVDKSR WQQGNVFSCSVMHEALHNHYTQKSLSLSPGK SEQ ID NO: 31 | SEQ ID NO: 32 | Human camel chimeric antibody |

(continued)

| Name | Amino acid sequence (SEQ ID NO:) | Encoding DNA sequence (SEQ ID NO:) | Note |
|---|---|---|---|
| 1A11-V 1 | QVQLVESGGGLVQPGGSLRLSCAASGFTFNSAC MGWFRQAPGKGREGVSRIETGYGGTVYADSVK GRFTISRDNSKNTVYLQMNSLRAEDTAVYYCAA KRSWCTPTWWHELDYNYWGQGTQVTVSSGEP KSSDKTHTCPPCPAPELLGGPSVFLFPPKPKDTL MISRTPEVTCVVVDVSHEDPEVKFNWYVDGVE VHNAKTKPREEQYNSTYRVVSVLTVLHQDWLN GKEYKCKVSNKALPAPIEKTISKAKGQPREPQV YTLPPSRDELTKNQVSLTCLVKGFYPSDIAVEWE SNGQPENNYKTTPPVLDSDGSFFLYSKLTVDKSR WQQGNVFSCSVMHEALHNHYTQKSLSLSPGK SEQ ID NO: 55 | SEQ ID NO: 56 | Humanized antibody |
| 1A11-V 2 | QVQLVESGGGLVQPGGSLRLSCAASGFTFNSAC MGWFRQAPGKEREGVSRIETGYGGTVYADSVK GRFTISRDNSKNTVYLQMNSLKAEDTAVYYCAA KRSWCTPTWWHELDYNYWGQGTQVTVSSGEP KSSDKTHTCPPCPAPELLGGPSVFLFPPKPKDTL MISRTPEVTCVVVDVSHEDPEVKFNWYVDGVE VHNAKTKPREEQYNSTYRVVSVLTVLHQDWLN GKEYKCKVSNKALPAPIEKTISKAKGQPREPQV YTLPPSRDELTKNQVSLTCLVKGFYPSDIAVEWE SNGQPENNYKTTPPVLDSDGSFFLYSKLTVDKSR WQQGNVFSCSVMHEALHNHYTQKSLSLSPGK SEQ ID NO: 57 | SEQ ID NO: 58 | Humanized antibody |

**Example 8. Binding of Anti-Human BCMA V_HH-Fc chimeric antibodies and Humanized Antibodies Thereof to Antigen Human BCMA**

The affinity of the test antibody for human BCMA protein was determined by Biacore T200 (GE) as follows: A certain amount of the test antibody (including 1A10-Fc, 1A11-Fc, 1A10-V1, 1A10-V2, 1A11-V1, and 1A11-V2) was captured by a CM5 chip (Cytiva, product catalog No. 29149603) coupled to Anti-hIgG (Cytiva, product catalog No. 29234600), and then human BCMA was allowed to flow over the chip surface. The response signals were detected in real time using Biacore software, and association and dissociation curves were obtained. The buffer used in the experiment was Biacore universal buffer (137 mM NaCl, 2.7 mM KCl, 10 mM $Na_2HPO_4 \cdot 12H_2O$, 1.8 mM $KH_2PO_4$, 0.05% surfactant P-20 (w/v), pH 7.4). Anti-hIgG was coupled to a CM5 chip surface at a response value of up to about 9000 RU, and the test antibody (including 1A10-Fc, 1A11-Fc, 1A10-V1, 1A10-V2, 1A11-V1, and 1A11-V2) was captured at about 200 RU. Then the signal values of the interaction of different concentrations of human BCMA (100 nM, 50 nM, 25 nM, 12.5 nM, 6.25 nM, and 3.125 nM) with the test antibody (including 1A10-Fc, 1A11-Fc, 1A10-V1, 1A10-V2, 1A11-V1, and 1A11-V2) were measured. The flow rate in the flow cell was at 50 µL/min, the association was performed for 240 s, the dissociation was performed for 1400 s, the regeneration was performed using 3 M $MgCl_2$ (GE) for 60 s, and the baseline was stable. Results were obtained by calculation according to the affinity and kinetics 1:1 binding mode in biacore evaluation software. The affinity of anti-human BCMA V_HH-Fc chimeric antibodies and humanized antibodies thereof (including 1A10-Fc, 1A11-Fc, 1A10-V1, 1A10-V2, 1A11-V1, and 1A11-V2) for human BCMA is shown in Table 8.

Table 8. Binding affinity of anti-human BCMA V_HH-Fc chimeric antibodies and humanized antibodies for human BCMA

| Name | Note | Antigen BCMA (KD) |
|---|---|---|
| 1A10-Fc | Human camel chimeric antibody | 0.94 nM |
| 1A10-V1 | Humanized antibody | 0.337 nM |

(continued)

| Name | Note | Antigen BCMA (KD) |
|---|---|---|
| 1A10-V2 | Humanized antibody | 1.31 nM |
| 1A11-Fc | Human camel chimeric antibody | 3.96 nM |
| 1A11-V1 | Humanized antibody | 5.05 nM |
| 1A11-V2 | Humanized antibody | 4.37 nM |

**Example 9. Construction, Expression and Purification of Anti-BCMA/Anti-CD3 Multispecific Antibodies**

Anti-BCMA/anti-CD3 multispecific antibodies were produced as human IgG1 by knobs-into-holes (Ridgway, et al., 1996) Fc engineering. H435R and Y436F mutations (Jendeberg et al.,1997) were designed in the sequence of one polypeptide of the Fc domain to reduce the affinity of Fc for protein A, which was favorable for removing the homodimers formed during the assembly of multispecific antibodies in the protein A affinity purification.

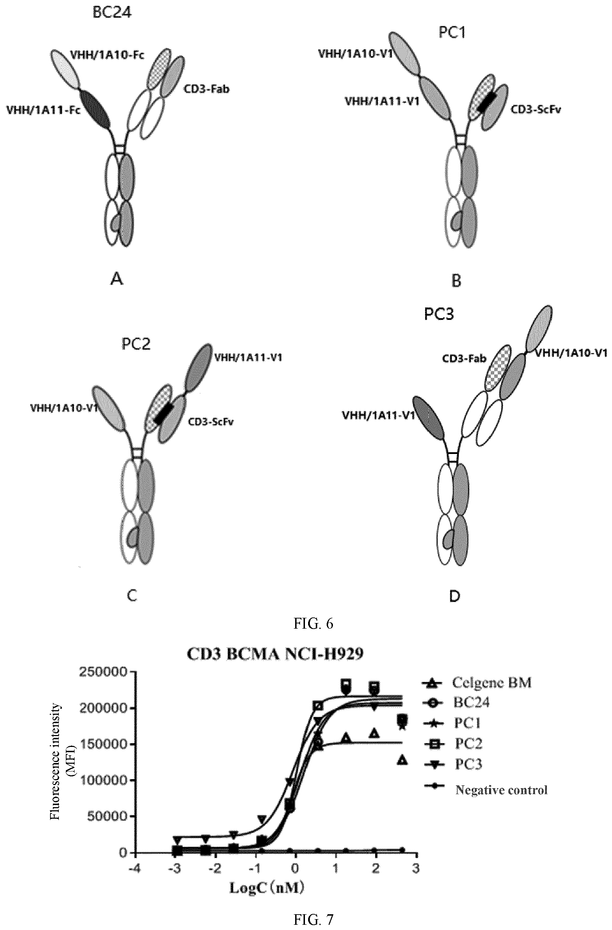

In this example, four anti-BCMA/anti-CD3 multispecific antibodies disclosed herein were constructed, named BC24, PC1, PC2, and PC3, respectively, wherein the configuration of BC24 is shown in FIG. 6A, the configuration of PC1 is shown in FIG. 6B, the configuration of PC2 is shown in FIG. 6C, and the configuration of PC3 is shown in FIG. 6D. In the constructed multispecific antibodies, the antigen-binding domain binding to BCMA was derived from an anti-human BCMA $V_H$H-Fc chimeric antibody and a humanized antibody thereof, and the antigen-binding domain binding to CD3 was obtained by humanizing an sp34 antibody (Silvana Pessano, et al., The T3/T cell receptor complex: antigenic distinction between the two 20-kd T3 (T3-delta and T3 - epsilon) subunits, EMBO J. 1985 Feb; 4(2): 337-344).

The anti-BCMA/anti-CD3 multispecific antibody BC24 has three polypeptide chains, wherein one polypeptide chain (named A10-linker-A11-Fc) comprises two antigen-binding domains binding to BCMA connected in tandem, the two antigen-binding domains binding to BCMA are derived from single variable domains of 1A10-Fc and 1A11-Fc, respectively, and the polypeptide chain A10-linker-A11 has an amino acid sequence set forth in SEQ ID NO: 59 and a nucleotide sequence set forth in SEQ ID NO: 60; the other two polypeptide chains of the anti-BCMA/anti-CD3 multispecific antibody BC24, named anti-CD3-HC-Fc (having an amino acid sequence set forth in SEQ ID NO: 61 and a nucleotide sequence set forth in SEQ ID NO: 62) and anti-CD3-LC (having an amino acid sequence set forth in SEQ ID NO: 63 and a nucleotide sequence set forth in SEQ ID NO: 64), respectively, form a structure comprising an antigen-binding domain binding to CD3 in the form of an Fab.

The anti-BCMA/anti-CD3 multispecific antibody PC1 has two polypeptide chains, wherein one polypeptide chain (named anti-BCMA-A10-v1-linker-A11-v1-Fc) comprises two antigen-binding domains binding to BCMA connected in tandem, the two antigen-binding domains binding to BCMA are derived from single variable domains of the humanized antibodies 1A10-V1 and 1A11-V1, respectively, the N-terminus of one polypeptide of Fc is fused to the antigen-binding domain binding to BCMA (derived from 1A11-V1), and the polypeptide chain anti-BCMA-A10-v1-linker-A11-v1-Fc has an amino acid sequence set forth in SEQ ID NO: 65 and a nucleotide sequence set forth in SEQ ID NO: 66; the other polypeptide chain of the anti-BCMA/anti-CD3 multispecific antibody PC1 (named anti-CD3-scFv-Fc) comprises an antigen-binding domain binding to CD3, the ScFv structure of which is formed by the tandem connection of a heavy chain variable region and a light chain variable region binding to CD3, the N-terminus of the other polypeptide of the Fc is fused to the antigen-binding domain binding to CD3, and the polypeptide chain anti-CD3-scFv-Fc has an amino acid sequence set forth in SEQ ID NO: 67 and a nucleotide sequence set forth in SEQ ID NO: 68.

The anti-BCMA/anti-CD3 multispecific antibody PC2 has two polypeptide chains, wherein one polypeptide chain (named anti-BCMA-A10-v1-Fc) comprises an antigen-binding domain binding to BCMA, the antigen-binding domain binding to BCMA is derived from a single variable domain of the humanized antibody 1A10-v1, the N-terminus of one polypeptide of Fc is fused to the antigen-binding domain binding to BCMA, and the polypeptide chain anti-BCMA-A10-v1-Fc has an amino acid sequence set forth in SEQ ID NO: 69 and a nucleotide sequence set forth in SEQ ID NO: 70; the other polypeptide chain of the anti-BCMA/anti-CD3 multispecific antibody PC2 (named anti-BCMA-A11-v1-linker-anti-CD3-scFv-Fc) comprises an antigen-binding domain binding to CD3 and an antigen-binding domain binding to BCMA connected in tandem, the ScFv structure of the antigen-binding domain binding to CD3 is formed by the tandem connection of a heavy chain variable region and a light chain variable region binding to CD3, the antigen-binding domain binding to BCMA is derived from a single variable domain of the humanized antibody 1A11-v1, the N-terminus of the other polypeptide of the Fc is fused to the antigen-binding domain binding to CD3, and the polypeptide chain anti-BCMA-A11-v1-linker-anti-CD3-scFv-Fc has an amino acid sequence set forth in SEQ ID NO: 71 and a nucleotide sequence set forth in SEQ ID NO: 72.

The anti-BCMA/anti-CD3 multispecific antibody PC3 has three polypeptide chains, wherein one polypeptide chain (named anti-BCMA-A11-v1-Fc) comprises an antigen-binding domain binding to BCMA, the antigen-binding domain binding to BCMA is derived from a single variable domain of the humanized antibody 1A11-v1, the N-terminus of one polypeptide of Fc is fused to the antigen-binding domain binding to BCMA, and the polypeptide chain anti-BCMA-A11-v1-Fc has an amino acid sequence set forth in SEQ ID NO: 73 and a nucleotide sequence set forth in SEQ ID NO: 74; the other two polypeptide chains of the anti-BCMA/anti-CD3 multispecific antibody PC3 form a structure comprising an antigen-binding domain binding to CD3 in the form of an Fab, the N-terminus of the Fab light chain is fused to the antigen-binding domain binding to BCMA, the antigen-binding domain binding to BCMA is derived from a single variable domain of the humanized antibody 1A10-v1, the N-terminus of another polypeptide of the Fc is fused to the Fab heavy chain of the antigen-binding domain binding to CD3, and the two polypeptide chains are named as anti-CD3-HC-Fc (having an amino acid sequence set forth in SEQ ID NO: 61 and a nucleotide sequence set forth in SEQ ID NO: 62) and anti-BCMA-A10-v1-linker-anti-CD3-VL (having an amino acid sequence set forth in SEQ ID NO: 75 and a nucleotide sequence set forth in SEQ ID NO: 76), respectively.

In this example, the anti-BCMA/anti-CD3 bispecific antibody from Celgene Corporation was used as a control antibody, and the sequence was derived from US20190263920A1. The anti-BCMA/anti-CD3 bispecific control antibody Benchmark (celgene BM for short) in this example comprises two antigen-binding domains binding to BCMA in the form of an Fab and an antigen-binding domain binding to CD3 in the form of an Fab, wherein one anti-BCMA arm is in anti-BCMA-Fab-Fc structural form, comprising the polypeptide chains anti-BCMA-HC-Fc (having an amino acid sequence set forth in SEQ ID NO: 77 and a nucleotide sequence set forth in SEQ ID NO: 78) and anti-BCMA-LC (having an amino acid sequence set forth in SEQ ID NO: 79 and a nucleotide sequence set forth in SEQ ID NO: 80), and H435R and Y436F point mutations are added in the anti-BCMA-HC-Fc (SEQ ID NO: 77), thereby improving the purification process yield; the structure of the other anti-BCMA/anti-CD3 arm of the anti-BCMA/anti-CD3 bispecific control antibody BM is in anti-BCMA-Fab-CD3-Fab-Fc structural form, comprising the polypeptide chains anti-BCMA-VH-anti-CD3-VL-Fc (having an amino acid sequence set forth in SEQ ID NO: 81 and a nucleotide sequence set forth in SEQ ID NO: 82), anti-CD3-VH-CL (having an amino acid sequence set forth in SEQ ID NO: 83 and a nucleotide sequence set forth in SEQ ID NO: 84), and anti-BCMA-LC (having an amino acid sequence set forth in SEQ ID NO: 79 and a nucleotide sequence set forth in SEQ ID NO: 80).

For each anti-BCMA/anti-CD3 antibody, DNA sequences of each polypeptide chain for encoding and constituting the antibody were inserted into pCDNA3.1(+) vectors, respectively, to obtain expression vectors expressing the corresponding polypeptide chains. Protein expression was driven by the CMV promoter. Polyadenylation was driven by the synthetic polyA signal sequence located at the 3' end of the CDS. In addition, each vector contained an Ori sequence for autonomous replication.

To generate these antibody molecules, each antibody combination was expressed using an ExpiCHO expression kit (Thermo Fisher, catalog No. A29133). Firstly, the corresponding expression vector was transfected into ExpiCHO cells (CHO-S, Thermo) in a specific ratio (the transfection ratio in PC1: vector (anti-BCMA-A10-v1-linker-A11-v1-Fc):vector (anti-CD3-scFv-Fc) was 1:1.5; the transfection ratio in PC2: vector (anti-BCMA-A10-v1-Fc):vector (anti-BCMA-A11-v1-linker-anti-CD3-scFv-Fc) was 1:1.5; the transfection ratio in PC3: vector (anti-BCMA-A11-v1-Fc):vector (anti-CD3-HC-Fc):vector (anti-BCMA-A10-v1-linker-anti-CD3-VL) was 1:1.5:1.5; the transfection ratio in celgene BM: vector (anti-BCMA-HC-Fc):vector (anti-BCMA-LC):vector (anti-BCMA-VH-anti-CD3-VL-Fc):vector (anti-CD3-VH-CL) was 1:2:1:1.5; the transfection ratio in BC24: vector (A10-linker-A11-Fc):vector (anti-CD3-HC-Fc):vector (anti-CD3-LC) was 1:1.5:2), and the cells were cultured in ExpiCHO expression medium in a humidified atmosphere in an incubator at 37 °C with 8% $CO_2$ on an orbital shaker spinning at 130 rpm. The cell culture supernatant was harvested after 10-14 days of culture, and the cells were removed by centrifugation. The supernatant was purified by affinity chromatography, ion exchange chromatography, and gel chromatography in AKTA purifier 100 (GE) system.

The protein concentration of the purified protein sample was determined by measuring the optical density (OD) at 280 nm using a molar extinction coefficient calculated based on the amino acid sequence.

Collected samples in all the fraction tubes were analyzed for purity by size exclusion chromatography (SEC). The specific method: the aggregate content of the molecules was analyzed using an ACQUITY UPLC Protein BEH SEC column analytical size exclusion chromatography column (WATERS) at 25 °C with 16 mM $NaH_2PO_4$, 34 mM $Na_2HPO_4$, 200 mM NaCl, and running buffer with a pH of 7.0. According to the SEC results, the samples with a purity greater than 95% were combined.

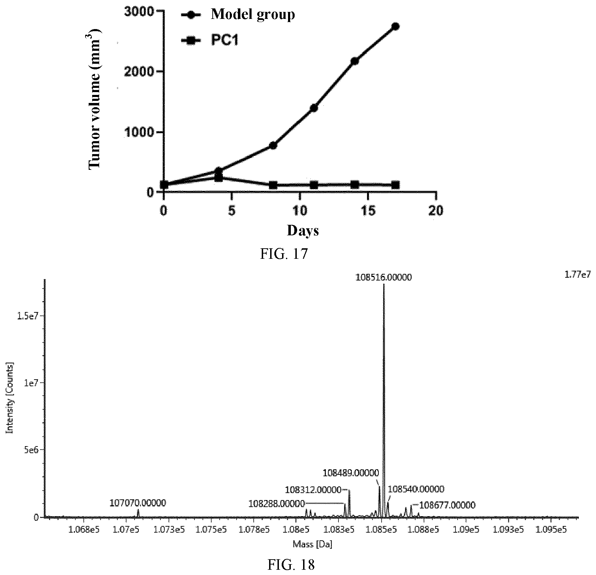

The complete molecular weight analysis was performed using a Waters LC-MS system (Waters, Singapore, USA, UK). A chromatography column MAbPac RP 4 $\mu$m 2.1 × 50 mm (Thermo, USA) was adopted. Phase A (0.1% aqueous formic acid solution) and phase B (0.1% formic acid in acetonitrile) were used as mobile phases, and the detection wavelength was 280 nm. 1 $\mu$g of protein was injected into the liquid chromatography-mass spectrometry system with a gradient of 5% B to 100% B within 5.5 min. The mass spectrometer adopted a positive ion mode, and the scanning range was 200-4000 m/z. Data were collected using MassLynx 4.1 and processed by UNIFI 1.8.2.169. The LC-MS analysis shows that the measured molecular weight of each anti-BCMA/anti-CD3 antibody sample is consistent with the theoretical molecular weight; in FIG. 18, the complete molecular weight detection result of the PC1 is shown, the molecular weight of the main peak of the sample is 108516 Da, which is consistent with the theoretical molecular weight of the PC1, and a homologous impurity peak is not seen.

Table 9. Sequence information of anti-BCMA/anti-CD3 multispecific antibody

| Name | Composition | Amino acid sequence (SEQ ID NO:) | Nucleotide (SEQ ID NO:) |
|---|---|---|---|
| BC24 | A10-linker-A11 -Fc | QVQLVESGGGSVQAGGSLRLSCAASGYSSN VACMAWYRQAPGKEREWVATIVADFGTTNY AASVKGRFTISQDNAKNTVYLQMNSLKPED SAMYYCAATQRGGIDWCDEINYWGQGTLV TVSSGGGGSGGGGSQVQLVESGGGSVQAGG SLRLSCAASGVTFNSACMGWFRQAPGKERE GVARIETGYGGTVYADSVKGRFTISRDNAKN TVYLQMNSLKPEDTAMYYCAAKRSWCTPT WWHELDYNYWGQGTQVTVSSGEPKSSDKT HTCPPCPAPEAAGGPSVFLFPPKPKDTLMISR TPEVTCVVVDVSHEDPEVKFNWYVDGVEV HNAKTKPREEQYNSTYRVVSVLTVLHQDWL NGKEYKCKVSNKALAAPIEKTISKAKGQPRE PQVCTLPPSREEMTKNQVSLSCAVKGFYPSD IAVEWESNGQPENNYKTTPPVLDSDGSFFLV SKLTVDKSRWQQGNVFSCSVMHEALHNHY TQKSLSLSPGK<br>SEQ ID NO: 59 | SEQ ID NO: 60 |
| | anti-CD3-HC-F c | EVQLVESGGGLVQPGGSLRLSCAASGFTFNT YAMNWVRQAPGKGLEWVARIRSKYNNYAT YYADSVKDRFTISRDDSKNTAYLQMNNLKT EDTAMYYCVRHGNFGNSYVSWFAYWGQGT LVTVSSASTKGPSVFPLAPSSKSTSGGTAALG CLVKDYFPEPVTVSWNSGALTSGVHTFPAVL QSSGLYSLSSVVTVPSSSLGTQTYICNVNHKP SNTKVDKKVEPKSCDKTHTCPPCPAPEAAG GPSVFLFPPKPKDTLMISRTPEVTCVVVDVS HEDPEVKFNWYVDGVEVHNAKTKPREEQY NSTYRVVSVLTVLHQDWLNGKEYKCKVSN KALAAPIEKTISKAKGQPREPQVYTLPPCREE MTKNQVSLWCLVKGFYPSDIAVEWESNGQP ENNYKTTPPVLDSDGSFFLYSKLTVDKSRWQ QGNVFSCSVMHEALHNRFTQKSLSLSPGK<br>SEQ ID NO: 61 | SEQ ID NO: 62 |
| | anti-CD3-LC | ELVVTQEPSLTTSPGGTVTLTCRSSTGAVTTS NYANWVQQKPGQAPRGLIGGTNKRAPGTPA RFSGSLLGGKAALTITGVQPEDEAEYYCALW YSNLWVFGGGTKLTVLGQPKANPTVTLFPPS SEELQANKATLVCLISDFYPGAVTVAWKADG SPVKAGVETTKPSKQSNNKYAASSYLSLTPE QWKSHRSYSCQVTHEGSTVEKTVAPTECS<br>SEQ ID NO: 63 | SEQ ID NO: 64 |

(continued)

| Name | Composition | Amino acid sequence (SEQ ID NO:) | Nucleotide (SEQ ID NO:) |
|---|---|---|---|
| PC1 | anti-BCMA-A1 0-v1-linker-A11 -v1-Fc | QVQLVESGGGLVQPGGSLRLSCAASGYSSNV ACMAWYRQAPGKGLEWVATIVADFGTTNYA ASVKGRFTISQDNSKNTVYLQMNSLRAEDTA VYYCAATQRGGIDWCDEINYWGQGTLVTVS SGGGGSGGGGSQVQLVESGGGLVQPGGSLR LSCAASGFTFNSACMGWFRQAPGKGREGVS RIETGYGGTVYADSVKGRFTISRDNSKNTVY LQMNSLRAEDTAVYYCAAKRSWCTPTWWH ELDYNYWGQGTQVTVSSGEPKSSDKTHTCP PCPAPEAAGGPSVFLFPPKPKDTLMISRTPEV TCVVVDVSHEDPEVKFNWYVDGVEVHNAK TKPREEQYNSTYRVVSVLTVLHQDWLNGKE YKCKVSNKALAAPIEKTISKAKGQPREPQVC TLPPSREEMTKNQVSLSCAVKGFYPSDIAVE WESNGQPENNYKTTPPVLDSDGSFFLVSKLT VDKSRWQQGNVFSCSVMHEALHNHYTQKS LSLSPGK SEQ ID NO: 65 | SEQ ID NO: 66 |
| | anti-CD3-scFv-Fc | ELVVTQEPSLTTSPGGTVTLTCRSSTGAVTTS NYANWVQQKPGQAPRGLIGGTNKRAPGTPA RFSGSLLGGKAALTITGVQPEDEAEYYCALW YSNLWVFGGGTKLTVLGGGGSGGGGSGGG GSEVQLVESGGGLVQPGGSLRLSCAASGFTF NTYAMNWVRQAPGKGLEWVARIRSKYNNY ATYYADSVKDRFTISRDDSKNTAYLQMNNL KTEDTAMYYCVRHGNFGNSYVSWFAYWGQ GTLVTVSSGEPKSSDKTHTCPPCPAPEAAGG PSVFLFPPKPKDTLMISRTPEVTCVVVDVSHE DPEVKFNWYVDGVEVHNAKTKPREEQYNS TYRVVSVLTVLHQDWLNGKEYKCKVSNKA LAAPIEKTISKAKGQPREPQVYTLPPCREEMT KNQVSLWCLVKGFYPSDIAVEWESNGQPEN NYKTTPPVLDSDGSFFLYSKLTVDKSRWQQG NVFSCSVMHEALHNRFTQKSLSLSPGK SEQ ID NO: 67 | SEQ ID NO: 68 |
| PC2 | anti-BCMA-A1 0-v1-Fc | QVQLVESGGGLVQPGGSLRLSCAASGYSSNV ACMAWYRQAPGKGLEWVATIVADFGTTNYA ASVKGRFTISQDNSKNTVYLQMNSLRAEDTA VYYCAATQRGGIDWCDEINYWGQGTLVTVS SGEPKSSDKTHTCPPCPAPEAAGGPSVFLFPP KPKDTLMISRTPEVTCVVVDVSHEDPEVKFN WYVDGVEVHNAKTKPREEQYNSTYRVVSV LTVLHQDWLNGKEYKCKVSNKALAAPIEKT ISKAKGQPREPQVCTLPPSREEMTKNQVSLS CAVKGFYPSDIAVEWESNGQPENNYKTTPPV LDSDGSFFLVSKLTVDKSRWQQGNVFSCSV | SEQ ID NO: 70 |

(continued)

| Name | Composition | Amino acid sequence (SEQ ID NO:) | Nucleotide (SEQ ID NO:) |
|------|-------------|----------------------------------|-------------------------|
|  |  | MHEALHNHYTQKSLSLSPGK<br>SEQ ID NO: 69 |  |
|  | anti-BCMA-A1 1-v1-linker-anti-CD3-scFv-Fc | QVQLVESGGGLVQPGGSLRLSCAASGFTFNS ACMGWFRQAPGKGREGVSRIETGYGGTVY ADSVKGRFTISRDNSKNTVYLQMNSLRAED TAVYYCAAKRSWCTPTWWHELDYNYWGQ GTQVTVSSGGGGSELVVTQEPSLTTSPGGTV TLTCRSSTGAVTTSNYANWVQQKPGQAPRG LIGGTNKRAPGTPARFSGSLLGGKAALTITGV QPEDEAEYYCALWYSNLWVFGGGTKLTVLG GGGSGGGGSGGGGSEVQLVESGGGLVQPGG SLRLSCAASGFTFNTYAMNWVRQAPGKGLE WVARIRSKYNNYATYYADSVKDRFTISRDDS KNTAYLQMNNLKTEDTAMYYCVRHGNFGN SYVSWFAYWGQGTLVTVSSGEPKSSDKTHT CPPCPAPEAAGGPSVFLFPPKPKDTLMISRTP EVTCVVVDVSHEDPEVKFNWYVDGVEVHN AKTKPREEQYNSTYRVVSVLTVLHQDWLNG KEYKCKVSNKALAAPIEKTISKAKGQPREPQ VYTLPPCREEMTKNQVSLWCLVKGFYPSDIA VEWESNGQPENNYKTTPPVLDSDGSFFLYSK LTVDKSRWQQGNVFSCSVMHEALHNRFTQK SLSLSPGK<br>SEQ ID NO: 71 | SEQ ID NO: 72 |

(continued)

| Name | Composition | Amino acid sequence (SEQ ID NO:) | Nucleotide (SEQ ID NO:) |
|------|-------------|----------------------------------|-------------------------|
| PC3 | anti-BCMA-A1 1-v1-Fc | QVQLVESGGGLVQPGGSLRLSCAASGFTFNS ACMGWFRQAPGKGREGVSRIETGYGGTVY ADSVKGRFTISRDNSKNTVYLQMNSLRAED TAVYYCAAKRSWCTPTWWHELDYNYWGQ GTQVTVSSGEPKSSDKTHTCPPCPAPEAAGG PSVFLFPPKPKDTLMISRTPEVTCVVVDVSHE DPEVKFNWYVDGVEVHNAKTKPREEQYNS TYRVVSVLTVLHQDWLNGKEYKCKVSNKA LAAPIEKTISKAKGQPREPQVCTLPPSREEMT KNQVSLSCAVGFYPSDIAVEWESNGQPENN YKTTPPVLDSDGSFFLVSKLTVDKSRWQQGN VFSCSVMHEALHNHYTQKSLSLSPGK SEQ ID NO: 73 | SEQ ID NO: 74 |
| | anti-CD3-HC-F c | EVQLVESGGGLVQPGGSLRLSCAASGFTFNT YAMNWVRQAPGKGLEWVARIRSKYNNYAT YYADSVKDRFTISRDDSKNTAYLQMNNLKT EDTAMYYCVRHGNFGNSYVSWFAYWGQGT LVTVSSASTKGPSVFPLAPSSKSTSGGTAALG CLVKDYFPEPVTVSWNSGALTSGVHTFPAVL QSSGLYSLSSVVTVPSSSLGTQTYICNVNHKP SNTKVDKKVEPKSCDKTHTCPPCPAPEAAG GPSVFLFPPKPKDTLMISRTPEVTCVVVDVS HEDPEVKFNWYVDGVEVHNAKTKPREEQY NSTYRVVSVLTVLHQDWLNGKEYKCKVSN KALAAPIEKTISKAKGQPREPQVYTLPPCREE MTKNQVSLWCLVKGFYPSDIAVEWESNGQP ENNYKTTPPVLDSDGSFFLYSKLTVDKSRWQ QGNVFSCSVMHEALHNRFTQKSLSLSPGK SEQ ID NO: 61 | SEQ ID NO: 62 |
| | anti-BCMA-A1 0-v1-linker-anti-CD3-VL | QVQLVESGGGLVQPGGSLRLSCAASGYSSNV ACMAWYRQAPGKGLEWVATIVADFGTTNYA ASVKGRFTISQDNSKNTVYLQMNSLRAEDTA VYYCAATQRGGIDWCDEINYWGQGTLVTVS SGGGGSELVVTQEPSLTTSPGGTVTLTCRSST GAVTTSNYANWVQQKPGQAPRGLIGGTNKR APGTPARFSGSLLGGKAALTITGVQPEDEAE YYCALWYSNLWVFGGGTKLTVLGQPKANP TVTLFPPSSEELQANKATLVCLISDFYPGAVT VAWKADGSPVKAGVETTKPSKQSNNKYAAS SYLSLTPEQWKSHRSYSCQVTHEGSTVEKTV APTECS SEQ ID NO: 75 | SEQ ID NO: 76 |

(continued)

| Name | Composition | Amino acid sequence (SEQ ID NO:) | Nucleotide (SEQ ID NO:) |
|---|---|---|---|
| Celgen eBM | anti-BCMA-HC -Fc | EVQLLESGGGLVQPGGSLRLSCAASGFTFSS YAMSWVRQAPGKGLEWVSAISGSGGSTYYA DSVKGRFTISRDNSKNTLYLQMNSLRAEDTA VYYCAKVLGWFDYWGQGTLVTVSSASTKG PSVFPLAPSSKSTSGGTAALGCLVEDYFPEPV TVSWNSGALTSGVHTFPAVLQSSGLYSLSSV VTVPSSSLGTQTYICNVNHKPSNTKVDEKVE PKSCDKTHTCPPCPAPEAAGGPSVFLFPPKPK DTLMISRTPEVTCVVVDVSHEDPEVKFNWY VDGVEVHNAKTKPREEQYNSTYRVVSVLTV LHQDWLNGKEYKCKVSNKALGAPIEKTISK AKGQPREPQVCTLPPSRDELTKNQVSLSCAV KGFYPSDIAVEWESNGQPENNYKTTPPVLDS DGSFFLVSKLTVDKSRWQQGNVFSCSVMHE ALHNRFTQKSLSLSPGK<br>SEQ ID NO: 77 | SEQ ID NO: 78 |
| | anti-BCMA-LC | EIVLTQSPGTLSLSPGERATLSCRASQSVSSS YLAWYQQKPGQAPRLLIYGASSRATGIPDRF SGSGSGTDFTLTISRLEPEDFAVYYCQQYGY PPDFTFGQGTKVEIKRTVAAPSVFIFPPSDRK LKSGTASVVCLLNNFYPREAKVQWKVDNA LQSGNSQESVTEQDSKDSTYSLSSTLTLSKA DYEKHKVYACEVTHQGLSSPVTKSFNRGEC<br>SEQ ID NO: 79 | SEQ ID NO: 80 |
| | anti-BCMA-VH -anti-CD3-VL-F c | EVQLLESGGGLVQPGGSLRLSCAASGFTFSS YAMSWVRQAPGKGLEWVSAISGSGGSTYYA DSVKGRFTISRDNSKNTLYLQMNSLRAEDTA VYYCAKVLGWFDYWGQGTLVTVSSASTKG PSVFPLAPSSKSTSGGTAALGCLVEDYFPEPV TVSWNSGALTSGVHTFPAVLQSSGLYSLSSV VTVPSSSLGTQTYICNVNHKPSNTKVDEKVE PKSCDGGGGSGGGGSQAVVTQEPSLTVSPGG TVTLTCGSSTGAVTTSNYANWVQEKPGQAF RGLIGGTNKRAPGTPARFSGSLLGGKAALTL SGAQPEDEAEYYCALWYSNLWVFGGGTKLT VLSSASTKGPSVFPLAPSSKSTSGGTAALGCL VKDYFPEPVTVSWNSGALTSGVHTFPAVLQS SGLYSLSSVVTVPSSSLGTQTYICNVNHKPSN TKVDKKVEPKSCDKTHTCPPCPAPEAAGGPS VFLFPPKPKDTLMISRTPEVTCVVVDVSHED PEVKFNWYVDGVEVHNAKTKPREEQYNST YRVVSVLTVLHQDWLNGKEYKCKVSNKAL GAPIEKTISKAKGQPREPQVYTLPPCRDELTK NQVSLWCLVKGFYPSDIAVEWESNGQPENN YKTTPPVLDSDGSFFLYSKLTVDKSRWQQGN VFSCSVMHEALHNHYTQKSLSLSPGK<br>SEQ ID NO: 81 | SEQ ID NO: 82 |

(continued)

| Name | Composition | Amino acid sequence (SEQ ID NO:) | Nucleotide (SEQ ID NO:) |
|---|---|---|---|
| | anti-CD3-VH-C L | EVQLLESGGGLVQPGGSLRLSCAASGFTFST YAMNWVRQAPGKGLEWVSRIRSKYNNYAT YYADSVKGRFTISRDDSKNTLYLQMNSLRAE DTAVYYCVRHGNFGNSYVSWFAYWGQGTL VTVSSASVAAPSVFIFPPSDEQLKSGTASVVC LLNNFYPREAKVQWKVDNALQSGNSQESVT EQDSKDSTYSLSSTLTLSKADYEKHKVYACE VTHQGLSSPVTKSFNRGEC SEQ ID NO: 83 | SEQ ID NO: 84 |

**Example 10. SPR of Binding of Anti-BCMA/Anti-CD3 Multispecific Antibody and Antigen**

The affinity of the test antibody for human CD3e or human BCMA protein was determined by Biacore T200 (GE) as follows:

A certain amount of the anti-BCMA/anti-CD3 antibody (including BC24, PC1, PC2, PC3, and Celgene BM) was captured by a CM5 chip (Cytiva, product catalog No. 29149603) coupled to Anti-hIgG (Cytiva, product catalog No. 29234600), and then human CD3e (Aero, Cat. No. CDE-H5223) or human BCMA was allowed to flow over the chip surface. The response signals were detected in real time using Biacore software, and association and dissociation curves were obtained. The buffer used in the experiment was Biacore universal buffer (137 mM NaCl, 2.7 mM KCl, 10 mM $Na_2HPO_4 \cdot 12H_2O$, 1.8 mM $KH_2PO_4$, 0.05% surfactant P-20 (w/v), pH 7.4). Anti-hIgG was coupled to a CM5 chip surface at a response value of up to about 9000 RU, and the anti-BCMA/anti-CD3 antibody (including BC24, PC1, PC2, PC3, and Celgene BM) was captured at about 200 RU. Then the signal values of the interaction of different concentrations of human CD3e protein (100 nM, 50 nM, 25 nM, 12.5 nM, 6.25 nM, and 3.125 nM) or human BCMA (100 nM, 50 nM, 25 nM, 12.5 nM, 6.25 nM, and 3.125 nM) with the anti-BCMA/anti-CD3 antibody (including BC24, PC1, PC2, PC3, and Celgene BM) were measured. The flow rate in the flow cell was at 50 μL/min, the association was performed for 240 s, the dissociation was performed for 1400 s, the regeneration was performed using 3 M $MgCl_2$ (GE) for 60 s, and the baseline was stable. Results were obtained by calculation according to the affinity and kinetics 1:1 binding mode in biacore evaluation software. The affinity of the anti-BCMA/anti-CD3 antibody (including BC24, PC1, PC2, PC3, and Celgene BM) for human CD3e protein or human BCMA is shown in Table 10.

Table 10. Affinity of anti-BCMA/anti-CD3 antibody for human CD3e or human BCMA protein

| Name | Antigen hBCMA (KD) | Antigen hCD3e (KD) |
|---|---|---|
| BC24 | 0.0627 nM | 0.286 nM |
| PC1 | 0.0396 nM | 1.43 nM |
| PC2 | 0.0185 nM | 1.33 nM |
| PC3 | 0.0347 nM | 1.17 nM |
| Celgene BM | 0.821 nM | 1.60 nM |

**Example 11. Binding of Anti-BCMA/Anti-CD3 Multispecific Antibodies to BCMA on Surface of Multiple Myeloma Cells**

The binding of the anti-BCMA/anti-CD3 antibody (including BC24, PC1, PC2, PC3, and Celgene BM) to BCMA in BCMA-expressing NCI-H929 cells (cell strain expressing BCMA at high levels, from Nanjing Cobioer Biosciences), MM1S cells (cell strain expressing BCMA at medium levels, from Beina Bio), and RPMI-8226 cells (cell strain expressing BCMA at low levels, from Cell Resource Center, Institute of Basic Medical Sciences, Chinese Academy of Medical Sciences) was analyzed by flow cytometry, and the negative sample was trastuzumab.

The specific procedures are as follows:

NCI-H929 cells (BCMA+++), MM1S cells (BCMA++), and RPMI-8226 cells (BCMA+) in the logarithmic growth phase were taken, adjusted to a cell density of $5 \times 10^6$-$10 \times 10^6$ cells/mL using RPMI medium (Hyclon, catalog No. SH30809.01) containing 2% FBS (fetal bovine serum), respectively, and seeded in a 96-well U-bottom cell culture plate (Costar, catalog No. 3799) at 50 μL per well. The test antibodies at different concentrations were prepared using the medium described above. For cells expressing BCMA at high levels and medium levels, the highest concentration of the antibodies was 440 nM, and the antibodies were diluted 5-fold to obtain a total of 9 concentration gradients. For cells expressing BCMA at low levels, the highest concentration of the antibodies was 2200 nM, and the antibodies were diluted 5-fold to obtain a total of 10 concentration gradients. The antibodies at different concentrations were added to the 96-well cell culture plate described above at 50 μL per well, mixed well and incubated at 4 °C for 1 h. The cells were washed with pre-cooled running buffer (MACS, catalog No. 130-091-221), and the supernatant was discarded. Pre-cooled fluorescent labeled goat anti-human IgG antibodies (Jackson, catalog No. 109-116-170) were added, and the cells were resuspended at 100 μL per well, mixed well and incubated at 4 °C for 30 min. After washing, 100 μL of pre-cooled running buffer was added to resuspend the cells. The cells were mixed well and collected while flowing on an Attune TM NxT flow cytometer, and the data was analyzed using Graphpad Prism5 software. FIGs. 7-9 and Table 11 show the binding ability of the anti-BCMA/anti-CD3 multispecific antibodies to NCI-H929 cells (BCMA+++), MM1S cells (BCMA++), and RPMI-8226 cells (BCMA+). The results show that in target cells expressing BCMA at high levels and medium levels, the maximum binding amount of PC1, PC2, PC3, and BC24 for the target antigen is better than that of Celgene BM. In target cells expressing BCMA at low levels, the binding of PC3 and BC24 to the target antigen is better than that of other samples.

Table 11. Binding $EC_{50}$ value and maximum binding amount of each test sample in different cells to target cells

| Sample name | NCI-H929 | | MM1S | |
|---|---|---|---|---|
| | $EC_{50}$(nM) | Top(MFI) | $EC_{50}$(nM) | Top(MFI) |
| Celgene BM | 0.8207 | 165553 | 0.3808 | 71438 |
| BC24 | 1.556 | 185797 | 0.535 | 106924 |
| PC1 | 1.261 | 226017 | 0.3503 | 100056.5 |
| PC2 | 1.035 | 233759 | 0.311 | 114684 |
| PC3 | 0.8366 | 222679.5 | 0.3378 | 123519 |
| Negative control | 67.32 | 4046 | / | 7405 |

**Example 12. Binding of Anti-BCMA/Anti-CD3 Multispecific Antibodies to T Cell Surface CD3**

The binding of the anti-BCMA/anti-CD3 antibody (including BC24, PC1, PC2, PC3, and Celgene BM) to T cell surface CD3 was analyzed by flow cytometry. The negative control was trastuzumab.

The specific procedures are as follows:

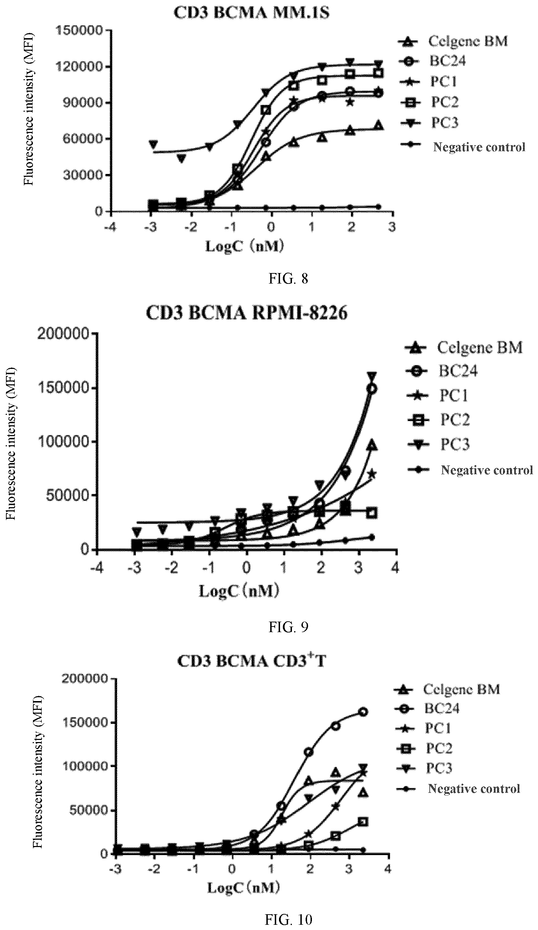

Human PBMC cells were sorted for CD3+ T cells using CD3 magnetic beads (Miltenyi, catalog No. 130-097-043): the cryopreserved PBMCs were thawed, and washed by centrifugation twice for counting. Running buffer (MACS, catalog No. 130-091-221) was added in a cell-to-buffer ratio of $10^7$:80 μL, CD3 beads were added in a cell-to-bead ratio of $10^7$:20 μL, and the mixture was mixed well and incubated at 4 °C for 15 min. The cells were washed with buffer in a cell-to-buffer ratio of $10^7$:1-2 mL. After centrifugation, the supernatant was discarded, and 500 μL of buffer was added to resuspend the cell pellet. The LS sorting column (Miltenyi, catalog No. 130-042-401) was placed on the MidiMACS Starting Kit (LS) magnetic rack (Miltenyi, catalog No. 130-091-051). After rinsing, the cell suspension was added and washed thrice. The LS sorting column was removed from the magnetic rack and placed on a clean centrifuge tube and 5 mL buffer was added to collect the sorted CD3+ T cells. After counting, the CD3+ T cells were adjusted to a cell density of $5 \times 10^6$-$10 \times 10^6$ cells/mL using RPMI medium (Hyclone, catalog No. SH30809.01) containing 2% FBS (fetal bovine serum) and seeded in a 96-well U-bottom cell culture plate (Costar, catalog No. 3799) at 50 μL per well. The test antibodies at different concentrations were prepared using the medium described above. The highest concentration of the antibodies was 2200 nM, and the antibodies were diluted 5-fold to obtain a total of 10 concentration gradients. The antibodies at different concentrations were added to the 96-well cell culture plate described above at 50 μL per well, mixed well and incubated at 4 °C for 1 h. The cells were washed with pre-cooled running buffer (MACS, catalog No. 130-091-221), and the supernatant was discarded. Pre-cooled fluorescent labeled goat anti-human IgG antibodies (Jackson, catalog No. 109-116-170) were added, and the cells were resuspended at 100 μL per well, mixed well and incubated at 4 °C for 30 min. After washing, 100 μL of pre-cooled running buffer was added to resuspend the cells. The cells were mixed well and collected while flowing on an Attune TM NxT flow cytometer, and the data was analyzed using Graphpad PrismS software. FIG. 10 and Table 12 show the binding ability of the anti-BCMA/anti-CD3 multispecific antibodies to CD3+ T cells. The results indicate that each anti-BCMA/anti-CD3 multispecific antibody can bind to CD3+ T cells.

Table 12. Binding $EC_{50}$ and maximum binding amount of test antibodies to CD3+ T cells

| Sample name | $EC_{50}$(nM) | Top(MFI) |
| --- | --- | --- |
| Celgene BM | 18.12 | 93499 |
| BC24 | 38.34 | 162535 |
| PC1 | 485.4 | 92854 |
| PC2 | ~461.6 | 37242 |
| PC3 | 85.81 | 98353 |
| Negative control | NA | 5980 |

## Example 13. Killing of Multiple Myeloma Target Cell NCI-H929 by Anti-BCMA/Anti-CD3 Multispecific Antibodies

The killing effects induced by the antibodies (including BC24, PC1, PC2, PC3, and Celgene BM) on target cells (NCI-H929 BCMA+++, from Nanjing Cobioer Biosciences) were studied using T cells provided by human PBMCs (peripheral blood mononuclear cells). The negative control was trastuzumab.

The specific procedures are as follows:

The target cells NCI-H929 cells were adjusted to a cell density of $5 \times 10^5$ cells/mL using 1640 medium for experiment containing 2% FBS (fetal bovine serum) and seeded in a 96-well cell culture plate (eppendorf, catalog No. 0030730199) at 50 μL per well. The test antibodies at different concentrations were prepared using the medium for experiment. The highest concentration of the antibodies was 8 nM, and the antibodies were diluted 5-fold to obtain a total of 10 concentration gradients. The antibodies at different concentrations were added to the 96-well cell culture plate described above at 50 μL per well. The effector cells human PBMCs were adjusted to a cell density of $2.5 \times 10^6$ cells/mL using medium for experiment and added at 100 μL per well. An administration group (50 μL of target cell + 100 μL of effector cell + 50 μL of antibody), a target cell group (50 μL of target cell + 150 μL of culture medium), an effector cell group (100 μL of human PBMC + 100 μL of culture medium), a target cell + effector cell group (50 μL of target cell + 100 μL of effector cell + 50 μL of culture medium), a blank control group (200 μL of culture medium) and a lysis solution control group (200 μL of culture medium + 20 μL of lysis solution), and a target cell maximum release group (50 μL of target cell + 150 μL of culture medium + 20 μL of lysis solution) were set, with the effector-to-target cell ratio being 10:1 and the co-incubation time being 24 h. 45 min prior to the assay, 20 μL/well of lysis solution (Promega, catalog No. G182A) was added to the target cell maximum release group and the lysis solution control group. The cell lysis rates were measured using a CytoTox96® nonradioactive cytotoxicity assay kit (Promega, G1780).

$$\text{Lysis rate } (\%) = (\text{OD}_{\text{administration group}} - \text{OD}_{\text{target cell + effector cell group}})/(\text{OD}_{\text{target cell maximum release group}} - \text{OD}_{\text{target cell group}}) \times 100\%$$

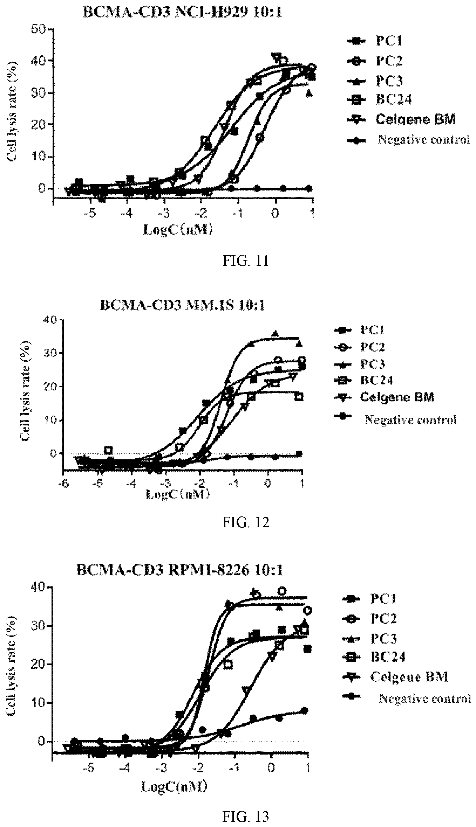

FIG. 11 and Table 13 show the lysis rate of NCI-H929/BCMA+++ tumor cells by effector cells induced by the anti-BCMA/anti-CD3 multispecific antibodies. The cell killing effect induced by the anti-BCMA/anti-CD3 antibody PC1 is better than that of PC2 and PC3, wherein the $EC_{50}$ of PC1 is about 0.067 nM, and the $EC_{50}$ of PC2 and PC3 is about 0.53 nM and 0.19 nM, respectively. The killing effect of BC24 and Celgene BM on NCI-H929 is better than that of PC1, PC2, and PC3, the $EC_{50}$ of BC24 is about 0.022 nM, and the $EC_{50}$ of Celgene BM is about 0.044 nM.

Table 13. Lysis $EC_{50}$ value and maximum lysis rate of each test sample to NCI-H929 cells

| Sample name | $EC_{50}$(nM) | Maximum lysis rate |
| --- | --- | --- |
| PC1 | 0.06736 | 36% |
| PC2 | 0.5268 | 38% |
| PC3 | 0.1871 | 35% |
| BC24 | 0.02177 | 40% |

(continued)

| Sample name | $EC_{50}$(nM) | Maximum lysis rate |
|---|---|---|
| Celgene BM | 0.04435 | 41% |

### Example 14. Killing of Multiple Myeloma Target Cell MM1S by Anti-BCMA/Anti-CD3 Multispecific Antibodies

The killing effects induced by the antibodies (including BC24, PC1, PC2, PC3, and Celgene BM) on target cells (MM1S BCMA++, from Beina Bio) were studied using T cells provided by human PBMCs (peripheral blood mononuclear cells). The negative control was trastuzumab.

The specific procedures are as follows:

The target cells MM1S cells were adjusted to a cell density of $5 \times 10^5$ cells/mL using 1640 medium for experiment containing 2% FBS (fetal bovine serum) and seeded in a 96-well cell culture plate (eppendorf, catalog No. 0030730199) at 50 $\mu$L per well. The test antibodies at different concentrations were prepared using the medium for experiment. The highest concentration of the antibodies was 8 nM, and the antibodies were diluted 5-fold to obtain a total of 10 concentration gradients. The antibodies at different concentrations were added to the 96-well cell culture plate described above at 50 $\mu$L per well. The effector cells human PBMCs were adjusted to a cell density of $2.5 \times 10^6$ cells/mL using medium for experiment and added at 100 $\mu$L per well. An administration group (50 $\mu$L of target cell + 100 $\mu$L of effector cell + 50 $\mu$L of antibody), a target cell group (50 $\mu$L of target cell + 150 $\mu$L of culture medium), an effector cell group (100 $\mu$L of human PBMC + 100 $\mu$L of culture medium), a target cell + effector cell group (50 $\mu$L of target cell + 100 $\mu$L of effector cell + 50 $\mu$L of culture medium), a blank control group (200 $\mu$L of culture medium) and a lysis solution control group (200 $\mu$L of culture medium + 20 $\mu$L of lysis solution), and a target cell maximum release group (50 $\mu$L of target cell + 150 $\mu$L of culture medium + 20 $\mu$L of lysis solution) were set, with the effector-to-target cell ratio being 10:1 and the co-incubation time being 24 h. 45 min prior to the assay, 20 $\mu$L/well of lysis solution (Promega, catalog No. G182A) was added to the target cell maximum release group and the lysis solution control group. The cell lysis rates were measured using a CytoTox96® nonradioactive cytotoxicity assay kit (Promega, G1780).

$$\text{Lysis rate (\%)} = (\text{OD}_{\text{administration group}} - \text{OD}_{\text{target cell + effector cell group}})/(\text{OD}_{\text{target cell maximum release group}} - \text{OD}_{\text{target cell group}}) \times 100\%$$

FIG. 12 and Table 14 show the lysis rate of MM1SBCMA++ tumor cells by effector cells induced by the anti-BCMA/anti-CD3 multispecific antibodies. The cell killing induced by the anti-BCMA/anti-CD3 multispecific antibody PC1 is better than that of PC2 and PC3, wherein the $EC_{50}$ of PC1 is about 0.0084 nM, and the $EC_{50}$ of PC2 and PC3 is about 0.061 nM and 0.041 nM, respectively. PC3 has better maximum lysis rate, and Celgene BM has the weakest killing effect on MM1S, wherein the $EC_{50}$ is about 0.085 nM, and the maximum lysis rate is 23%.

Table 14. Lysis $EC_{50}$ value and maximum lysis rate of each test sample to MM1S cells

| Sample name | $EC_{50}$(nM) | Maximum lysis rate |
|---|---|---|
| PC1 | 0.008398 | 26% |
| PC2 | 0.06052 | 28% |
| PC3 | 0.04069 | 36% |
| BC24 | 0.009373 | 21% |
| Celgene BM | 0.08465 | 23% |

### Example 15. Killing of Multiple Myeloma Target Cell RPMI-8226 by Anti-BCMA/Anti-CD3 Multispecific Antibodies

The killing effects induced by the antibodies (including BC24, PC1, PC2, PC3, and Celgene BM) on target cells (RPMI-8226 BCMA+, from Cell Resource Center, Institute of Basic Medical Sciences, Chinese Academy of Medical Sciences) were studied using T cells provided by human PBMCs (peripheral blood mononuclear cells). The negative control was trastuzumab.

The specific procedures are as follows:

The target cells RPMI-8226 cells were adjusted to a cell density of $5 \times 10^5$ cells/mL using 1640 medium for experiment containing 2% FBS (fetal bovine serum) and seeded in a 96-well cell culture plate (eppendorf, catalog No. 0030730199) at 50 μL per well. The test antibodies at different concentrations were prepared using the medium for experiment. The highest concentration of the antibodies was 8 nM, and the antibodies were diluted 5-fold to obtain a total of 10 concentration gradients. The antibodies at different concentrations were added to the 96-well cell culture plate described above at 50 μL per well. The effector cells human PBMCs were adjusted to a cell density of $2.5 \times 10^6$ cells/mL using medium for experiment and added at 100 μL per well. An administration group (50 μL of target cell + 100 μL of effector cell + 50 μL of antibody), a target cell group (50 μL of target cell + 150 μL of culture medium), an effector cell group (100 μL of human PBMC + 100 μL of culture medium), a target cell + effector cell group (50 μL of target cell + 100 μL of effector cell + 50 μL of culture medium), a blank control group (200 μL of culture medium) and a lysis solution control group (200 μL of culture medium + 20 μL of lysis solution), and a target cell maximum release group (50 μL of target cell + 150 μL of culture medium + 20 μL of lysis solution) were set, with the effector-to-target cell ratio being 10:1 and the co-incubation time being 24 h. 45 min prior to the assay, 20 μL/well of lysis solution (Promega, catalog No. G182A) was added to the target cell maximum release group and the lysis solution control group. The cell lysis rates were measured using a CytoTox96® nonradioactive cytotoxicity assay kit (Promega, G1780).

$$\text{Lysis rate } (\%) = (OD_{\text{administration group}} - OD_{\text{target cell + effector cell group}})/(OD_{\text{target cell maximum release group}} - OD_{\text{target cell group}}) \times 100\%$$

FIG. 13 and Table 15 show the lysis rate of RPMI-8226/BCMA+ tumor cells by effector cells induced by the anti-BCMA/anti-CD3 multispecific antibodies. The maximum lysis rate of the anti-BCMA/anti-CD3 multispecific antibody PC1 to RPMI-8226 is equivalent to that of BC24, but the $EC_{50}$ is lower; the killing effect of PC2 and PC3 on RPMI-8226 is equivalent, the $EC_{50}$ of PC2 and PC3 are 0.0185 nM and 0.0142 nM, respectively, and the maximum lysis rates are both 39%. Celgene BM has the weakest killing effect on RPMI-8226, wherein the $EC_{50}$ is 0.27 nM, and the maximum lysis rate is 29%.

Table 15. Lysis $EC_{50}$ value and maximum lysis rate of each test sample to RPMI-8226 cells

| Sample name | $EC_{50}$(nM) | Maximum lysis rate |
|---|---|---|
| PC1 | 0.0070 | 29% |
| PC2 | 0.0185 | 39% |
| PC3 | 0.01419 | 39% |
| BC24 | 0.01129 | 29% |
| Celgene BM | 0.2712 | 29% |

**Example 16. T Cell Activation Induced by Anti-BCMA/Anti-CD3 Multispecific Antibodies**

CD69 and CD25 are T cell activation markers, and this example relates to the detection of T cell activation by multispecific antibodies (including BC24, PC1, PC2, PC3, and Celgene BM) in the presence of myeloma target cells. The negative control (NC) was trastuzumab.
The specific procedures are as follows:
The target cells NCI-H929 cells and MM1S were adjusted to a cell density of $5 \times 10^5$ cells/mL using 1640 medium for experiment containing 2% FBS (fetal bovine serum) and seeded in a 96-well cell culture plate (eppendorf, catalog No. 0030730199) at 50 μL per well, respectively. The test antibodies at different concentrations were prepared using the medium for experiment. The highest concentration of the antibodies was 8 nM, and the antibodies were diluted 5-fold to obtain a total of 10 concentration gradients. The antibodies at different concentrations were added to the 96-well cell culture plate described above at 50 μL per well. The effector cells human PBMCs were adjusted to a cell density of $2.5 \times 10^6$ cells/mL using medium for experiment and added at 100 μL per well. An administration group (50 μL of target cell + 100 μL of effector cell + 50 μL of antibody), a non-administration group (50 uL of culture medium + 100 μL of effector cell + 50 μL of antibody), a target cell group (50 μL of target cell + 150 μL of culture medium), an effector cell group (100 μL of human PBMC + 100 μL of culture medium), a target cell + effector cell group (50 μL of target cell + 100 μL of effector cell + 50 μL of culture medium), a blank control group (200 μL of culture medium) were set, and the negative control antibody NC was trastuzumab, with the effector-to-target cell ratio being 10:1 and the co-incubation time being 24 h. The mixture was centrifuged and the supernatant was removed. The cells were centrifuged and washed with pre-cooled running buffer (MACS, catalog No. 130-091-221). The supernatant was removed, 100 μL of the running

buffer was added per well, the cells were mixed well, CD4-BV421 (BD, catalog No. 564713), CD8-PE-Cy7 (BD, catalog No. 557746), CD25-APC (BD, catalog No. 555434), and CD69-FITC (BD, catalog No. 555530) were added per well, with each being 2.5 $\mu$L, and the mixture was incubated on ice for 30 min. The cells were washed with pre-cooled running buffer, 75 $\mu$L/well of running buffer was added to resuspend the cells, and the cells were mixed well and detected using a flow cytometer.

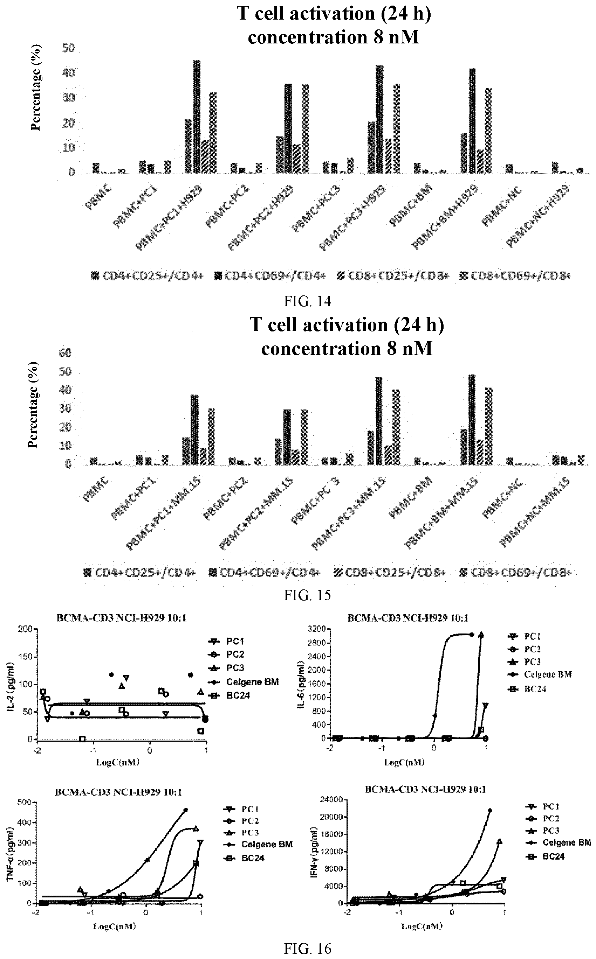

FIGs. 14-15 show the activation degree of T cells when the anti-BCMA/anti-CD3 antibodies are at a concentration of 8 nM in the presence and absence of NCI-H929 or MM1S target cells, with the ordinate showing the percentage of CD8 or CD4 cells positive for the activation marker CD25 or CD69, and the abscissa showing the different groups, wherein CD4+CD25+/CD4+ shows the percentage of CD4 cells positive for CD25 in a certain group, CD4+CD69+/CD4+ shows the percentage of CD4 cells positive for CD69 in a certain group, CD8+CD25+/CD8+ shows the percentage of CD8 cells positive for CD25 in a certain group, and CD8+CD69+/CD8+ shows the percentage of CD8 cells positive for CD69 in a certain group. The results indicate that in the absence of target cells, T cells are not substantially activated, whereas in the presence of target cells, antibodies BC24, PC1, PC2, and PC3 can activate T cells.

## Example 17. Cytokine Release Induced by Anti-BCMA/Anti-CD3 Multispecific Antibodies

The cytokine release induced by the multispecific antibodies in the presence of target cells was investigated by measuring the contents of IL-2, IL-6, TNF-$\alpha$, and IFN-$\gamma$. The safety of the multispecific antibodies was assessed according to the amount of cytokine released.

The specific procedures are as follows:
The target cells NCI-H929 cells were adjusted to a cell density of $5 \times 10^5$ cells/mL using 1640 medium for experiment containing 2% FBS (fetal bovine serum) and seeded in a 96-well cell culture plate (eppendorf, catalog No. 0030730199) at 50 $\mu$L per well. The test antibodies at different concentrations were prepared using the medium for experiment. The highest concentration of the antibodies was 8 nM, and the antibodies were diluted 5-fold to obtain a total of 10 concentration gradients. The antibodies at different concentrations were added to the 96-well cell culture plate described above at 50 $\mu$L per well. The effector cells human PBMCs were adjusted to a cell density of $2.5 \times 10^6$ cells/mL using medium for experiment and added at 100 $\mu$L per well. A standard group, an administration group (50 $\mu$L of target cell + 100 $\mu$L of effector cell + 50 $\mu$L of antibody), a target cell group (50 $\mu$L of target cell + 150 $\mu$L of culture medium), an effector cell group (100 $\mu$L of human PBMC + 100 $\mu$L of culture medium), a target cell + effector cell group (50 $\mu$L of target cell + 100 $\mu$L of effector cell + 50 $\mu$L of culture medium), a effector cell + antibody group (100 $\mu$L of effector cell + 50 $\mu$L of antibody + 50 $\mu$L of culture medium), and a blank control group (200 $\mu$L of culture medium) were set, with the effector-to-target cell ratio being 10:1 and the co-incubation time being 24 h. The mixture was centrifuged, and the supernatant was collected. The sample was treated using assay kits of human-IL2 kit (Cisbio, catalog No. 62HIL02PEG), human-IL6 kit (Cisbio, catalog No. 62HIL06PEG), human-TNF-a kit (Cisbio, catalog No. 62HTNFAPEG), and human-IFN-$\gamma$ kit (Cisbio, catalog No. 62HIFNGPEG) according to the specifications of the kit, and assayed and analyzed using a microplate reader (Molecular Devices, model: Paradigm).

Ratio values of all samples were calculated. With the standard Ratio (Net) as the Y axis and Log(standard concentration value) as the X axis, four-parameter fitting was performed by Graph Pad Prism, Ratio (Net) values of standard and the test sample were substituted, respectively, to obtain the corresponding Log(concentration measurement value), and the concentration measurement value of each factor of the test sample was calculated.

FIG. 16 and Table 16 show the release amount of cytokines IL-2, IL-6, TNF-$\alpha$, and IFN-$\gamma$ after incubation of different concentrations of anti-BCMA/anti-CD3 antibodies with NCI-H929 cells and effector cells for 24 h. The IL-2 release was low for each test antibody. The BC24, PC1, PC2, and PC3 multispecific antibodies disclosed herein have lower cytokines IL-6, TNF-$\alpha$, and IFN-$\gamma$ release levels relative to Celgene BM, and show better safety.

Table 16. Maximum cytokine release amount of each test antibody

| Sample name | IL-2(pg/ml) | IL-6(pg/ml) | TNF-$\alpha$(pg/ml) | IFN-$\gamma$(pg/ml) |
|---|---|---|---|---|
| PC1 | 112.06 | 964.63 | 302.56 | 5463.63 |
| PC2 | 82.49 | 1 | 42.69 | 2841.61 |
| PC3 | 97.51 | 3045.68 | 371.69 | 14391.37 |
| BC24 | 88.07 | 260.3 | 200.25 | 4753.13 |
| Celgene BM | 117.37 | 3040.77 | 464.18 | 21552.72 |

**Example 18. Growth Inhibition of Anti-BCMA/Anti-CD3 Bispecific Antibodies against Multiple Myeloma Target Cell NCI-H929 in Mouse Tumor Model**

The anti-tumor activity of anti-BCMA/anti-CD3 multispecific antibodies was tested using a mouse model of human myeloma NCI-H929 cells. NCI-H929 cells at a concentration of $7.5 \times 10^6$/mouse and hPBMC human peripheral lymphocytes at a concentration of $0.5 \times 10^6$/mouse were mixed and inoculated under aseptic conditions into right side armpit of 8-10 week-old female B-NDG mice (from Biocytogen, Certificate No. 320726200100179034). After subcutaneous grafting, the animals were randomized into 2 groups when the tumor volume reached 100-300 $mm^3$: i. model group (blank control group, normal saline); ii. PC1 group: PC1 test antibody. On day 0, mice were divided into groups and dosed twice a week for one week at a dose of 1.5 mpk at a dose volume of 10 mL/kg. Weights and diameter of the tumor were measured every three days, and the behavior of mice was observed daily. The observation was stopped on day 17. During the study, the tumor volume was measured on days 0, 4, 8, 11, 14, and 17. The tumor volume and the tumor growth inhibition were calculated using the following formulae:

$$\text{Tumor volume (TV)} = (\text{Length} \times \text{Width}^2)/2;$$

Relative Tumor Volume (RTV) = $TV_t/TV_0$, wherein $TV_0$ is the tumor volume at the time of administration following division into cages, and $TV_t$ is the tumor volume at each measurement; Relative tumor proliferation rate T/C (%) = $(T_{RTV}/C_{RTV}) \times 100\%$, wherein $T_{RTV}$ is RTV of treatment group, and $C_{RTV}$ is RTV of blank control group;

$$\text{Tumor growth inhibition (TGI)} = (1\text{-}T/C) \times 100\%.$$

The experimental results are shown in FIG. 17. Compared with the model group, the PC1 antibody drug group can significantly inhibit the growth of human multiple myeloma NCI-H929 subcutaneous xenograft tumor. The tumor growth inhibition of the PC1 antibody on days 8, 11, 14, and 17 are 84.4%, 90.9%, 94.2%, and 95.5%, respectively.

**Example 19. Pharmacokinetics of Single Intravenous Injection of Anti-BCMA/Anti-CD3 Multispecific Antibodies in Cynomolgus Monkeys**

A single intravenous infusion of PC1 injection was performed in cynomolgus monkeys (No. 1M01-1M03 and 2M01-2M03, 3 per dose group, male, body weight: 2-3 kg). The PC1 multispecific antibody was administered at a dose of 1 or 9 mg/kg, and the intravenous drip time was 30 min. Blood samples were collected at each time point before and after administration, and centrifuged at 4000 rpm for about 10 min (2-8 °C) within 1 h to separate sera, and drug concentrations in the blood samples at each time point were measured by ELISA. Sampling time: 0 h (before administration), and 0.5 h, 4 h, 24 h, 48 h, 96 h, 168 h, 240 h, and 336 h after administration.

The parameters were as follows:

$C_{max}$: maximum observed drug concentration
AUC: area under curve of plasma concentration-time
$T_{1/2}$: half life
Cl: clearance
MRT: mean retention time

The experimental results show that when 1 mg/kg and 9 mg/kg of anti-BCMA/anti-CD3 multispecific antibodies are administered by a single intravenous infusion, the drug's terminal elimination half-life $t_{1/2}$ is 105 and 112 h, respectively, the $C_{max}$ is 19 and 200 $\mu$g/mL, respectively, the $AUC_{last}$ is 700 and 7200 h*µg/mL, respectively, and the exposure amount of the product in serum is substantially increased in proportion to the dose.

Table 17. Major mean pharmacokinetic parameters of PC1 injection after single intravenous injection of 1 and 9 mg/kg in cynomolgus monkeys

| Group | $C_{max}$ (µg/mL) | $AUC_{last}$ (h*µg/mL) | $t_{1/2}$ (h) | Cl (mL/h/kg) | $MRT_{last}$ (h) |
|---|---|---|---|---|---|
| 1 mg/kg | 19 | 700 | 105 | 1.417 | 70 |
| 9 mg/kg | 200 | 7200 | 112 | 1.174 | 66 |

**Claims**

1. A multispecific antibody, comprising

    (i) a first antigen-binding moiety that binds to a first antigen;
    (ii) a second antigen-binding moiety that binds to a second antigen; and
    (iii) a third antigen-binding moiety that binds to the first antigen;

    wherein the first antigen is BCMA and the second antigen is an activating T cell antigen, and the first antigen-binding moiety and the third antigen-binding moiety are both single variable domains and each independently comprise any one of:

    (b) CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12;
    (c) CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15;
    (a) CDR1 comprising an amino acid sequence set forth in SEQ ID NO: 7, CDR2 comprising an amino acid sequence set forth in SEQ ID NO: 8, and CDR3 comprising an amino acid sequence set forth in SEQ ID NO: 9; or
    (d) CDR1 comprising an amino acid sequence set forth in SEQ ID NO: 16, CDR2 comprising an amino acid sequence set forth in SEQ ID NO: 17, and CDR3 comprising an amino acid sequence set forth in SEQ ID NO: 18.

2. The multispecific antibody according to claim 1, wherein the first antigen-binding moiety and the third antigen-binding moiety each independently comprise any one of:

    (b) CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12; or
    (c) CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15.

3. The multispecific antibody according to claim 2, wherein the first antigen-binding moiety comprises the following complementarity determining regions: CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12; and the third antigen-binding moiety comprises the following complementarity determining regions: CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15.

4. The multispecific antibody according to claim 2, wherein the third antigen-binding moiety comprises the following complementarity determining regions: CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12; and the first antigen-binding moiety comprises the following complementarity determining regions: CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15.

5. The multispecific antibody according to any one of claims 1-4, wherein

    the first antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 21, 37, or 38, and the third antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 23, 39, or 40; or
    the first antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 37, and the third antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 39.

6. The multispecific antibody according to any one of claims 1-4, wherein

the first antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 23, 39, or 40, and the third antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 21, 37, or 38; or the first antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 39, and the third antigen-binding moiety comprises an amino acid sequence having at least 85%, 90%, 91%, 92%, 93%, 94%, 95%, 96%, 97%, 98%, 99%, or 100% identity to a sequence set forth in SEQ ID NO: 37.

7. The multispecific antibody according to any one of claims 1-6, wherein the first antigen-binding moiety and the third antigen-binding moiety are selected from any one of:

(1) a first antigen-binding moiety comprising an amino acid sequence set forth in SEQ ID NO: 21 and a third antigen-binding moiety comprising an amino acid sequence set forth in SEQ ID NO: 23, 39, or 40;
(2) a first antigen-binding moiety comprising an amino acid sequence set forth in SEQ ID NO: 37 and a third antigen-binding moiety comprising an amino acid sequence set forth in SEQ ID NO: 23, 39, or 40;
(3) a first antigen-binding moiety comprising an amino acid sequence set forth in SEQ ID NO: 38 and a third antigen-binding moiety comprising an amino acid sequence set forth in SEQ ID NO: 23, 39, or 40;
(4) a third antigen-binding moiety comprising an amino acid sequence set forth in SEQ ID NO: 21 and a first antigen-binding moiety comprising an amino acid sequence set forth in SEQ ID NO: 23, 39, or 40;
(5) a third antigen-binding moiety comprising an amino acid sequence set forth in SEQ ID NO: 37 and a first antigen-binding moiety comprising an amino acid sequence set forth in SEQ ID NO: 23, 39, or 40;
(6) a third antigen-binding moiety comprising an amino acid sequence set forth in SEQ ID NO: 38 and a first antigen-binding moiety comprising an amino acid sequence set forth in SEQ ID NO: 23, 39, or 40;
(7) a third antigen-binding moiety comprising an amino acid sequence set forth in SEQ ID NO: 41 and a first antigen-binding moiety comprising an amino acid sequence set forth in SEQ ID NO: 42;
(8) a third antigen-binding moiety comprising an amino acid sequence set forth in SEQ ID NO: 42 and a first antigen-binding moiety comprising an amino acid sequence set forth in SEQ ID NO: 41;
(9) a third antigen-binding moiety comprising an amino acid sequence set forth in SEQ ID NO: 37 and a first antigen-binding moiety comprising an amino acid sequence set forth in SEQ ID NO: 39; or
(10) a third antigen-binding moiety comprising an amino acid sequence set forth in SEQ ID NO: 39 and a first antigen-binding moiety comprising an amino acid sequence set forth in SEQ ID NO: 37.

8. The multispecific antibody according to any one of claims 1-7, wherein the activating T cell antigen is CD3.

9. The multispecific antibody according to any one of claims 1-8, wherein the second antigen-binding moiety comprises a heavy chain variable region and a light chain variable region, wherein the heavy chain variable region comprises the following complementarity determining regions: HCDR1 comprising an amino acid sequence set forth in SEQ ID NO: 43, HCDR2 comprising an amino acid sequence set forth in SEQ ID NO: 44, and HCDR3 comprising an amino acid sequence set forth in SEQ ID NO: 45; the light chain variable region comprises LCDR1 comprising an amino acid sequence set forth in SEQ ID NO: 46, LCDR2 comprising an amino acid sequence set forth in SEQ ID NO: 47, and LCDR3 comprising an amino acid sequence set forth in SEQ ID NO: 48; or the heavy chain variable region comprises HCDR1, HCDR2, and HCDR3 of a variable region set forth in SEQ ID NO: 49, and the light chain variable region comprises LCDR1, HCDR2, and LCDR3 of a variable region set forth in SEQ ID NO: 50.

10. The multispecific antibody according to any one of claims 1-9, wherein the second antigen-binding moiety comprises a heavy chain variable region having an amino acid sequence having at least 85%, 90%, 91%, 92%, 93%, 94%, 95%, 96%, 97%, 98%, 99%, or 100% identity to a sequence set forth in SEQ ID NO: 49 and a light chain variable region having an amino acid sequence having at least 85%, 90%, 91%, 92%, 93%, 94%, 95%, 96%, 97%, 98%, 99%, or 100% identity to a sequence set forth in SEQ ID NO: 50; or the heavy chain variable region of the second antigen-binding moiety comprises an amino acid sequence set forth in SEQ ID NO: 49 and the light chain variable region thereof comprises an amino acid sequence set forth in SEQ ID NO: 50.

11. The multispecific antibody according to any one of claims 1-10, further comprising (iv) an Fc domain composed of two Fc polypeptides.

12. The multispecific antibody according to claim 11, wherein

(1) the second antigen-binding moiety is an ScFv, the first antigen-binding moiety, at its C-terminus, is fused to the N-terminus of one Fc polypeptide of the Fc domain, the second antigen-binding moiety, at its C-terminus, is fused to the N-terminus of the other Fc polypeptide of the Fc domain, and the third antigen-binding moiety, at its C-terminus, is fused to the N-terminus of the first antigen-binding moiety;

(2) the second antigen-binding moiety is an ScFv, the first antigen-binding moiety, at its C-terminus, is fused to the N-terminus of one Fc polypeptide of the Fc domain, the second antigen-binding moiety, at its C-terminus, is fused to the N-terminus of the other Fc polypeptide of the Fc domain, and the third antigen-binding moiety, at its C-terminus, is fused to the N-terminus of the second antigen-binding moiety;

(3) the second antigen-binding moiety is an Fab, the first antigen-binding moiety, at its C-terminus, is fused to the N-terminus of one Fc polypeptide of the Fc domain, the second antigen-binding moiety, at the C-terminus of its Fab heavy chain, is fused to the N-terminus of the other Fc polypeptide of the Fc domain, and the third antigen-binding moiety, at its C-terminus, is fused to the N-terminus of the first antigen-binding moiety; or

(4) the second antigen-binding moiety is an Fab, the first antigen-binding moiety, at its C-terminus, is fused to the N-terminus of one Fc polypeptide of the Fc domain, the second antigen-binding moiety, at the C-terminus of its Fab heavy chain, is fused to the N-terminus of the other Fc polypeptide of the Fc domain, and the third antigen-binding moiety, at its C-terminus, is fused to the N-terminus of the Fab light chain of the second antigen-binding moiety or the N-terminus of the Fab heavy chain of the second antigen-binding moiety.

**13.** The multispecific antibody according to any one of claims 11-12, wherein the Fc domain is an IgG Fc domain, a human IgG Fc domain, or an Fc domain of human IgG1 or human IgG4;

optionally, the Fc domain comprises an amino acid substitution that promotes association of the two Fc polypeptides of the Fc domain; or/and

the Fc domain comprises an amino acid substitution that reduces the binding affinity of the Fc domain for an Fc receptor and/or effector function of the Fc domain; or/and

the Fc domain comprises an amino acid substitution that reduces or eliminates the binding of a CH3 region of one Fc polypeptide in the Fc domain to Protein A.

**14.** The multispecific antibody according to any one of claims 1-13, wherein the multispecific antibody consists of:

(1) two polypeptide chains, wherein one polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 65, and the other polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 67;

(2) two polypeptide chains, wherein one polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 69, and the other polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 71;

(3) three polypeptide chains, wherein one polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 59, the another polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 61, and the yet another polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 63;

(4) three polypeptide chains, wherein one polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 73, the another polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 61, and the yet another polypeptide chain comprises an amino acid sequence having at least 85%, 90%, 91%, 92%, 93%, 94%, 95%, 96%, 97%, 98%, 99%, or 100% identity to a sequence set forth in SEQ ID NO: 75;

(5) two polypeptide chains, wherein one polypeptide chain comprises an amino acid sequence set forth in SEQ ID NO: 65, and the other polypeptide chain comprises an amino acid sequence set forth in SEQ ID NO: 67;

(6) two polypeptide chains, wherein one polypeptide chain comprises an amino acid sequence set forth in SEQ ID NO: 69, and the other polypeptide chain comprises an amino acid sequence set forth in SEQ ID NO: 71;

(7) three polypeptide chains, wherein one polypeptide chain comprises an amino acid sequence set forth in SEQ ID NO: 59, the another polypeptide chain comprises an amino acid sequence set forth in SEQ ID NO: 61, and the yet another polypeptide chain comprises an amino acid sequence set forth in SEQ ID NO: 63; or

(8) three polypeptide chains, wherein one polypeptide chain comprises an amino acid sequence set forth in SEQ ID NO: 73, the another polypeptide chain comprises an amino acid sequence set forth in SEQ ID NO: 61, and the yet another polypeptide chain comprises an amino acid sequence set forth in SEQ ID NO: 75.

15. A pharmaceutical composition comprising the multispecific antibody according to any one of claims 1-14 and a pharmaceutically acceptable carrier.

16. A method for treating a disease related to BCMA expression in a subject, comprising administering to the subject a therapeutically effective amount of the multispecific antibody according to any one of claims 1-14 or the pharmaceutical composition according to claim 15, wherein the disease is lymphoma, multiple myeloma, leukemia, systemic lupus erythematosus, or rheumatoid arthritis.

17. A BCMA-binding antibody comprising a single variable domain, wherein the single variable domain comprises:

(2) CDR1 comprising an amino acid sequence set forth in SEQ ID NO: 10, CDR2 comprising an amino acid sequence set forth in SEQ ID NO: 11, and CDR3 comprising an amino acid sequence set forth in SEQ ID NO: 12;
(3) CDR1 comprising an amino acid sequence set forth in SEQ ID NO: 13, CDR2 comprising an amino acid sequence set forth in SEQ ID NO: 14, and CDR3 comprising an amino acid sequence set forth in SEQ ID NO: 15;
(1) CDR1 comprising an amino acid sequence set forth in SEQ ID NO: 7, CDR2 comprising an amino acid sequence set forth in SEQ ID NO: 8, and CDR3 comprising an amino acid sequence set forth in SEQ ID NO: 9; or
(4) CDR1 comprising an amino acid sequence set forth in SEQ ID NO: 16, CDR2 comprising an amino acid sequence set forth in SEQ ID NO: 17, and CDR3 comprising an amino acid sequence set forth in SEQ ID NO: 18.

CHO-hBCMA

FIG. 1A

U266

FIG. 1B

RPMI8226

FIG. 1C

HUVEC

FIG. 1D

A  HEK293T-CynoBCMA      B    HEK293T

FIG. 2

APRIL

FIG. 3

```
                1        10        20        30        40      50  54
                |--------+---------+---------+--------+---------+---|
Hu-BCMA-ECD     MLQMAGQCSQNEYFDSLLHACIPCQLRCSSNTPPLTCQRYCNASVTNSVKGTNA
Cyno-BCMA-ECD   MLQMARQCSQNEYFDSLLHDCKPCQLRCSS-TPPLTCQRYCNASMTNSVKGMNA
```

FIG. 4A

FIG. 4B

FIG. 5

FIG. 6

FIG. 7

**CD3 BCMA MM.1S**

FIG. 8

**CD3 BCMA RPMI-8226**

FIG. 9

**CD3 BCMA CD3$^{+}$T**

FIG. 10

FIG. 11

FIG. 12

FIG. 13

FIG. 14

FIG. 15

FIG. 16

FIG. 17

FIG. 18

## INTERNATIONAL SEARCH REPORT

| International application No. |
| --- |
| **PCT/CN2022/086842** |

**A. CLASSIFICATION OF SUBJECT MATTER**

C07K 16/46(2006.01)i; C07K 16/28(2006.01)i; A61K 39/395(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNABS, SIPOABS, DWPI, CNKI, 万方, WANFANG, NCBI, ISI web of knowledge, 百度, BAIDU, STN: BCMA, CD3, 抗体, 双特异抗体, 多特异抗体, 单域抗体, VHH, B细胞成熟抗原, bispecific, multipecific, single domain, B cell maturation antigen, T cell antigen, 序列7-50 blast

**C. DOCUMENTS CONSIDERED TO BE RELEVANT**

| Category* | Citation of document, with indication, where appropriate, of the relevant passages | Relevant to claim No. |
| --- | --- | --- |
| PY | CN 112794916 A (CHIA TAI TIANQING PHARMACEUTICAL GROUP NANJING SHUNXIN PHARMACEUTICAL CO., LTD. et al.) 14 May 2021 (2021-05-14) <br> claims 1-9, and sequences 37-44 | 1-16 |
| PY | WO 2022037528 A1 (CHIA TAI TIANQING PHARMACEUTICAL GROUP CO., LTD.) 24 February 2022 (2022-02-24) <br> claims 1-15, and sequences 7-25 | 1-16 |
| PX | WO 2022037528 A1 (CHIA TAI TIANQING PHARMACEUTICAL GROUP CO., LTD.) 24 February 2022 (2022-02-24) <br> claims 1-15, and sequences 7-25 | 17 |
| A | CN 111138542 A (SHANGDONG NEW TIME PHARMACEUTICAL CO., LTD.) 12 May 2020 (2020-05-12) <br> entire document | 1-17 |
| A | CN 110551221 A (GUANGZHOU EXCELMAB BIOMEDICAL TECHNOLOGY CO., LTD.) 10 December 2019 (2019-12-10) <br> entire document | 1-17 |

☑ Further documents are listed in the continuation of Box C.     ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **17 June 2022** | **18 July 2022** |

| Name and mailing address of the ISA/CN | Authorized officer |
| --- | --- |
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/086842** |

**C.      DOCUMENTS CONSIDERED TO BE RELEVANT**

| Category* | Citation of document, with indication, where appropriate, of the relevant passages | Relevant to claim No. |
| --- | --- | --- |
| A | CN 110891971 A (TENEOBIO, INC.) 17 March 2020 (2020-03-17)<br>      entire document | 1-17 |
| A | DILILLO, D. J. et al. "A BCMAxCD3 bispecific T cell–engaging antibody demonstrates robust antitumor efficacy similar to that of anti-BCMA CAR T cells"<br>*BLOOD ADVANCES*, Vol. 5, No. 5, 02 March 2021 (2021-03-02),<br>      pp. 1291-1304 | 1-17 |
| A | HIPP, S. et al. "A novel BCMA/CD3 bispecific T-cell engager for the treatment of multiple myeloma induces selective lysis in vitro and in vivo"<br>*LEUKEMIA*, 13 January 2017 (2017-01-13),<br>      pp. 1743-1751 | 1-17 |

Form PCT/ISA/210 (second sheet) (January 2015)

| INTERNATIONAL SEARCH REPORT | International application No. |
| --- | --- |
| | **PCT/CN2022/086842** |

| Box No. I | Nucleotide and/or amino acid sequence(s) (Continuation of item 1.c of the first sheet) |
| --- | --- |

1.  With regard to any nucleotide and/or amino acid sequence disclosed in the international application, the international search was carried out on the basis of a sequence listing:

     a.   ☑   forming part of the international application as filed:

         ☑   in the form of an Annex C/ST.25 text file.

         ☐   on paper or in the form of an image file.

     b.   ☐   furnished together with the international application under PCT Rule 13*ter*.1(a) for the purposes of international search only in the form of an Annex C/ST.25 text file.

     c.   ☐   furnished subsequent to the international filing date for the purposes of international search only:

         ☐   in the form of an Annex C/ST.25 text file (Rule 13*ter*.1(a)).

         ☐   on paper or in the form of an image file (Rule 13*ter*.1(b) and Administrative Instructions, Section 713).

2. ☐   In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

| International application No. |
| --- |
| **PCT/CN2022/086842** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **16**
    because they relate to subject matter not required to be searched by this Authority, namely:

    [1]   Claim 16 comprises a method for treating a living human or animal body, and therefore, said claim does not comply with PCT Rule 39.1(iv). The present report is based on a search conducted on the basis of a pharmaceutical use of the antibody or pharmaceutical composition in claim 16.

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2022/086842**

| Patent document cited in search report | | | Publication date (day/month/year) | Patent family member(s) | | | Publication date (day/month/year) |
|---|---|---|---|---|---|---|---|
| CN | 112794916 | A | 14 May 2021 | None | | | |
| WO | 2022037528 | A1 | 24 February 2022 | None | | | |
| CN | 111138542 | A | 12 May 2020 | EP | 3875485 | A1 | 08 September 2021 |
| | | | | AU | 2019370758 | A1 | 10 June 2021 |
| | | | | EP | 3875479 | A1 | 08 September 2021 |
| | | | | US | 2021371526 | A1 | 02 December 2021 |
| | | | | US | 2022002431 | A1 | 06 January 2022 |
| | | | | CN | 112996817 | A | 18 June 2021 |
| | | | | CN | 111138544 | A | 12 May 2020 |
| | | | | CN | 112996807 | A | 18 June 2021 |
| | | | | CN | 111138545 | A | 12 May 2020 |
| | | | | WO | 2020088608 | A1 | 07 May 2020 |
| | | | | WO | 2020088164 | A1 | 07 May 2020 |
| | | | | KR | 20210087472 | A | 12 July 2021 |
| | | | | CA | 3118238 | A1 | 07 May 2020 |
| | | | | EP | 3875489 | A1 | 08 September 2021 |
| | | | | KR | 20210089697 | A | 16 July 2021 |
| | | | | CN | 112996810 | A | 18 June 2021 |
| | | | | AU | 2019370339 | A1 | 10 June 2021 |
| | | | | CN | 111138547 | A | 12 May 2020 |
| | | | | WO | 2020088403 | A1 | 07 May 2020 |
| | | | | EP | 3889174 | A1 | 06 October 2021 |
| | | | | CA | 3118397 | A1 | 07 May 2020 |
| | | | | US | 2022002407 | A1 | 06 January 2022 |
| | | | | CO | 2021006970 | A2 | 30 July 2021 |
| | | | | WO | 2020088605 | A1 | 07 May 2020 |
| | | | | CN | 112955461 | A | 11 June 2021 |
| | | | | PE | 20211867 | A1 | 21 September 2021 |
| | | | | JP | 2022512865 | A | 07 February 2022 |
| | | | | CN | 111138546 | A | 12 May 2020 |
| | | | | JP | 2022512997 | A | 07 February 2022 |
| | | | | WO | 2020088437 | A1 | 07 May 2020 |
| | | | | EP | 3889179 | A1 | 06 October 2021 |
| | | | | CL | 2021001143 | A1 | 22 October 2021 |
| CN | 110551221 | A | 10 December 2019 | EP | 3967713 | A1 | 16 March 2022 |
| | | | | AU | 2019452936 | A1 | 20 January 2022 |
| | | | | WO | 2021000530 | A1 | 07 January 2021 |
| CN | 110891971 | A | 17 March 2020 | IL | 271194 | D0 | 30 January 2020 |
| | | | | RU | 2020100891 | A3 | 20 July 2021 |
| | | | | WO | 2018237037 | A2 | 27 December 2018 |
| | | | | CA | 3067584 | A1 | 27 December 2018 |
| | | | | KR | 20200018498 | A | 19 February 2020 |
| | | | | AU | 2018289515 | A1 | 06 February 2020 |
| | | | | BR | 112019026907 | A2 | 30 June 2020 |
| | | | | JP | 2020524506 | A | 20 August 2020 |
| | | | | EP | 3642237 | A2 | 29 April 2020 |
| | | | | US | 2021147564 | A1 | 20 May 2021 |
| | | | | SG | 11201912774 R | A | 30 January 2020 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019195535 A

- US 20190263920 A1

**Non-patent literature cited in the description**

- **CYRUS CHOTHIA et al.** Canonical Structures for the Hypervariable Regions of Immunoglobulins. *J. Mol. Biol.,* 1987, vol. 196, 901-917
- **ELVIN A. KABAT et al.** Sequences of Proteins of Immunological Interest. Public Health Service, National Institutes of Health, 1991
- **KABAT, E.A. et al.** Sequences of Proteins of Immunological Interest. Public Health Service, National Institutes of Health, 1991

- **BOSSEN, C. et al.** *Semin. Immunol.,* 2006, vol. 18 (5), 263-275
- **SILVANA PESSANO et al.** The T3/T cell receptor complex: antigenic distinction between the two 20-kd T3 (T3-delta and T3 - epsilon) subunits. *EMBO J.,* February 1985, vol. 4 (2), 337-344